(19) Europäisches Patentamt / European Patent Office / Office européen des brevets

(11) **EP 4 294 257 B1**

(12) **EUROPEAN PATENT SPECIFICATION**

(45) Date of publication and mention
of the grant of the patent:
**05.11.2025 Bulletin 2025/45**

(21) Application number: **22708698.0**

(22) Date of filing: **22.02.2022**

(51) International Patent Classification (IPC):
***A61B 5/00*** (2006.01)  ***A61B 6/00*** (2024.01)

(52) Cooperative Patent Classification (CPC):
**A61B 5/4872; A61B 5/7275; A61B 6/482;
A61B 6/5217**

(86) International application number:
**PCT/US2022/017222**

(87) International publication number:
**WO 2022/178382 (25.08.2022 Gazette 2022/34)**

(54) **METHOD FOR DIAGNOSING AND TREATING PARTIAL LIPODYSTROPHY**

VERFAHREN ZUR DIAGNOSE UND BEHANDLUNG VON PARTIELLER LIPODYSTROPHIE

MÉTHODE DE DIAGNOSTIC ET DE TRAITEMENT D'UNE LIPODYSTROPHIE PARTIELLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.02.2021 US 202163152100 P**

(43) Date of publication of application:
**27.12.2023 Bulletin 2023/52**

(73) Proprietor: **Regeneron Pharmaceuticals, Inc.
Tarrytown, NY 10591 (US)**

(72) Inventors:
• **ZHENG, Wenjun
Tarrytown, New York 10591 (US)**
• **HARRIS, Charles
Chappaqua, New York 10514 (US)**
• **MUSSER, Bret
Tarrytown, New York 10591 (US)**

(74) Representative: **J A Kemp LLP
80 Turnmill Street
London EC1M 5QU (GB)**

(56) References cited:
**US-A1- 2019 309 079**

• **CYNTHIA M VALERIO ET AL: "Body composition study by dual-energy x-ray absorptiometry in familial partial lipodystrophy: finding new tools for an objective evaluation", DIABETOLOGY & METABOLIC SYNDROME, BIOMED CENTRAL LTD, LONDON, UK, vol. 4, no. 1, 31 August 2012 (2012-08-31), pages 40, XP021130060, ISSN: 1758-5996, DOI: 10.1186/1758-5996-4-40**
• **JOY T ET AL: "Predicting abdominal adipose tissue among women with familial partial lipodystrophy", METABOLISM, CLINICAL AND EXPERIMENTAL, W.B. SAUNDERS CO., PHILADELPHIA, PA, US, vol. 58, no. 6, 1 June 2009 (2009-06-01), pages 828 - 834, XP026110512, ISSN: 0026-0495, [retrieved on 20090513], DOI: 10.1016/J.METABOL.2009.03.001**
• **GUILLÍN-AMARELLE CRISTINA ET AL: "Type 1 familial partial lipodystrophy: understanding the Köbberling syndrome", ENDOCRINE, HUMANA PRESS, INC, US, vol. 54, no. 2, 30 July 2016 (2016-07-30), pages 411 - 421, XP036085923, ISSN: 1355-008X, [retrieved on 20160730], DOI: 10.1007/S12020-016-1002-X**

Remarks:
The complete document including Reference Table(s) and the Sequence Listing(s) can be downloaded from the EPO website

**Description**

**RELATED APPLICATIONS**

This application claims priority under 35 U.S.C. 119 (e) to U.S. Provisional Patent Application Serial No. 63/152,100, entitled "METHOD FOR DIAGNOSING AND TREATING PARTIAL LIPODYSTROPHY", filed February 22, 2021.

**FIELD OF THE INVENTION**

Disclosed herein are methods for diagnosing partial lipodystrophy and methods of treatment thereof. The invention is defined by the appended claims.

**SEQUENCE LISTING**

The sequence listing of the present application is submitted electronically as an ASCII formatted sequence listing with a file name 10850WO01_Sequence_Listing_ST25, creation date of February 22, 2022, and a size of about 49,152 bytes. This sequence listing submitted is part of the specification and is herein by reference in its entirety.

**BACKGROUND OF THE INVENTION**

Rare diseases, such as lipodystrophies, may be complex, chronic, and often not well known conditions with debilitating health consequences. Such conditions are poorly understood by general practitioners and medical specialists alike, in part because of their low prevalence and frequent phenotypic heterogeneity. Lack of knowledge often leads to long delays in diagnosis and sometimes to misdiagnosis, which can be greatly detrimental to patients.

Lipodystrophy syndromes are a collection of rare heterogeneous disorders which may be characterized by a deficiency of adipose tissue without evidence of nutrition deprivation or a catabolic state. At first glance, certain types of partial lipodystrophy (PLD) cannot be clearly distinguished from other common metabolic diseases (e.g., poorly controlled diabetes mellitus with truncal obesity) based on phenotype unless the physician is suspicious for lipodystrophy and checks carefully for certain characteristics such as the appearance of the limbs which look thinner than in a normal person. Also, the onset of fat loss may be gradual and delay the diagnosis both in genetic and acquired forms (Akinci et al., Lipodystrophy Syndromes: Presentation and Treatment. [Updated 2018 Apr 24]. In: Feingold KR, Anawalt B, Boyce A, et al., editors. Endotext [Internet]. South Dartmouth (MA): MDText.com, Inc.; 2000-. Available from: www.ncbi.nlm.nih.gov/-books/NBK513130/; Brown, et al., The Diagnosis and Management of Lipodystrophy Syndromes: A Multi-Society Practice Guideline. The Journal of clinical endocrinology and metabolism 2016;101:4500-4511; and Handelsman et al., The clinical approach to the detection of lipodystrophy - an AACE consensus statement. Endocr Pract 2013;19:107-116).

It has been estimated that 15% of the general population has missense mutations in certain genes linked to PLD; thus, making many of these mutations relatively common (current work). For example, of 37 patients in the Geisinger Healthcare System with a reported pathogenic *LMNA* variant, none had evidence of lipodystrophy based on prevalence of diabetes and triglyceride levels. Thus, a physician or investigator studying PLD may identify a mutation in a patient with suspected PLD and assume the mutation is causative. This is not an effective approach.

In addition, the percent of published mutations linked to PLD categorized as "pathogenic" by ClinVar is small. ClinVar is a freely accessible, public archive of reports of the relationships among human variations and phenotypes hosted by the National Center for Biotechnology Information (NCBI) and funded by intramural National Institutes of Health (NIH) funding. The reason for this is that the genetic architecture of familial partial lipodystrophy (FPLD) is such that there are many variants, each with a small number of patients. Therefore, the evidence from these small numbers of patients may not reach the evidence thresholds set forth by ClinVar. This further confounds accurate diagnosis of FPLD. It is not known what the overall burden of metabolic disease is in the sum total of subjects with these mutations. Gonzaga-Jauregui *et al.,* demonstrated that 75% of patients with pathogenic familial partial lipodystrophy (FPLD) mutations were not diagnosed as having lipodystrophy by their physicians (defined as an absence of the appropriate diagnosis code from ICD9/ICD10) despite having disease defining co-morbidities such as diabetes and hypertriglyceridemia.

Current means for diagnosing PLD and its subtypes lack sufficient specificity or sensitivity. A basic algorithm for differential diagnosis of lipodystrophy subtypes has been proposed, but these algorithms are very generalized not taking into account the pattern of fat loss seen in PLD patients and the extent thereof (Araújo-Vilar & Santini, Diagnosis and treatment of lipodystrophy: a step-by-step approach, Journal of Endocrinological Investigation (2019) 42: 61-73). Attempts to use dual-energy X-ray absorptiometry (DXA) criteria to diagnose FPLD have also used static measures such as "trunk/leg fat percent ratio" greater than a constant (Ajluni et al., Spectrum of disease associated with partial lipodystrophy: lessons from a trial cohort. Clin Endocrinol (Oxf) 2017; 86:698-707). Other attempts have used normalized measures such as a leg fat % <1 percentile for age in comparison to large datasets of control populations (Vasandani et al., Diagnostic Value of Anthropometric Measurements for Familial Partial Lipodystrophy, Dunnigan Variety. J Clin Endocrinol Metab 2020; 105(7): 2132-2141). These authors stated that 1st percentile of leg fat performed better than other measures they tested such as arm fat, trunk fat, trunk/leg fat percent ratio, and triceps skin thickness assessed with calipers. However, the latter approach was only tested in patients with FPLD2. Furthermore, using the 1st percentile of leg fat may perform poorly in obese subjects. Finally, some have demonstrated diagnosing PLD based on the examination of images visualizing adipose tissue that are generated by DXA (Meral, 2018). However, this is not practical on a larger scale since it requires several lipodystrophy experts to review the images given the imperfect concordance by lipodystrophy experts on the diagnosis using this method.

## SUMMARY OF THE INVENTION

Disclosed is a method for determining whether a subject has or is likely to have partial lipodystrophy (e.g., familial partial lipodystrophy) comprising determining:

(1) if (i) total body fat percentage is greater than about 36%; and (ii) trunk/leg fat percent ratio is greater than about 1.35 (e.g., about 1.3 or 1.353); or (2) if (i) total body fat percentage is less than or equal to about 36%; and (ii) (trunk/leg fat percent ratio)-(0.0311 X total body fat percentage) $\geq$ about 0.232 (e.g., 0.2 or 0.23) (preferably, wherein the subject is female);
or
(1) if (i) total body fat percentage is greater than about 25.7%; and (ii) trunk/leg fat percent ratio is greater than about 1.5; or (2) if (i) total body fat percentage is less than or equal to about 25.7%; and (ii) (trunk/leg fat percent ratio)-(0.0429 X total body fat percentage) > about 0.4 (preferably, wherein the subject is male)
or
(1) if (i) total body fat percentage is greater than about 27.5%; and (ii) trunk/leg fat percent ratio is greater than about 1.5; or (2) if (i) total body fat percentage is less than or equal to about 27.5%; and (ii) (trunk/leg fat percent ratio)-(0.0429 X total body fat percentage) > about 0.321 (e.g., 0.3 or 0.32) (preferably, wherein the subject is male), wherein, if the criteria are met, the subject has or is likely to have partial lipodystrophy and, optionally, if the criteria are not met, the patient does not have or is not likely to have partial lipodystrophy.

Methods for determining whether a subject of Korean ethnicity has or is likely to have partial lipodystrophy are provided as well. Such methods include the step of determining whether trunk/leg fat percent ratio is greater than about 1.54, e.g., wherein the subject is a female; and/or whether trunk/leg fat percent ratio is greater than about 1.90, e.g., wherein the subject is a male; wherein, if the criteria are met, the subject has or is likely to have partial lipodystrophy and, optionally, if the criteria are not met, the patient does not have or is not likely to have partial lipodystrophy.

Disclosed is method for identifying a trunk/leg fat percentage ratio indicative of an individual in a population, for whom trunk/leg fat percent ratio and total body fat percentages of individuals of such population are known (e.g., in the NHANES or KNHANES database), as having or as likely to have partial lipodystrophy comprising determining the trunk/leg fat percent ratio which is above that of about 99% of individuals in the population having the same total body fat percentage, wherein an individual in the population is determined to have or likely to have partial lipodystrophy if the individual has a trunk/leg fat percent ratio which is above 99% of individuals in the population having the same total body fat percentage.

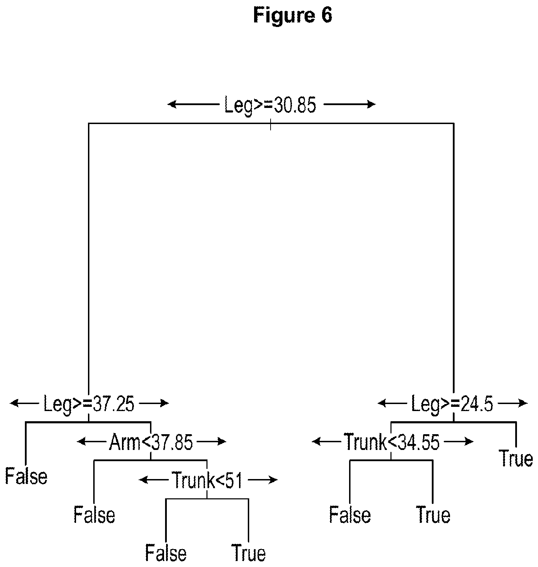

Disclosed is a method for determining whether a female subject has or is likely to have partial lipodystrophy (e.g., FPLD) comprising evaluating the decision tree of Figure 6, as follows: (a) evaluating leg fat percentage, arm fat percentage and trunk fat percentage at each decision node; wherein the left path is followed when an affirmative decision is made at a decision node and the right path if followed when a negative decision is made at a decision node; and (b) if a positive decision is reached at an end-point node, the subject has or is likely to have partial lipodystrophy, or if a negative decision is reached at an end-point node, the subject does not have or is not likely to have partial lipodystrophy.

Disclosed is a method for determining whether a female subject has or is likely to have partial lipodystrophy (e.g., FPLD) including the steps of evaluating leg, arm and/or trunk fat percentage wherein the subject has or is likely to have partial lipodystrophy if: leg fat percentage is less than about 31%; leg fat percentage is less than about 31%, but greater than about 25%; leg fat percentage is less than about 31%, but greater than or equal to about 25%; leg fat percentage is less than about 31%, but greater than or equal to about 25% and trunk fat percentage is greater than or equal to about 35%; leg fat percentage is greater than or equal to about 31% but less than 37% and arm fat percentage is greater than or equal to about 38%; or leg fat percentage is greater than or equal to about 31% but less than 37%, arm fat percentage is greater than or equal to about 38% and trunk fat percentage is greater than or equal to about 51%;
and/or the female subject does not have or is not likely to have partial lipodystrophy if: leg fat percentage is greater than or equal to about 31%; leg fat percentage is less than about 31%, but greater than or equal to about 25% and trunk fat is less than about 35%; leg fat percentage is greater than or equal to about 37%; leg fat percentage is greater than or equal to about 31% but less than 37%; leg fat percentage is greater than or equal to about 31% but less than 37% and arm fat percentage is less than about 38%; or leg fat percentage is greater than or equal to about 31% but less than 37%, arm fat percentage is greater than or equal to about 38% and trunk fat percentage is less than about 51%.

Disclosed is a method for determining whether a subject has or is likely to have PLD as discussed herein further includes the step of administering, to the subject, an effective amount of LEPR agonist (e.g., mibavademab, H4H16650P2; H4H16679P2; H4H17319P2; H4H17321P2; H4H18417P2; H4H18438P2; H4H18445P2; H4H18446P2; H4H18449P2; H4H18482P2; H4H18487P2 and/or H4H18492P2) if the subject has or is likely to have PLD.

It is disclosed that any of the methods for determining that are set forth herein include the step of performing DXA analysis on the subject to determine the appropriate body fat percentage values and these values are then employed as set forth herein in the method. It is disclosed that the subject's fat distribution is also examined by a physician or clinician and, for example, determined to have altered fat distribution indicative of PLD. It is disclosed that the subject's clinical history and blood levels (e.g., serum leptin, lipid, and/or sugar levels) are examined and, for example, are determined to be indicative of PLD. It is disclosed that the subject's genotype is analyzed, for example, and determined to have alleles of, for example, *CIDEC, LIPE, PCYT1A, LMNA, PPARG, AKT2, PLIN1, CAV1, ADRA2A, PIK3R1, ZMPSTE24, PSMB8, WRN, POLD1* and/or *BLM* indicative of PLD (e.g., FPLD).

Disclosed is a method for treating partial lipodystrophy (e.g., FPLD), in a subject, comprising determining whether the subject has or is likely to have partial lipodystrophy by a method of the present invention and, if the subject has partial lipodystrophy or is likely to have partial lipodystrophy, then administering, to the subject, an effective amount of LEPR agonist (e.g., mibavademab, H4H16650P2; H4H16679P2; H4H17319P2; H4H17321P2; H4H18417P2; H4H18438P2; H4H18445P2; H4H18446P2; H4H18449P2; H4H18482P2; H4H18487P2 and/or H4H18492P2).

Disclosed is a method of treating partial lipodystrophy (e.g., FPLD) in a subject in need thereof comprising administering a therapeutically effective amount of LEPR agonist (e.g., mibavademab, H4H16650P2; H4H16679P2; H4H17319P2; H4H17321P2; H4H18417P2; H4H18438P2; H4H18445P2; H4H18446P2; H4H18449P2; H4H18482P2; H4H18487P2 and/or H4H18492P2) to the subject, wherein the subject has been determined to have or to likely have partial lipodystrophy by a method disclosed herein.

Disclosed is a LEPR agonist (e.g., mibavademab, H4H16650P2; H4H16679P2; H4H17319P2; H4H17321P2; H4H18417P2; H4H18438P2; H4H18445P2; H4H18446P2; H4H18449P2; H4H18482P2; H4H18487P2 and/or H4H18492P2) for use in a method for treating partial lipodystrophy (e.g., FPLD), in a subject, the method comprising determining whether the subject has or is likely to have partial lipodystrophy by a method of the described herein and, if the subject has partial lipodystrophy or is likely to have partial lipodystrophy, then administering, to the subject, an effective amount of the LEPR agonist.

Disclosed is a LEPR agonist (e.g., mibavademab, H4H16650P2; H4H16679P2; H4H17319P2; H4H17321P2; H4H18417P2; H4H18438P2; H4H18445P2; H4H18446P2; H4H18449P2; H4H18482P2; H4H18487P2 and/or H4H18492P2) for use in a method of treating partial lipodystrophy (e.g., FPLD) in a subject in need thereof, the method comprising administering a therapeutically effective amount of the LEPR agonist to the subject, wherein the subject has been determined to have or to likely have partial lipodystrophy by a method disclosed herein.

Disclosed is the use of a LEPR agonist (e.g., mibavademab, H4H16650P2; H4H16679P2; H4H17319P2; H4H17321P2; H4H18417P2; H4H18438P2; H4H18445P2; H4H18446P2; H4H18449P2; H4H18482P2; H4H18487P2 and/or H4H18492P2) in the manufacture of a medicament for the treatment of partial lipodystrophy (e.g., FPLD), in a subject, comprising determining whether the subject has or is likely to have partial lipodystrophy by a method described herein and, if the subject has partial lipodystrophy or is likely to have partial lipodystrophy, then administering, to the subject, an effective amount of the LEPR agonist.

Disclosed is the use of a LEPR agonist (e.g., mibavademab, H4H16650P2; H4H16679P2; H4H17319P2; H4H17321P2; H4H18417P2; H4H18438P2; H4H18445P2; H4H18446P2; H4H18449P2; H4H18482P2; H4H18487P2 and/or H4H18492P2) in a method of treating partial lipodystrophy (e.g., FPLD) in a subject in need thereof, comprising administering a therapeutically effective amount of the LEPR agonist to the subject, wherein the subject has been determined to have or to likely have partial lipodystrophy by a method described herein.

It is disclosed that the familial partial lipodystrophy (FPLD) is FPLD1, FPLD2, FPLD3, FPLD4, FPLD5, FPLD6 or FPLD7. In an embodiment of the invention, the fat percentages are determined by Dual-energy X-ray Absorptiometry, e.g., using a 3-compartment model.

It is disclosed that the subject suffers from one or more selected from the group consisting of: acanthosis nigricans; atherosclerosis; atherosclerotic coronary heart disease; cardiac arrhythmia; cardiomyopathy; congestive heart failure; coronary artery disease; diabetes; dyslipidemia; eruptive xanthoma; glucose intolerance; hepatic steatosis; hepatomegaly; hirsutism; hyperandrogenemia; hyperglycemia; hypertension; hypertriglyceridemia; insulin resistance; liver cirrhosis; muscular dystrophy; myopathy; non-alcoholic steatohepatitis; oligomenorrhoea; pancreatitis; polycystic ovary syndrome; proteinuric renal disease; severe insulin resistance; and subfertility. In an embodiment of the invention, the subject has a homozygous or heterozygous mutation in the *LMNA, PPARG, PLIN1, AKT2, LIPE, CIDEC, ZMPSTE24,*

*PIK3R1, ANDRA2A, CAV1, PCYT1A, PSMB8, WRN, POLD1,* and/or the *BLM* gene.

It is disclosed that the LEPR agonist is an isolated agonist antibody or antigen-binding fragment that binds specifically to LEPR, e.g., comprising: (i) a light chain variable region that comprises the LCDR1, LCDR2 and LCDR3 of a light chain variable region that comprises the amino acid sequence set forth in SEQ ID NO: 10; and a heavy chain variable region that comprises the HCDR1, HCDR2 and HCDR3 of a heavy chain variable region that comprises the amino acid sequence set forth in SEQ ID NO: 2; (ii) a light chain variable region that comprises the LCDR1, LCDR2 and LCDR3 of a light chain variable region that comprises the amino acid sequence set forth in SEQ ID NO: 10; and a heavy chain variable region that comprises the HCDR1, HCDR2 and HCDR3 of a heavy chain variable region that comprises the amino acid sequence set forth in SEQ ID NO: 18; (iii) a light chain variable region that comprises the LCDR1, LCDR2 and LCDR3 of a light chain variable region that comprises the amino acid sequence set forth in SEQ ID NO: 10; and a heavy chain variable region that comprises the HCDR1, HCDR2 and HCDR3 of a heavy chain variable region that comprises the amino acid sequence set forth in SEQ ID NO: 26; (iv) a light chain variable region that comprises the LCDR1, LCDR2 and LCDR3 of a light chain variable region that comprises the amino acid sequence set forth in SEQ ID NO: 10; and a heavy chain variable region that comprises the HCDR1, HCDR2 and HCDR3 of a heavy chain variable region that comprises the amino acid sequence set forth in SEQ ID NO: 34; (v) a light chain variable region that comprises the LCDR1, LCDR2 and LCDR3 of a light chain variable region that comprises the amino acid sequence set forth in SEQ ID NO: 10; and a heavy chain variable region that comprises the HCDR1, HCDR2 and HCDR3 of a heavy chain variable region that comprises the amino acid sequence set forth in SEQ ID NO: 42; (vi) a light chain variable region that comprises the LCDR1, LCDR2 and LCDR3 of a light chain variable region that comprises the amino acid sequence set forth in SEQ ID NO: 10; and a heavy chain variable region that comprises the HCDR1, HCDR2 and HCDR3 of a heavy chain variable region that comprises the amino acid sequence set forth in SEQ ID NO: 50; (vii) a light chain variable region that comprises the LCDR1, LCDR2 and LCDR3 of a light chain variable region that comprises the amino acid sequence set forth in SEQ ID NO: 10; and a heavy chain variable region that comprises the HCDR1, HCDR2 and HCDR3 of a heavy chain variable region that comprises the amino acid sequence set forth in SEQ ID NO: 58; (viii) a light chain variable region that comprises the LCDR1, LCDR2 and LCDR3 of a light chain variable region that comprises the amino acid sequence set forth in SEQ ID NO: 10; and a heavy chain variable region that comprises the HCDR1, HCDR2 and HCDR3 of a heavy chain variable region that comprises the amino acid sequence set forth in SEQ ID NO: 66; (ix) a light chain variable region that comprises the LCDR1, LCDR2 and LCDR3 of a light chain variable region that comprises the amino acid sequence set forth in SEQ ID NO: 10; and a heavy chain variable region that comprises the HCDR1, HCDR2 and HCDR3 of a heavy chain variable region that comprises the amino acid sequence set forth in SEQ ID NO: 74; (x) a light chain variable region that comprises the LCDR1, LCDR2 and LCDR3 of a light chain variable region that comprises the amino acid sequence set forth in SEQ ID NO: 90; and a heavy chain variable region that comprises the HCDR1, HCDR2 and HCDR3 of a heavy chain variable region that comprises the amino acid sequence set forth in SEQ ID NO: 82; (xi) a light chain variable region that comprises the LCDR1, LCDR2 and LCDR3 of a light chain variable region that comprises the amino acid sequence set forth in SEQ ID NO: 90; and a heavy chain variable region that comprises the HCDR1, HCDR2 and HCDR3 of a heavy chain variable region that comprises the amino acid sequence set forth in SEQ ID NO: 98; or (xii) a light chain variable region that comprises the LCDR1, LCDR2 and LCDR3 of a light chain variable region that comprises the amino acid sequence set forth in SEQ ID NO: 90; and a heavy chain variable region that comprises the HCDR1, HCDR2 and HCDR3 of a heavy chain variable region that comprises the amino acid sequence set forth in SEQ ID NO: 106; or an antibody or antigen-binding fragment that binds to the same epitope as any one or more of (i)-(xii) and/or competes for binding to LEPR with any one or more of (i)-(xii); for example, comprising: (i) a light chain variable region that comprises the amino acid sequence set forth in SEQ ID NO: 10; and a heavy chain variable region that comprises the amino acid sequence set forth in SEQ ID NO: 2; (ii) a light chain variable region that comprises the amino acid sequence set forth in SEQ ID NO: 10; and a heavy chain variable region that comprises the amino acid sequence set forth in SEQ ID NO: 18; (iii) a light chain variable region that comprises the amino acid sequence set forth in SEQ ID NO: 10; and a heavy chain variable region that comprises the amino acid sequence set forth in SEQ ID NO: 26; (iv) a light chain variable region that comprises the amino acid sequence set forth in SEQ ID NO: 10; and a heavy chain variable region that comprises the amino acid sequence set forth in SEQ ID NO: 34; (v) a light chain variable region that comprises the amino acid sequence set forth in SEQ ID NO: 10; and a heavy chain variable region that comprises the amino acid sequence set forth in SEQ ID NO: 42; (vi) a light chain variable region that comprises the amino acid sequence set forth in SEQ ID NO: 10; and a heavy chain variable region that comprises the amino acid sequence set forth in SEQ ID NO: 50; (vii) a light chain variable region that comprises the amino acid sequence set forth in SEQ ID NO: 10; and a heavy chain variable region that comprises the amino acid sequence set forth in SEQ ID NO: 58; (viii) a light chain variable region that comprises the amino acid sequence set forth in SEQ ID NO: 10; and a heavy chain variable region that comprises the amino acid sequence set forth in SEQ ID NO: 66; (ix) a light chain variable region that comprises the amino acid sequence set forth in SEQ ID NO: 10; and a heavy chain variable region that comprises the amino acid sequence set forth in SEQ ID NO: 74; (x) a light chain variable region that comprises the amino acid sequence set forth in SEQ ID NO: 90; and a heavy chain variable region that comprises the amino acid sequence set forth in SEQ ID NO: 82; (xi) a light chain variable region that comprises the amino acid sequence set forth in SEQ ID NO: 90; and a heavy chain variable region that comprises the amino acid sequence set forth in SEQ ID NO: 98; or (xii) a light chain variable region that comprises the amino acid sequence set forth in SEQ ID NO: 90; and a heavy chain variable region that comprises the amino acid sequence set forth in SEQ ID NO: 106; or an antibody or antigen-binding fragment that binds to the same epitope as any one or more of (i)-(xii) and/or competes for binding to LEPR with any one or more of (i)-(xii).

It is disclosed that a LEPR agonist is administered in association with a further therapeutic agent, for example, which is recombinant human leptin; metreleptin; a PCSK9 inhibitor; an anti-PCSK9 antibody or antigen-binding fragment thereof; alirocumab; evolocumab; bococizumab; lodelcizumab; ralpancizumab; an HMG CoA reductase inhibitor; atorvastatin; rosuvastatin; cerivastatin; pitavastatin; fluvastatin; simvastatin; lovastatin; pravastatin; ezetimibe; insulin; an insulin variant; an insulin secretagogue; metformin; a sulfonylurea; a sodium glucose cotransporter 2 (SGLT2) inhibitor; dapaglifozin; canaglifozin; empagliflozin; a GLP-1 agonist or analogue; exenatide; liraglutide; lixisenatide; albiglutide; dulaglutide; a glucagon (GCG) inhibitor; an anti-GCG antibody; a glucagon receptor (GCGR) inhibitor; an anti-GCGR antibody; a small molecule GCGR antagonist; a GCGR-specific antisense oligonucleotide; an anti-GCGR aptamer; a GCGR spiegelmer; an angiopoietin-like protein (ANGPTL) inhibitor; an anti-ANGPTL3 antibody; an anti-ANGPTL4 antibody; an anti-ANGPTL8 antibody; phentermine; orlistat; topiramate; bupropion; topiramate & phentermine; bupropion & naltrexone; bupropion & zonisamide; pramlintide & metreleptin; lorcaserin; cetilistat; tesofensine; velneperit; fish oil; pioglitazone; setmelanotide;, a fibrate; fenofibrate; prednisone; niacin; anticonvulsants; digoxin; coumadin; vitamin D; thyroxine; a thyroid supplement; a vitamin supplement; a calcium supplement; carnitine; coenzyme Q10; an anti-constipation medication; an anti-allergic medication; gabapentin; a narcotic; ketamine; lidocaine; and/or venlafaxine hydrochloride.

## BRIEF DESCRIPTION OF THE FIGURES

**Figure 1.** Ratio of trunk to leg fat vs. total fat percentage in large control dataset of women in the UK Biobank.

**Figure 2.** Ratio of trunk to leg fat vs. total fat percentage in subjects with FPLD (classified as FPLD2, having a PPARG mutation, having a POLD mutation or FPLDX) (Meral 2018).

**Figure 3.** Overlay of graphs in Figures 1 and 2 with a best-fit line was drawn to separate controls from FPLD patients.

**Figure 4.** Sensitivity Analysis of Algorithm. Trunk to leg fat ratio vs total fat in independent blinded data set of PLD patients. Three patients not correctly identified by the algorithm (A-C) are indicated.

**Figure 5A.** Receiver operating characteristic (ROC) Curve of sensitivity vs. specificity with algorithm of the present invention indicated with a dot. (A) overall curve.

**Figure 5B.** Receiver operating characteristic (ROC) Curve of sensitivity vs. specificity with algorithm of the present invention indicated with a dot. (B) curve blown-up in the region of interest to show more detail.

**Figure 6.** PLD diagnostic decision tree. The nodes at the bottom of the tree are end-point nodes and the other nodes are decision nodes; follow the left arrow if "yes" at decision nodes, Follow the right arrow if "no" at decision nodes, "True" at the end-point nodes indicates positive diagnosis of PLD, "False" at end-point nodes indicates negative diagnosis of PLD.

**Figure 7.** Receiver Operator Curve (ROC) for the combined dataset of NIH and NHANES (in females).

**Figure 8.** Distribution of trunk-leg ratios vs total fat among females in NHANES along with the values for the 1st, 3rd, 10th, 25th, 50th, 75th, 90th, 97th and 99th percentile alongside the discriminator lines separating FPLD from controls.

**Figure 9.** Discriminator function for males with FPLD using percentiles (100 % sensitivity and 98.1 % specificity).

**Figure 10.** Discriminator function for males with FPLD using percentiles (85.7 % sensitivity and 99.3 % specificity).

**Figure 11.** Receiver Operator Curve (ROC) - performance of two discriminators compared to using the trunk-leg ratio alone (in males).

**Figure 12.** Enlarged ROC curve (in males).

**Figure 13.** Relationship between trunk-leg ratio and total body fat percent of Mexican-American, Hispanic-other, White, Black and Other/Biracial groups (females) in NHANES.

**Figure 14.** Ratio of trunk/leg ratio and total fat percent compared between Korean females (NHANES) and American females (NHANES)

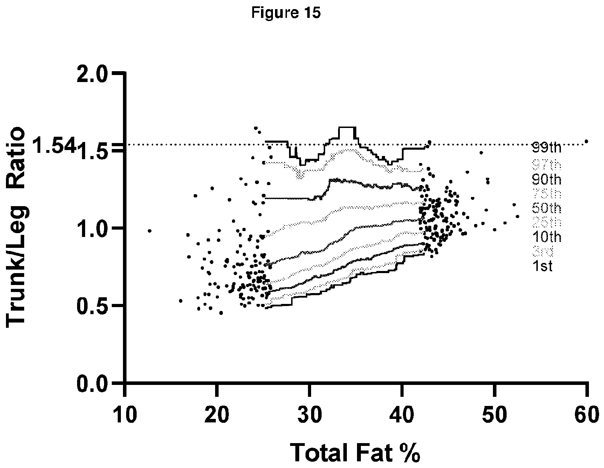

**Figure 15.** Demonstration of the technique to determine the discriminator function in Koreans based on 1st percentile of trunk-leg ratio for total adiposity

**Figure 16.** Demonstration of the relationship between pretest probability (related to prevalence in the study population) and positive predictive value for FPLD when using the empiric approach with specificity of 99.3% or the 99th percentile approach with a specificity of 99%.

## DETAILED DESCRIPTION OF THE INVENTION

Partial lipodystrophy (PLD) is a difficult disease to diagnose and there are currently no objective criteria to make the diagnosis. As discussed above, previous attempts to develop methods to diagnose PLD have not been optimal. The algorithms described herein are advantageous, in part, by accounting for the relationship between total fat and trunk-leg fat ratio and normalizing the trunk-leg ratio to total body percent fat. This approach has superior performance characteristics with a sensitivity of 95% and specificity of greater than 99%. Using female FPLD patients and female NHANES subjects as controls, one can see that the function is approximated by the 99th percentile of trunk/leg ratio for total adiposity compared to an appropriate control population. This approach can be generalized to other demographics such as race and ethnicity using FPLD subjects and controls, or using control data alone, using the 99th percentile of trunk/leg ratio for total adiposity in controls even in the absence of data from FPLD patients. Algorithms of the present invention are also advantageous since they take into account differences in the trunk-leg fat ratio between people of different ethnicities. The tree-based algorithm uses comprehensive DXA information from arm, leg, and trunk. Using female Caucasian FPLD patients and female NHANES subjects as controls, the tree-base algorithm performs well with a sensitivity of 84% and specificity of 98%.

### Diagnostics

Disclosed are highly sensitive and selective methods for diagnosing partial lipodystrophy (PLD) (e.g., familial partial lipodystrophy (FPLD)) in a subject based, for example, on the distribution of fat on the trunk and appendages and ratios thereof. Optionally, any such diagnostic method further includes the step of treating said PLD in the diagnosed individual by administering a therapeutically effective amount of LEPR agonist. Optionally, any such diagnostic method includes the step of diagnosing the subject as not having or likely not having PLD (e.g., FPLD) is the diagnostic criteria are not met.

"Sensitivity" measures the proportion of positives that are correctly identified. "Specificity" measures the proportion of negatives that are correctly identified.

Disclosed are methods for determining whether a subject (a female) has (or is likely to have) partial lipodystrophy (e.g., FPLD) comprising determining whether

(i) total body fat percentage is greater than about 36%;
and
(ii) trunk/leg fat percent ratio is greater than about 1.3 (e.g., about 1.35 or 1.353); or

(a) total body fat percentage is less than or equal to about 36%;
and

(b) (trunk/leg fat percent ratio)-(0.0311 X total body fat percentage)$\geq$ about 0.232 (e.g., about 0.2 or 0.23);

wherein, if the criteria are met, the subject has or is likely to have partial lipodystrophy and, optionally, if the criteria are not met, the patient does not have or is not likely to have partial lipodystrophy.

Trunk/leg fat percent ratio is the ratio of trunk leg fat percent and the leg fat percent. Disclosed are methods as discussed herein wherein the trunk/leg fat percent ratio is replaced with a ratio of trunk and leg fat that is expressed in units other than fat percent.

Disclosed are methods for determining whether a subject (a male) has (or is likely to have) partial lipodystrophy (e.g., FPLD) comprising determining whether

(i) total body fat percentage is greater than about 25.7, and
(ii) trunk-leg fat ratio is greater than about 1.5;

or

(a) total body fat percentage is less than or equal to about 25.7,
and

(b) (trunk/leg fat percent ratio)-(0.0429 X total body fat percentage) > about 0.4;

wherein, if the criteria are met, the subject has or is likely to have partial lipodystrophy and, optionally, if the criteria are not met, the patient does not have or is not likely to have partial lipodystrophy.

Disclosed are methods for determining whether a subject (*e.g.*, a female or male, preferably a male) has (or is likely to have) partial lipodystrophy (*e.g.*, FPLD) comprising determining whether

(i) total body fat percentage is greater than about 27.5,
and
(ii) trunk/leg fat ratio is greater than or equal to 1.5;
or

(a) total body fat percentage is less than or equal to 27.5, and

(b) (trunk/leg fat percent ratio)-(0.0429 X total body fat percentage) > about 0.321 (or >0.3 or 0.32);

wherein, if the criteria are met, the subject has or is likely to have partial lipodystrophy and, optionally, if the criteria are not met, the patient does not have or is not likely to have partial lipodystrophy.

Disclosed are methods for determining whether a subject of Korean ethnicity has (or is likely to have) partial lipodystrophy (e.g., FPLD) comprising determining:

whether trunk/leg fat percent ratio is greater than about 1.54, wherein the subject is a Korean female; and/or
whether trunk/leg fat percent ratio is greater than about 1.90, wherein the subject is a Korean male;
wherein, if the criteria are met, the subject has or is likely to have partial lipodystrophy and, optionally, if the criteria are not met, the patient does not have or is not likely to have partial lipodystrophy.

It is disclosed that the attributes of the subject of Korean ethnicity are in the KNHANES database. It is disclosed that a subject is characterized as being of Korean ethnicity if they can be characterized as having genetic traits *(e.g.,* haplotypes) common to ethnic Koreans. Kim et al., The Origin and Composition of Korean Ethnicity Analyzed by Ancient and Present-Day Genome Sequences, Genome Biol. Evol. 12(5):553-565 (2020); The 1000 Genome Project (1KGP); 1000 Genomes Project Consortium, et al. 2015. A global reference for human genetic variation. Nature 526:68; or Jeon et al., Korean Genome Project: 1094 Korean personal genomes with clinical information, Science Advances, Vol. 6, no. 22 (2020).

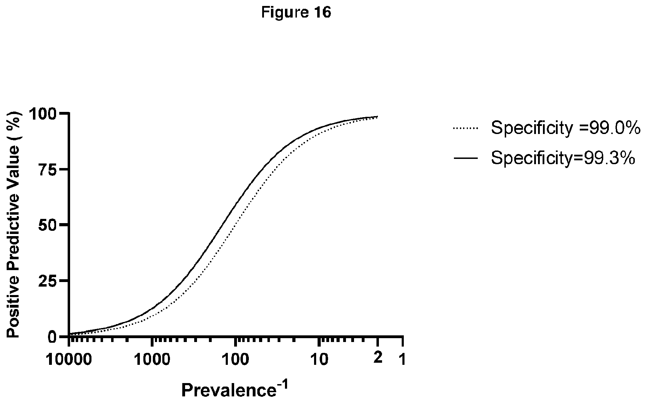

Methods for identifying PLD (e.g., FPLD) diagnostic criteria for a given population of individuals are also provided. For example, disclosed is method for identifying a trunk/leg fat percent ratio indicative of an individual in a population, for whom trunk/leg fat percent ratio and total body fat percentages of individuals of such population are known, as having or likely having partial lipodystrophy (e.g., FPLD) comprising: determining the trunk/leg fat percent ratio percentage which is above that of 99% of individuals in the population having each total body fat percentage; wherein an individual in the population is determined to have or likely to have partial lipodystrophy if the individual has a trunk/leg fat percent ratio which is above 99% of individuals in the population having the same total body fat percentage. For example, in an embodiment of the invention, a plot of total body fat percentage (X-axis) vs. trunk/leg fat percent ratio (Y-axis) is plotted. A 99-percentile curve is then generated, wherein 99% of the individuals having a total body fat percentage in any given range that has been plotted lie below the curve. Trunk-leg fat ratios lying above the curve at a given total body fat percentage are determinative of having or likely having PLD (e.g., FPLD). It should be noted that the positive-predictive value of this algorithm is a function of the pre-test probability. It is notoriously difficult to screen for rare-disease because of this. The algorithms may not be as of maximal usefulness when used in the general population. For example, using the diagnostic approach derived from NHANES controls and FPLD patients, the specificity was 99.3%. Using the published FPLD prevalence of 1:6,000, for every 1 true positive found out of 6,000 screened subjects there would be 42 False Positives (FP=1-specificity) which would yield a PPV of 2.3% (TP/[TP+FP] or 1/[1+42]). The required positive predictive value of a test depends on the implications of a positive test result. For example, well established newborn screening programs can have PPV of ~1% (Kwon & Farrell, The Magnitude and Challenge of False-Positive Newborn Screening Test Results. Arch Pediatr Adolesc Med. 2000;154(7):714-718). For the diagnosis of FPLD, such a low PPV would not be acceptable. In order for the PPV to be 50%, the pre-test probability would need to be 1:142. Therefore, the algorithm will have higher positive predictive value when the pre-test probability is higher than what is seen in the general population. The relationship between incidence and PPV is shown in Figure 16. In order to achieve a PPV of 50%, the pre-test probability would need to be greater than 0.7% or 1 in 142. In other words, one would have to screen subjects with risk factors that would place them into a category where the probability of having FPLD is 0.7% or greater. Another way of stating this is that one would need to enrich the general population 42-fold such that the prevalence was increased from 1:6000 to 1:142. While data are lacking on the exact criteria that could be used, appropriate screening populations would include:

1) First- and Second-degree Relatives of subjects with FPLD

2) Non-obese diabetics
3) Individuals with triglycerides >400 mg/dL
4) Individuals in which the clinician notices an altered fat distribution

Relatives of subjects with PLD would be at risk of having FPLD themselves, depending on the strength of the relationship. The following relatives of an FPLD patient would have a risk greater than 1% and be worth screening, shown with the expected DNA shared and the observed range of DNA shared (where available) (website: customercare.23andme.com/hc/en-us/articles/212170668-Average-Percent-DNA-Shared-Between-Relatives): Parent or child (50%); Full Sibling (50%, 38-61%); grandparent, grandchild, aunt, uncle, niece, nephew, half-sibling (25%, 17-34%); first cousin, great-grandparent, great-grandchild, great-uncle, great-aunt, great nephew, great niece (12.5%, 4-23%); first-cousin once removed, half first cousin (6.25%, 2-11.5%); second cousin (3.1%, 2-6%). It would not be useful to screen all diagnosed diabetics, because diagnosed diabetes affects 8% of the US population and doing so would result in a PPV of ~20%. However, because obesity is a strong risk factor for diabetes, FPLD subjects with diabetes have lower BMI and a lower percent are obese (BMI>30) than diabetics without FPLD. Approximately 28% of diabetics are not obese (Centers for Disease Control, National Diabetes Statistics Report 2020: Estimate of Diabetes and Its Burden in the United States, website: www.cdc.gov/diabetes/pdfs/data/statistics/national-diabetes-statistics-report.pdf) such that the population of non-obese diabetics is 2.2%. Therefore, FPLD is expected to be present in ~0.8% of non-obese diabetics making this population worth screening. Given the high rate of elevated triglycerides observed in FPLD, if one screened a population of subjects with high triglycerides, the pre-test probability for having FPLD would be increased. Specifically, if one screened the population in the top 2.3% of triglycerides, the pre-test probability would be 50%. This would correspond to individuals with triglycerides >400 mg/dL.

It is disclosed that diagnosis of PLD (e.g., FPLD) is made to one or more subjects in a population that includes a greater number of subjects with PLD than that of the general population (e.g., general population within a given country or region (such as the USA, Europe, Korea or Asia) or worldwide). For example, such methods may be performed to diagnose PLD in subjects within a population having a pre-test probability of greater than or equal to 0.7% or 1 in 142. It is disclosed that the subject is within a population of first- and second-degree relatives of subjects with PLD (e.g., FPLD); non-obese diabetics; individuals with triglycerides >400 mg/dL; or individuals in which the clinician notices an altered fat distribution, or any combination of 1, 2, 3 or 4 thereof.

The term "likely to have" PLD refers, generally, to a probability of having PLD of greater than 50%.

Disclosed are methods for determining whether a subject has or likely has partial lipodystrophy (e.g., FPLD) comprising determining if any of the following three criteria (PLD diagnostic criteria) are met:

(1) Leg fat percentage less than 25;
(2) Leg fat percentage greater than or equal to 25 but less than 31; and trunk fat percentage greater than or equal to 35; and/or,
(3) Leg fat percentage greater than or equal to 31 but not greater than or equal to 37; and arm fat percentage greater than or equal to 38; and trunk fat greater than or equal to 51;

and,

diagnosing the patient as having lipodystrophy or likely having partial lipodystrophy if the three criterial are met, and, optionally, diagnosing the patient as not having or likely not having partial lipodystrophy if none of the three criterial are met, e.g., if any of the following negative diagnostic criteria are met:

(a) Leg fat percentage less than 31, but greater than or equal to 25; and trunk fat percentage less than 35;
(b) Leg fat percentage greater than or equal to 37;
(c) Leg fat percentage greater than or equal to 31 but less than 37; and arm fat percentage less than 38; or
(d) Leg fat percentage greater than or equal to 31 but less than 37; arm fat percentage greater than or equal to 38; and trunk fat percentage less than 51.

It is disclosed that, such a diagnostic method is as embodied in the decision tree that is set forth in Figure 6 herein. Thus, the present invention provides a method for determining if a female subject has or is likely to have PLD (e.g., FPLD) comprising evaluating, as needed, the following:

(1) the leg fat percentage at the first decision node; then
(2) leg fat percentage at a second decision node; then
(3) arm fat percentage or trunk fat percentage at a third decision node; then
(4) trunk fat percentage at a fourth decision node;

and making a final determination as to whether the subject has or is likely to have PLD according to the end node that is reached;

wherein, after an affirmative decision at a decision node, the next node evaluated is to the left and down the decision tree; and

after, a negative decision at a decision node, the next node evaluated is to the right and down the decision tree.

For example, in any of such methods for determining whether a patient has PLD, the patient may first be subject to a body scan for determining body fat percentages of the body, appendages (*e.g.,* arm and/or leg) and/or trunk; *e.g.,* wherein the scan is DXA. Thus, disclosed are methods for diagnosing PLD in a subject comprising subjecting the subject to DXA scan and determining such fat percentages and, then, diagnosing the subject with PLD if the PLD diagnostic criteria, as discussed herein, are met.

It is disclosed that as mentioned above, a subject who is positively diagnosed with PLD (*e.g.*, FPLD) is treated with a therapeutically effective amount of LEPR agonist (*e.g.*, REGN4461). Thus, disclosed are methods for treating PLD in a subject comprising determining if the subject has PLD using a method discussed herein and, if so, positively diagnosing the subject with PLD and then administering, to the subject, a therapeutically effective amount of LEPR agonist (*e.g.*, REGN4461).

### Dual-energy X-ray Absorptiometry (DXA or DEXA) and Body Fat Determination

The fundamental principle of DXA is the measurement of the transmission of X-rays through the body at high and low energies (Ward et al., Tools for measuring bone in children and adolescents (2007) In: Sawyer et al, (eds). Bone densitometry in growing patients -Guidelines for clinical practice. Humana Press: Totowa, New Jersey: 2007. pp 15-40). The X-ray source generates a beam of X-rays, which consists of photon particles carried through electromagnetic energy. As photons traverse the subject's tissues, physical interactions take place that attenuate beam intensity. The differential attenuation of the X-ray beam at these two energies is used to calculate body composition in the scanned region. Methods discussed herein for determining whether a subject has PLD are based, in part, on body fat measurements. Thus, such methods may include the step of subjecting the subject to DXA determination of the appropriate body fat content (*e.g.*, as set forth herein). Such a method may be performed by an appropriate technician as ordered by a subject's clinician or treating physician. Results of the DXA measurements may then be sent to the clinician or physician for performance of the methods as discussed herein.

Models of body composition are based on assumptions that the human body consists of two (2-C), three (3-C), or four (4-C) compartments. The use of DXA for measuring body composition and the 2-, 3- and 4-compartment models are known in the art. (See *e.g.,* Meral et al., 'Fat Shadows' from DXA for the Qualitative Assessment of Lipodystrophy: When a Picture Is Worth a Thousand Numbers, Diabetes Care 41:2255-2258 (2018); Hind et al., In vivo precision of the GE Lunar iDXA densitometer for the measurement of total body composition and fat distribution in adults. Eur J Clin Nutr 2011; 65:140-142; Chen et al., Precision of total body BMD and body composition using the GE Lunar Prodigy densitometer [Abstract], The American Society for Bone and Mineral Research (ASBMR) 2005:P SU132.48; Kiebzak et al., Measurement precision of body composition variables using the lunar DPX-L densitometer, J Clin Densitom 2000;3:35-41; Cordero-MacIntyre et al., Reproducibility of DXA in obese women. J Clin Densitom. 2002; 5:35-44; Genton et al., Comparison of body weight and composition measured by two different dual energy X-ray absorptiometry devices and three acquisition modes in obese women. Clin Nutr 2006; 25:428-437; Johnson et al., Precision and stability of dual-energy X-ray absorptiometry measurements. Calcif Tissue Int 1991;49: 174-178; Rezzi et al., Body composition precision with the Lunar iDXA [abstract]. J Clin Densitom 2009; 12: 402, P176; and Tothill et al., Comparisons between Hologic, Lunar and Norland dual-energy X-ray absorptiometers and other techniques used for whole-body soft tissue measurements. Eur J Clin Nutr 1994; 48: 781-794). A 2-C model, such as underwater weighing, divides the body into two compartments-fat mass and fat free mass. A 3-C model includes fat mass and two constituents of the fat free mass. DXA is an example of a 3-C model that assesses three body compartments-fat mass, lean mass, and bone mineral. The 4-C model separates the lean mass into water and protein, thus providing a picture of four body compartments-fat mass, protein, water, and bone mineral. In order to determine the amount of fat and fat-free soft tissue in the body, DXA measures the ratio of attenuation of the two photon energies at anatomical sites that do not contain any bone (typically the pure soft tissue that is adjacent to bone). When no bone is present, the ratio of the attenuation of the two photon energies is linearly related to the proportion of fat in the soft tissue. After the attenuation of the X-ray beam has been analyzed in regions with soft tissue and bone, as well as regions with soft tissue only, fat mass, lean tissue mass, and bone mineral mass can be discriminated.

DXA systems are commercially available, for example: Hologic QDR 1000W (Hologic Inc.; Marlborough, MA); Lunar DPX (GE Healthcare; Chicago, IL); Norland XR 26 Mark II HS (Swissray International, Inc.; Edison, NJ); Lunar Prodigy (GE Healthcare; Chicago, IL); and Lunar iDXA (GE Healthcare; Chicago, IL).

Typically, a DXA scanner includes a table for supporting a patient and in which is positioned an X-ray source (typically composed of an X-ray generator, an X-ray tube, an X-ray filter and an X-ray collimator) that is movable with respect to the table below the patient. In some DXA systems/scanners, the detector is placed within the arm opposite the detector, such that the detector and source are located on opposed sides of the patient. The detector is mainly one-dimensional, but can be two dimensional or other suitable dimensional configurations, and can be moved to capture X-ray photons emitted by the X-ray source and going through the patient body. The arm moves the detector and is associated with the X-ray source that is moving in synchronization with the detector on the arm. The arm moves both the detector and X-ray source in a direction corresponding to the longer dimension of the DXA table. In DXA scanner implementing raster scan (pencil beam or fan beam), both detector and X-ray source can be moved in a direction perpendicular to the longer dimension of the DXA table in order to scan the table/body along its width. In an embodiment of the invention, the table includes an X-ray detector disposed within an arm spaced above the table that is movable with respect to the table. The table, along with the detector and the X-ray source and arm are operably connected to a computer system that can control the operation of the X-ray source and/or arm, and that can receive imaging data from the detector resulting from X-rays from the X-ray source passing through the patient and striking the detector. The present invention includes methods, as discussed herein, wherein the composition of a subject's body or body part is as determined using a DXA system having any of these components.

There are various types of DXA methods. For example, pencil beam systems use a highly collimated pencil beam of X-rays with a single detector; fan beam systems use a fan-beam X-ray source and a set of detectors wherein measurement of the whole body can be made with a single sweep of the X-ray arm; and narrow fan beam systems scan in

a rectilinear fashion with a fan beam that is wider than the pencil beam but still narrower than the fan beams wherein each pass of the narrow fan beam across the body overlaps the previous one and the overlapping images are matched and reconstructed, resulting in a

more accurate estimation of the depth of the bone and reducing the magnification effect. See Toombs et al., The Impact of Recent Technological Advances on the Trueness and Precision of DXA to Assess Body Composition, Obesity 20: 30-39 (2012). The present invention includes methods, as discussed herein, wherein the composition of a subject's body or body part is as determined using any of these DXA methods.

**Antigen-binding proteins**

Disclosed are methods for treating (e.g., diagnosing and treating) partial lipodystrophy (e.g., FPLD) in a patient who has been diagnosed as having the condition, by administering, to the patient, an effective dose of LEPR agonist.

A LEPR agonist binds LEPR and activates LEPR signaling. "Activation of LEPR signaling" and the like means the stimulation of an intracellular effect that normally results from the interaction of leptin with LEPR in cells that express LEPR, e.g., the transcriptional activation of STAT3, which can be detected using any method that can measure or identify, directly or indirectly, STAT3 activity, *e.g.*, using a labeled version of STAT3 expressed in a reporter cell line. In an embodiment of the invention, a LEPR agonist is an antibody or antigen-binding fragment thereof.

The term "antibody", as used herein, refers to immunoglobulin molecules comprising four polypeptide chains, two heavy chains (HCs) and two light chains (LCs), inter-connected by disulfide bonds (e.g., IgG). In an embodiment of the invention, each antibody heavy chain (HC) comprises a heavy chain variable region ("HCVR" or "$V_H$") (*e.g.*, SEQ ID NO: 2, 18, 26, 34, 42, 50, 58, 66, 74, 82, 98 or 106 or a variant thereof) and a heavy chain constant region; and each antibody light chain (LC) comprises a light chain variable region ("LCVR or "$V_L$") (*e.g.,* SEQ ID NO: 10 or 90 or a variant thereof) and a light chain constant region (CL). The $V_H$ and $V_L$ regions can be further subdivided into regions of hypervariability, termed complementarity determining regions (CDR), interspersed with regions that are more conserved, termed framework regions (FR). Each $V_H$ and $V_L$ comprises three CDRs and four FRs.

It is disclosed that the assignment of amino acids to each framework or CDR domain is in accordance with the definitions of Sequences of Proteins of Immunological Interest, Kabat, et al.; National Institutes of Health, Bethesda, Md.; 5th ed.; NIH Publ. No. 91-3242 (1991); Kabat (1978) Adv. Prot. Chem. 32:1-75; Kabat, et al., (1977) J. Biol. Chem. 252:6609-6616; Chothia, et al., (1987) J Mol. Biol. 196:901-917 or Chothia, et al., (1989) Nature 342:878-883. Thus, the present invention includes methods of using antibodies and antigen-binding fragments including the CDRs of a $V_H$ and the CDRs of a $V_L$, which $V_H$ and $V_L$ comprise amino acid sequences as set forth herein (or a variant thereof), wherein the CDRs are as defined according to Kabat and/or Chothia.

It is disclosed that an anti-LEPR antigen-binding protein, *e.g.*, antibody or antigen-binding fragment, comprises a heavy chain constant domain, *e.g.*, of the type IgA (*e.g.,* IgA1 or IgA2), IgD, IgE, IgG (*e.g.,* IgG1, IgG2, IgG3 and IgG4 (*e.g.,* comprising a S228P and/or S108P mutation)) or IgM. In an embodiment of the invention, an antigen-binding protein, *e.g.*, antibody or antigen-binding fragment, comprises a light chain constant domain, *e.g.,* of the type kappa or lambda. The present invention includes methods of using antigen-binding proteins comprising the variable domains set forth herein (*e.g.*, mibavademab, H4H16650P2; H4H16679P2; H4H17319P2; H4H17321P2; H4H18417P2; H4H18438P2; H4H18445P2; H4H18446P2; H4H18449P2; H4H18482P2; H4H18487P2; and/or H4H18492P2) which are linked to a heavy and/or light chain constant domain, *e.g.*, as set forth above. See WHO Drug Information Vol 34, No. 4, 2020; Proposed INN: List 124, @ "mibavademab".

"Isolated" antigen-binding proteins (*e.g.*, antibodies or antigen-binding fragments thereof), polypeptides, polynucleotides and vectors, are at least partially free of other biological molecules from the cells or cell culture from which they are produced. Such biological molecules include nucleic acids, proteins, other antibodies or antigen-binding fragments, lipids, carbohydrates, or other material such as cellular debris and growth medium. An isolated antigen-binding protein may further be at least partially free of expression system components such as biological molecules from a host cell or of the growth medium thereof. Generally, the term "isolated" is not intended to refer to a complete absence of such biological molecules (*e.g.*, minor or insignificant amounts of impurity may remain) or to an absence of water, buffers, or salts or to components of a pharmaceutical formulation that includes the antigen-binding proteins (*e.g.*, antibodies or antigen-binding fragments).

Exemplary immunoglobulin chains of LEPR agonists which may be used in connection with the present invention are set forth below in Tables A-1 and A-2.

**Table A-1. Nucleotide Sequences Encoding Anti-LEPR Agonist Antibodies and Antigen-Binding Fragments thereof**

| Antibody/Fragment Designation | SEQ ID NOs: | | | | | | | |
|---|---|---|---|---|---|---|---|---|
| | HCVR | HCDR1 | HCDR2 | HCDR3 | LCVR | LCDR1 | LCDR2 | LCDR3 |
| H4H16650P2 | 1 | 3 | 5 | 7 | 9 | 11 | 13 | 15 |
| H4H16679P2 | 17 | 19 | 21 | 23 | 9 | 11 | 13 | 15 |
| H4H17319P2 | 25 | 27 | 29 | 31 | 9 | 11 | 13 | 15 |
| H4H17321P2 | 33 | 35 | 37 | 39 | 9 | 11 | 13 | 15 |
| H4H18417P2 | 41 | 43 | 45 | 47 | 9 | 11 | 13 | 15 |
| H4H18438P2 | 49 | 51 | 53 | 55 | 9 | 11 | 13 | 15 |
| H4H18445P2 | 57 | 59 | 61 | 63 | 9 | 11 | 13 | 15 |
| H4H18446P2 | 65 | 67 | 69 | 71 | 9 | 11 | 13 | 15 |
| H4H18449P2 | 73 | 75 | 77 | 79 | 9 | 11 | 13 | 15 |
| H4H18482P2 | 81 | 83 | 85 | 87 | 89 | 91 | 93 | 95 |
| H4H18487P2 | 97 | 99 | 101 | 103 | 89 | 91 | 93 | 95 |
| H4H18492P2 | 105 | 107 | 109 | 111 | 89 | 91 | 93 | 95 |

**Table A-2. Amino Acid Sequences of Anti-LEPR Agonist Antibodies and Antigen-Binding Fragments thereof**

| Antibody/Fragment Designation | SEQ ID NOs: | | | | | | | |
|---|---|---|---|---|---|---|---|---|
| | HCVR | HCDR1 | HCDR2 | HCDR3 | LCVR | LCDR1 | LCDR2 | LCDR3 |
| H4H16650P2 | 2 | 4 | 6 | 8 | 10 | 12 | 14 | 16 |
| H4H16679P2 | 18 | 20 | 22 | 24 | 10 | 12 | 14 | 16 |
| H4H17319P2 | 26 | 28 | 30 | 32 | 10 | 12 | 14 | 16 |
| H4H17321P2 | 34 | 36 | 38 | 40 | 10 | 12 | 14 | 16 |
| H4H18417P2 | 42 | 44 | 46 | 48 | 10 | 12 | 14 | 16 |
| H4H18438P2 | 50 | 52 | 54 | 56 | 10 | 12 | 14 | 16 |
| H4H18445P2 | 58 | 60 | 62 | 64 | 10 | 12 | 14 | 16 |
| H4H18446P2 | 66 | 68 | 70 | 72 | 10 | 12 | 14 | 16 |
| H4H18449P2 | 74 | 76 | 78 | 80 | 10 | 12 | 14 | 16 |
| H4H18482P2 | 82 | 84 | 86 | 88 | 90 | 92 | 94 | 96 |

(continued)

| Antibody/Fragment Designation | SEQ ID NOs: | | | | | | | |
|---|---|---|---|---|---|---|---|---|
| | HCVR | HCDR1 | HCDR2 | HCDR3 | LCVR | LCDR1 | LCDR2 | LCDR3 |
| H4H18487P2 | 98 | 100 | 102 | 104 | 90 | 92 | 94 | 96 |
| H4H18492P2 | 106 | 108 | 110 | 112 | 90 | 92 | 94 | 96 |

See International Patent Application No. PCT/US2016/056465; publication no. WO2017/066204.

Heavy chain and light chain variable regions of exemplary anti-LEPR agonist antibodies and antigen-binding fragments thereof are set forth below.

H4H16650P2

HCVR (V$_H$)

QVQLVESGGGVVQPGKSLRLSCVASGFTFSSDAMYWVRQAPGKGLEWVAVIYYDGNYQYY
EDSVKGRFTISRDNSQNTLDLQMNSLRVDDTAVYFCARLNWDYWYLDLWGRGTLVTVSS
(SEQ ID NO: 2)

LCVR (V$_L$)

DIQMTQSPSSLSASVGDRVTITCRASQSISSYLNWYQQKPGKAPKLLIYAASSLQSGVPS
RFSGSGSGTDFTLTISSLQPEDFATYYCQQSYSTPPITFGQGTRLEIK
(SEQ ID NO: 10)

H4H16679P2

HCVR

QVQLVESGGGVVQPGRSLRLSCTASGFTFSSYAMYWVRQAPGKGLEWVSVIYYDGSYKYY
ADSVKGRFTISRDNSKNTLYLQMDSLRAEDTAVYYCASYNWNYWYFDFWGRGTLVTVSS
(SEQ ID NO: 18)

LCVR

DIQMTQSPSSLSASVGDRVTITCRASQSISSYLNWYQQKPGKAPKLLIYAASSLQSGVPS
RFSGSGSGTDFTLTISSLQPEDFATYYCQQSYSTPPITFGQGTRLEIK
(SEQ ID NO: 10)

H4H17319P2

HCVR

QVQLVESGGSVVQPGRSLRLSCAASGFTFSTYAMYWVRQTPGKGLEWVAVLYSDGSNKYY
IDSVKGRFTISRDTSTNTLYLQMSSLRADDSALYYCARLNWDYWYFDLWGRGTLVTVSS
(SEQ ID NO: 26)

LCVR

```
DIQMTQSPSSLSASVGDRVTITCRASQSISSYLNWYQQKPGKAPKLLIYAASSLQSGVPS
RFSGSGSGTDFTLTISSLQPEDFATYYCQQSYSTPPITFGQGTRLEIK
```

(SEQ ID NO: 10)

The amino acid sequence for the REGN4461 (mibavademab) heavy chain is:

```
QVQLVESGGSVVQPGRSLRLSCAASGFTFSTYAMYWVRQTPGKGLEWVAVLYSDGSNKYYIDSVKGRFTISRDTS
TNTLYLQMSSLRADDSALYYCARLNWDYWYFDLWGRGTLVTVSSASTKGPSVFPLAPCSRSTSESTAALGCLVKD
YFPEPVTVSWNSGALTSGVHTFPAVLQSSGLYSLSSVVTVPSSSLGTKTYTCNVDHKPSNTKVDKRVESKYGPPC
PPCPAPEFLGGPSVFLFPPKPKDTLMISRTPEVTCVVVDVSQEDPEVQFNWYVDGVEVHNAKTKPREEQFNSTYR
VVSVLTVLHQDWLNGKEYKCKVSNKGLPSSIEKTISKAKGQPREPQVYTLPPSQEEMTKNQVSLTCLVKGFYPSD
IAVEWESNGQPENNYKTTPPVLDSDGSFFLYSRLTVDKSRWQEGNVFSCSVMHEALHNHYTQKSLSLSLGK
```

(SEQ ID NO: 113)

The amino acid sequence for the REGN4461 (mibavademab) light chain is:

```
DIQMTQSPSSLSASVGDRVTITCRASQSISSYLNWYQQKPGKAPKLLIYAASSLQSGVPSRFSGSGSGTDFTLTI
SSLQPEDFATYYCQQSYSTPPITFGQGTRLEIKRTVAAPSVFIFPPSDEQLKSGTASVVCLLNNFYPREAKVQWK
VDNALQSGNSQESVTEQDSKDSTYSLSSTLTLSKADYEKHKVYACEVTHQGLSSPVTKSFNRGEC
```

(SEQ ID NO: 114)

H4H17321P2

HCVR

```
                QVQLVESGGGVVQPGRSLRLSCEASGFSSSDNAMYWVRQAPGKGLEWVSVIYHDGSYKYY
                EDSVKGRFTIARDNSKNTLYLQMNSLRAEDTAVYYCARYNWNHWYFDVWGRGTLVTVSS
```

(SEQ ID NO: 34)

LCVR

```
                DIQMTQSPSSLSASVGDRVTITCRASQSISSYLNWYQQKPGKAPKLLIYAASSLQSGVPS
                RFSGSGSGTDFTLTISSLQPEDFATYYCQQSYSTPPITFGQGTRLEIK
```

(SEQ ID NO: 10)

H4H18417P2

HCVR

```
                QVQLVESGGGVVQPGRSLRLSCAASGFTFSTYGMHWVRQAPGKGLEWVSVISYDESNKYY
                ADSVKGRFTISRDNSKNALYLQMNSLRNEDTAVYYCARDRPFGLVTGWFDPWGQGTLVTV
                SS
```

(SEQ ID NO: 42)

LCVR

DIQMTQSPSSLSASVGDRVTITCRASQSISSYLNWYQQKPGKAPKLLIYAASSLQSGVPS
RFSGSGSGTDFTLTISSLQPEDFATYYCQQSYSTPPITFGQGTRLEIK
(SEQ ID NO: 10)

H4H18438P2

HCVR

QVQLVESGGGVVQPGRSLRLSCAASGFSFNTYGMHWVRQAPGKGLEWVTIIWYDGSIKYY
ADSVKGRFTISRDNSKNTLYLQMNSLRAEDTAVYYCARGGYSGYLYFDYWGQGTLVTVSS
(SEQ ID NO: 50)

LCVR

DIQMTQSPSSLSASVGDRVTITCRASQSISSYLNWYQQKPGKAPKLLIYAASSLQSGVPS
RFSGSGSGTDFTLTISSLQPEDFATYYCQQSYSTPPITFGQGTRLEIK
(SEQ ID NO: 10)

H4H18445P2

HCVR

QVQLQESGPGLVKPSQTLSLTCTVSGGSISSGGDYWSWIRQLPGKGLEWIGYIYYSGSAY
YNPSLKSRGTISIDTSKNQFSLKLTSVTAADTAVYFCVKLRFLEWFLGGWFGPWGQGTLV
TVSS
(SEQ ID NO: 58)

LCVR

DIQMTQSPSSLSASVGDRVTITCRASQSISSYLNWYQQKPGKAPKLLIYAASSLQSGVPS
RFSGSGSGTDFTLTISSLQPEDFATYYCQQSYSTPPITFGQGTRLEIK
(SEQ ID NO: 10)

H4H18446P2

HCVR

EVQLVESGGGLVQPGGSLRLSCAASGFTFSNYGMTWVRQAPGKGLEWVSAITGGGGSTYY
SNSVKGRFTISRDNSKNTVYLRMNSVRAEDTAVYYCAKYKWNFVDDWGQGTTVTVSS
(SEQ ID NO: 66)

LCVR

DIQMTQSPSSLSASVGDRVTITCRASQSISSYLNWYQQKPGKAPKLLIYAASSLQSGVPS
RFSGSGSGTDFTLTISSLQPEDFATYYCQQSYSTPPITFGQGTRLEIK
(SEQ ID NO: 10)

H4H18449P2

HCVR

EVQLVESGGGLVQPGGSLRLSCVASGFTFNKYDMHWVRQTTGKGLEWVSGIDTDGDTYYP
GSVKGRFTISRENAENSLYLQMNGLRVGDTAVYYCARWPWSGFYGAFDIWGQGTMVTVSS

(SEQ ID NO: 74)

LCVR

DIQMTQSPSSLSASVGDRVTITCRASQSISSYLNWYQQKPGKAPKLLIYAASSLQSGVPS
RFSGSGSGTDFTLTISSLQPEDFATYYCQQSYSTPPITFGQGTRLEIK

(SEQ ID NO: 10)

H4H18482P2

HCVR

QVQLQESGPGLVKPSQTLSLTCTVSGGSISSGNYYWNWIRQQPGEGLEWIAYIYHNGVTN
FNPSLKSRLTISVDTSKTQFSLKLRSVTAADTAVYYCARSGSWFENWYFDLWGRGTLVTV
SS

(SEQ ID NO: 82)

LCVR

EIVLTQSPGTLSLSPGERATLSCRASQSVSSSYLAWYQQKPGQAPRLLIYGASSRATGIP
DRFSGSGSGTDFTLTISRLEPEDFAVYYCQQYGSSPWTFGQGTKVEIK

(SEQ ID NO: 90)

H4H18487P2

HCVR

QVQLQESGPGLVKPSETLSLTCTVSGGSISNSYWSWIRQPPGKGLEWIGYVYSRGNTKYN
PSLTSRVTMSFDTSKNQFSLKLRSVTAADTAVYYCARSSSWYEDWYFDLWGRGTLVTVSS

(SEQ ID NO: 98)

LCVR

EIVLTQSPGTLSLSPGERATLSCRASQSVSSSYLAWYQQKPGQAPRLLIYGASSRATGIP
DRFSGSGSGTDFTLTISRLEPEDFAVYYCQQYGSSPWTFGQGTKVEIK

(SEQ ID NO: 90)

H4H18492P2

HCVR

QVQLQQWGAGLFKPSETLSLTCDVYGGSFRGYYWSWIRQPPGKGLEWIGEISYSGFTNYN
PSLKSRVIISIDTSKNQFSLKMSSVTAADTAVYYCARVTYGYGTFDYWGQGTLVTVSS

(SEQ ID NO: 106)

LCVR

```
EIVLTQSPGTLSLSPGERATLSCRASQSVSSSYLAWYQQKPGQAPRLLIYGASSRATGIP

DRFSGSGSGTDFTLTISRLEPEDFAVYYCQQYGSSPWTFGQGTKVEIK
```

(SEQ ID NO: 90)

"H4H16650P2", "H4H16679P2", "H4H17319P2", "H4H17321P2", "H4H18417P2", "H4H18438P2", "H4H18445P2", "H4H18446P2", "H4H18449P2", "H4H18482P2", "H4H18487P2", "H4H18492P2" and "mibavademab," refer to anti-LEPR agonist antibodies and antigen-binding fragments thereof (including multi-specific antigen-binding proteins), comprising the immunoglobulin heavy chain or variable region thereof ($V_H$) of SEQ ID NO: 2, 18, 26, 34, 42, 50, 58, 66, 74, 82, 98 or 106 (or a variant thereof); and the immunoglobulin light chain or variable region thereof ($V_L$) of 10 or 90 (or a variant thereof), as set forth above in Table A; or that comprise a heavy chain or $V_H$ that comprises the CDRs thereof (CDR-H1 (or a variant thereof), CDR-H2 (or a variant thereof) and CDR-H3 (or a variant thereof)) and/or a light chain or $V_L$ that comprises the CDRs thereof (CDR-L1 (or a variant thereof), CDR-L2 (or a variant thereof) and CDR-L3 (or a variant thereof)), e.g., wherein the immunoglobulin chains, variable regions and/or CDRs comprise the specific amino acid sequences described below. In an embodiment of the invention, the $V_H$ is linked to an IgG constant heavy chain domain (e.g., IgG1 or IgG4 or a variant thereof) and/or the $V_L$ is linked to a lambda or kappa constant light chain domain (or a variant thereof).

A "variant" of a polypeptide, such as an immunoglobulin chain (e.g., of mibavademab, H4H16650P2, H4H16679P2, H4H17319P2, H4H17321P2, H4H18417P2, H4H18438P2, H4H18445P2, H4H18446P2, H4H18449P2, H4H18482P2, H4H18487P2 and H4H18492P2 $V_H$, $V_L$, HC or LC), includes a polypeptide comprising an amino acid sequence that is at least about 70-99.9% (e.g., 70, 72, 74, 75, 76, 79, 80, 81, 82, 83, 84, 85, 86, 87, 88, 89, 90, 91, 92, 93, 94, 95, 96, 97, 98, 99, 99.5, 99.9%) identical or similar to a referenced amino acid sequence that is set forth herein (e.g., any of SEQ ID NOs: 2, 10, 18, 26, 34, 42, 50, 58, 66, 74, 82, 90, 98 or 106); when the comparison is performed by a BLAST algorithm wherein the parameters of the algorithm are selected to give the largest match between the respective sequences over the entire length of the respective reference sequences (e.g., expect threshold: 10; word size: 3; max matches in a query range: 0; BLOSUM 62 matrix; gap costs: existence 11, extension 1; conditional compositional score matrix adjustment). A variant may include a polypeptide identical or similar to a referenced amino acid sequence that is set forth herein (e.g., any of SEQ ID NOs: 2, 10, 18, 26, 34, 42, 50, 58, 66, 74, 82, 90, 98 or 106), but having 1, 2, 3, 4, 5, 6, 7, 8, 9, 10 or more mutations. The mutations can include point mutations which are conservative or non-conservative amino acid, substitutions, insertions or deletions.

The following references relate to BLAST algorithms often used for sequence analysis: BLAST ALGORITHMS: Altschul et al. (2005) FEBS J. 272(20): 5101-5109; Altschul, S. F., et al., (1990) J. Mol. Biol. 215:403-410; Gish, W., et al., (1993) Nature Genet. 3:266-272; Madden, T. L., et al., (1996) Meth. Enzymol. 266:131-141; Altschul, S. F., et al., (1997) Nucleic Acids Res. 25:3389-3402; Zhang, J., et al., (1997) Genome Res. 7:649-656; Wootton, J. C., et al., (1993) Comput. Chem. 17:149-163; Hancock, J. M. et al., (1994) Comput. Appl. Biosci. 10:67-70; ALIGNMENT SCORING SYSTEMS: Dayhoff, M. O., et al., "A model of evolutionary change in proteins." in Atlas of Protein Sequence and Structure, (1978) vol. 5, suppl. 3. M. O. Dayhoff (ed.), pp. 345-352, Natl. Biomed. Res. Found., Washington, D.C.; Schwartz, R. M., et al., "Matrices for detecting distant relationships." in Atlas of Protein Sequence and Structure, (1978) vol. 5, suppl. 3." M. O. Dayhoff (ed.), pp. 353-358, Natl. Biomed. Res. Found., Washington, D.C.; Altschul, S. F., (1991) J. Mol. Biol. 219:555-565; States, D. J., et al., (1991) Methods 3:66-70; Henikoff, S., et al., (1992) Proc. Natl. Acad. Sci. USA 89:10915-10919; Altschul, S.F., et al., (1993) J. Mol. Evol. 36:290-300; ALIGNMENT STATISTICS: Karlin, S., et al., (1990) Proc. Natl. Acad. Sci. USA 87:2264-2268; Karlin, S., et al., (1993) Proc. Natl. Acad. Sci. USA 90:5873-5877; Dembo, A., et al., (1994) Ann. Prob. 22:2022-2039; and Altschul, S. F. "Evaluating the statistical significance of multiple distinct local alignments." in Theoretical and Computational Methods in Genome Research (S. Suhai, ed.), (1997) pp. 1-14, Plenum, N.Y.

Exemplary anti-LEPR agonist antibodies and antigen-binding fragments thereof which are used in methods of the present invention are listed in Table A herein. Table A sets forth the amino acid sequence identifiers of the heavy chain variable regions (HCVRs), light chain variable regions (LCVRs), heavy chain complementarity determining regions (HCDR1, HCDR2 and HCDR3), and light chain complementarity determining regions (LCDR1, LCDR2 and LCDR3) of the exemplary anti-LEPR agonist antibodies and antigen-binding fragments.

Disclosed are methods of using anti-LEPR agonist antibodies and antigen-binding fragments thereof comprising an HCVR comprising an amino acid sequence selected from any of the HCVR amino acid sequences listed in Table A; or a variant thereof.

Disclosed are methods of using anti-LEPR agonist antibodies and antigen-binding fragments thereof, comprising an LCVR comprising an amino acid sequence selected from any of the LCVR amino acid sequences listed in Table A; or a variant thereof.

Disclosed are methods of using anti-LEPR agonist antibodies and antigen-binding fragments thereof, comprising an HCVR and an LCVR amino acid sequence pair (HCVR/LCVR) comprising any of the HCVR amino acid sequences listed in Table A; or a variant thereof paired with any of the LCVR amino acid sequences listed in Table A; or a variant thereof. According to certain embodiments, the present invention provides anti-LEPR agonist antibodies and antigen-binding fragments thereof comprising an HCVR/LCVR amino acid sequence pair contained within any of the exemplary antibodies and fragments listed in Table A; or a variant thereof. In certain embodiments, the HCVR/LCVR amino acid sequence pair is selected from the group consisting of SEQ ID NOs:. 2/10; 18/10; 26/10; 34/10; 42/10; 50/10; 58/10; 66/10; 74/10; 82/90; 98/90; and 106/90.

Disclosed is a method of using anti-LEPR agonist antibodies and antigen-binding fragments thereof, comprising a heavy chain CDR1 (HCDR1) comprising an amino acid sequence selected from any of the HCDR1 amino acid sequences listed in Table A; or a variant thereof.

Disclosed is a method of using anti-LEPR agonist antibodies and antigen-binding fragments thereof, comprising a heavy chain CDR2 (HCDR2) comprising an amino acid sequence selected from any of the HCDR2 amino acid sequences listed in Table A; or a variant thereof.

Disclosed is a method of using anti-LEPR agonist antibodies and antigen-binding fragments thereof, comprising a heavy chain CDR3 (HCDR3) comprising an amino acid sequence selected from any of the HCDR3 amino acid sequences listed in Table A; or a variant thereof.

Disclosed is a method of using anti-LEPR agonist antibodies and antigen-binding fragments thereof, comprising a light chain CDR1 (LCDR1) comprising an amino acid sequence selected from any of the LCDR1 amino acid sequences listed in Table A; or a variant thereof.

Disclosed is a method of using anti-LEPR agonist antibodies and antigen-binding fragments thereof, comprising a light chain CDR2 (LCDR2) comprising an amino acid sequence selected from any of the LCDR2 amino acid sequences listed in Table A; or a variant thereof.

Disclosed is method of using anti-LEPR agonist antibodies and antigen-binding fragments thereof, comprising a light chain CDR3 (LCDR3) comprising an amino acid sequence selected from any of the LCDR3 amino acid sequences listed in Table A; or a variant thereof.

Disclosed is a method of using anti-LEPR agonist antibodies and antigen-binding fragments thereof, comprising an HCDR3 and an LCDR3 amino acid sequence pair (HCDR3/LCDR3) comprising any of the HCDR3 amino acid sequences listed in Table A; or a variant thereof, paired with any of the LCDR3 amino acid sequences listed in Table A; or a variant thereof.

Disclosed are methods of using anti-LEPR agonist antibodies and antigen-binding fragments thereof, comprising a set of six CDRs (i.e., HCDR1, HCDR2 and HCDR3 of a HCVR and LCDR1, LCDR2, and LCDR3 of a LCVR) contained within any of the exemplary anti-LEPR antibodies listed in Table A; or a variant thereof. In certain embodiments, the HCDR1, HCDR2, HCDR3, LCDR1, LCDR2, and LCDR3 amino acid sequences set is selected from the group consisting of SEQ ID NOs: 4/6/8/12/14/16; 20/22/24/12/14/16; 28/30/32/12/14/16; 36/38/40/12/14/16; 44/46/48/12/14/16; 52/54/56/12/14/16; 60/62/64/12/14/16; 68/70/72/12/14/16; 76/78/80/12/14/16; 84/86/88/92/94/96; 100/102/104/92/94/96; and 108/110/112/92/94/96.

Disclosed are methods of using anti-LEPR agonist antibodies and antigen-binding fragments thereof, comprising a set of six CDRs (i.e., HCDR1, HCDR2, HCDR3, LCDR1, LCDR2, and LCDR3) contained within an HCVR/LCVR amino acid sequence pair as defined by any of the exemplary antibodies and fragments listed in Table A; or a variant thereof. For example, the present invention includes method of using anti-LEPR agonist antibodies and antigen-binding fragments thereof, comprising the HCDR1, HCDR2, HCDR3, LCDR1, LCDR2, and LCDR3 amino acid sequences set contained within an HCVR/LCVR amino acid sequence pair selected from the group consisting of SEQ ID NOs: 2/10; 18/10; 26/10; 34/10; 42/10; 50/10; 58/10; 66/10; 74/10; 82/90; 98/90; and 106/90. Methods and techniques for identifying CDRs within HCVR and LCVR amino acid sequences are well known in the art and can be used to identify CDRs within the specified HCVR and/or LCVR amino acid sequences disclosed herein. Exemplary conventions that can be used to identify the boundaries of CDRs include, e.g., the Kabat definition, the Chothia definition, and the AbM definition (discussed herein).

Disclosed are methods of using antibodies or antigen-binding fragments, that bind to the same epitope as an antibody or fragment specifically set forth herein (e.g., mibavademab,H4H16650P2, H4H16679P2, H4H17319P2, H4H17321P2, H4H18417P2, H4H18438P2, H4H18445P2, H4H18446P2, H4H18449P2, H4H18482P2, H4H18487P2 and/or H4H18492P2). See International Patent Application Publication No. WO2017/66204.

Disclosed are methods of using antibodies and antigen-binding fragments that compete for binding to LEPR with an antibody or antigen-binding fragment that is specifically set forth herein (e.g., mibavademab, H4H16650P2, H4H16679P2, H4H17319P2, H4H17321P2, H4H18417P2, H4H18438P2, H4H18445P2, H4H18446P2, H4H18449P2, H4H18482P2, H4H18487P2 and/or H4H18492P2). The term "competes" as used herein, refers to an antibody or antigen-binding fragment that binds to an antigen (e.g., LEPR) and inhibits or blocks the binding of another antibody or antigen-binding fragment to the antigen. The term also includes competition between two antibodies or antigen-binding fragments, in both orientations, e.g., where a first antibody that binds and blocks binding of second antibody and vice versa. In certain embodiments, the first antibody or fragment and second antibody or fragment may bind to the same epitope. Alternatively, the first and second antibody or fragment may bind to different, but, for example, overlapping epitopes, wherein binding of one inhibits or blocks the binding of the second antibody or fragment, e.g., via steric hindrance. Competition between antibody or fragment may be measured by methods known in the art, for example, by a real-time, label-free bio-layer interferometry assay. Also, binding competition between anti-LEPR antibody or fragment can be determined using a real time, label-free bio-layer interferometry assay on an Octet RED384 biosensor (Pall ForteBio Corp.). See International Patent Application Publication No. WO2017/66204.

**Partial Lipodystrophy**

Lipodystrophies are a heterogeneous group of disorders characterized by selective partial or generalized loss of adipose tissue. They can be either congenital or acquired in origin, and according to the distribution of adipose tissue loss, lipodystrophies are categorized to localized, partial, or generalized. Familial partial lipodystrophies (FPLDs) are generally dominantly inherited and are characterized by loss of subcutaneous adipose tissue (usually during late childhood or puberty) from the upper and lower extremities as well as from the truncal region. Despite the loss of subcutaneous adipose tissue from the trunk as well as the extremities, the trunk-leg fat ratio is higher in PLD because the trunk contains both subcutaneous as well as deep (visceral) adipose tissue which is not found in the extremities. Common clinical features of patients with lipodystrophies are discussed herein. Limited capacity of adipose tissue to store lipids and failure of buffering post-prandial lipid fluxes have a fundamental role in the pathogenesis of the metabolic disorders associated with lipodystrophies.

Diagnosis of lipodystrophy is frequently challenging, and partial forms may easily be confused with common central obesity and metabolic syndrome. Diagnosis of lipodystrophy is typically based on clinical history, physical examination, and assessment of body composition, with laboratory findings useful in some cases. While no firm diagnostic criteria for lipodystrophy have been established based on skinfold measurements or imaging procedures such as dual-energy X-ray absorptiometry and magnetic resonance imaging, all of these evaluations can assist with diagnosis. Although serum leptin levels in patients with lipodystrophy tend to be low (in absolute levels or relative to body mass index), no defined serum leptin level threshold can be used to rule out the diagnosis of lipodystrophy. Handelsman et al., The Clinical Approach to the Detection of Lipodystrophy - An AACE Consensus Statement, Endocr Pract. 19(1): 107-116 (2013); Araújo-Vilar & Santini, Diagnosis and treatment of lipodystrophy: a step-by-step approach, Journal of Endocrinological Investigation 42: 61-73 (2019).

Familial partial lipodystrophies (FPLD or FPL) are a group of, frequently, monogenic (both autosomal dominant and autosomal recessive) disorders which are characterized by variable loss of subcutaneous (sc) fat from the extremities and trunk, and predisposition to metabolic complications such as insulin resistance, diabetes, dyslipidemia, hepatic steatosis and premature atherosclerosis. See e.g., Hussain & Garg, Lipodystrophy Syndromes, Endocrinology and Metabolism Clinics of North America Volume 45, Issue 4, December 2016, Pages 783-797; and Jéru et al., Clinical Utility Gene Card for: Familial partial lipodystrophy, European Journal of Human Genetics (2016) 25: e1-e5. Amongst the autosomal dominant FPLD, Dunnigan variety (FPLD2) is the most common subtype and is caused by heterozygous missense disease-causing variants in lamin A/C *(LMNA)* gene located on chromosome 1q21(3-6). Other known genes that characterize autosomal dominant FPLD include *PPARG* (FPLD3), *PLIN1* (FPLD4) and *CAV1* (FPLD7). In addition, mutations in several genes have been associated with FPLD but do not have numerical FPLD designation. These include *ADRA2A, PIK3R1, AKT2, ZMPSTE24, PSMB8, WRN, POLD1* and *BLM.*

Genes characterizing autosomal recessive FPLD include *CIDEC* (FPLD5), *LIPE* (FPLD6) and *PCYT1A.* In addition, there are many patients with the clinical presentation of FPLD who do not have disease-causing variants in the known genes and some of them are considered to have a polygenic inheritance which has been named FPLD1 or Kobberling's syndrome. Some individuals with FPLD do not have known mutations, suggesting that additional, as yet unidentified genes can cause the disorder.

Patients with FPLD Type 1, Köbberlings Variety (FPL1) have symptoms that are similar to those seen in FPL2, Dunnigan variety. However, fat loss is generally confined to the arms and legs. Fat loss is usually more prominent on the lower (distal) portions of the arms and legs. Affected individuals have normal or slightly increased fat distribution on the face, neck, and trunk. In addition, some affected individuals may develop excess belly fat (central obesity). Metabolic abnormalities including insulin resistance, high blood pressure (hypertension), and severe hypertriglyceridemia have also been reported. The mode of inheritance of FPL, Köbberlings variety is unknown.

FPL Type 2, Dunnigan Variety (FPL2) is the most common form of FPL. Mutations in *LMNA* have been linked to FPLD2. Affected individuals usually have normal fat distribution during early childhood. However, around the time of puberty, subcutaneous fat in the arms and legs and trunk is gradually lost. In women, the loss of fat may be most striking in the buttocks and hips. At this time, fat may accumulate in other areas of the body including the face, causing a double chin, and the neck and upper back between the shoulder blades, causing a hump. Affected individuals may have a round face similar to individuals with Cushing's syndrome. This characteristic distribution of fat and the overall muscular appearance makes the disorder more easily recognizable in women than men.

Insulin resistance is common in FPLD2 and may be associated with a condition called acanthosis nigricans, a skin condition characterized by abnormally increased coloration (hyperpigmentation) and "velvety" thickening (hyperkeratosis) of the skin, particularly of skin fold regions, such as of the neck and groin and under the arms (axillae). An enlarged liver (hepatomegaly) is also common. Hepatomegaly is caused by the accumulation of fat in the liver (fatty liver or steatosis). Progressive accumulation of fat in the liver can cause scarring and damage to the liver (cirrhosis) and, eventually, liver dysfunction.

Other complications of insulin resistance may occur including glucose intolerance, hypertriglyceridemia, and diabetes. These abnormalities are often very difficult to control and diabetes is often severe. Affected women are at a greater risk of developing diabetes than affected men and often experience more severe metabolic complications (Vigouroux, 2000). Some individuals may experience extreme hypertriglyceridemia, resulting in episodes of acute inflammation of the pancreas (pancreatitis). Pancreatitis, a potentially life-threatening disease, can be associated with abdominal pain, chills, jaundice, weakness, sweating, vomiting, and weight loss.

After puberty, some women with FPLD, e.g., FPL2, may develop polycystic ovary syndrome (PCOS), a complex of symptoms that are not always present in every case. PCOS is often characterized by an imbalance of sex hormones as affected women may have excess androgens, the male hormones in the body. PCOS can result in irregular menstrual periods or a lack of menstruation, oily skin that is prone to acne, cysts on the ovaries, failure of the ovary to release eggs, and mild hirsutism (a male pattern of hair growth). Hair may develop on the upper lip, chin and other parts of the body.

Individuals with FPL, Dunnigan variety are predisposed to coronary artery disease and other types of atherosclerotic vascular disease.

FPLD Type 3 (FPLD3) also has been reported in a small number of individuals. Fat loss is more prominent in the calves and forearms than in the upper arms and thighs. Diabetes, hypertriglyceridemia, hypertension, fatty liver, pancreatitis, and hirsutism have also been reported. Metabolic abnormalities are more prominent than the lipodystrophy in this form of the disorder. FPLD3 is caused by mutations in the *PPARG* gene.

FPLD4 lipodystrophy is most prominent in the lower limbs and buttocks. Muscular hypertrophy may be prominent in the calves. Insulin resistance, severe hypertriglyceridemia, and diabetes were also reported. FPLD4 is caused by mutations in the *PLIN1* gene.

FPLD5 was reported in a family who had severe insulin resistance and diabetes mellitus (George et al., Science 304(5675):1325-1328 (2004)). Insulin resistance can appear around the ages of 20 to 30. Lipodystrophy most prominently affects the arms and legs. FPLD5 has been linked to mutations in the *CIDEC* gene.

FPLD6 is characterized by abnormal subcutaneous fat distribution, with variable excess accumulation of fat in the face, neck, shoulders, axillae, back, abdomen, and pubic region, and reduction in subcutaneous fat of the lower extremities. Progressive adult-onset myopathy is seen in some patients, and there is variable association with diabetes, hypertriglyceridemia, low high-density lipoprotein (HDL) cholesterol, and hepatic steatosis (Zolotov et al., A. Homozygous LIPE mutation in siblings with multiple symmetric lipomatosis, partial lipodystrophy, and myopathy, Am. J. Med. Genet. 173A: 190-194, 2017). FPLD6 is caused by mutations in the *LIPE* gene.

FPLD7 is an autosomal dominant disorder characterized by early-onset cataracts and later onset of spasticity of the lower limbs. FPLD7 has been linked to mutations in the *CAV1* gene. See Berger et al., Familial lipodystrophy associated with neurodegeneration and congenital cataracts. Neurology 58: 43-47, 2002; Cao et al., Heterozygous CAV1 frameshift mutations (MIM 601047) in patients with atypical partial lipodystrophy and hypertriglyceridemia. Lipids Health Dis. 7: 3, 2008 [Electronic Article]; Garg et al., Whole exome sequencing identifies de novo heterozygous CAV1 mutations associated with a novel neonatal onset lipodystrophy syndrome. Am. J. Med. Genet. 167A: 1796-1806, 2015; and Razani et al., Caveolin-1-deficient mice are lean, resistant to diet-induced obesity, and show hypertriglyceridemia with adipocyte abnormalities. J. Biol. Chem. 277: 8635-8647, 2002.

*LIPE* encodes hormone-sensitive lipase, an enzyme highly expressed in adipose tissue responsible for hydrolyzing esters to free fatty acids.

The *LMNA* gene is located on the long arm (q) of chromosome 1 (1q21-q22). The *LMNA* gene encodes the proteins lamin A and lamin C. These proteins are active in the nuclear lamina. Mutations of this gene lead to disruption of the normal functions of lamins A and C which may result in premature cell death of fat cells (adipocytes) in individuals with FPLD2, Dunnigan variety. Mutations of the *LMNA* gene have also been shown to cause a variety of other disorders (allelic disorders) including a form of mandibuloacral dysplasia, a couple forms of Emery-Dreifuss muscular dystrophy, a form of limb-girdle muscular dystrophy, a form of hereditary spastic paraplegia, a form of Charcot-Marie-Tooth disease, a form of dilated cardiomyopathy, Malouf syndrome, and Hutchinson-Gilford progeria syndrome.

The *PPARG* gene is located on the short arm of chromosome 3 (3p25) and encodes the transcription factor, PPAR gamma. PPAR gamma is essential for proper adipocyte cell differentiation. FPLD due to *PPARG* mutations results from improper adipocyte cell differentiation and maintenance.

The *PLIN1* gene is located on the long arm of chromosome 15 (15q26) and encodes for a protein known as perilipin. Perilipin is the most abundant protein coating the surface of lipid droplets where the fat is stored within the adipocytes. Perilipin may be essential for the storage of triglycerides and for the release of fatty acids from lipid droplets. One function of lipid droplets is the storage of lipids.

The *AKT2* gene is located on the long arm of chromosome 19 (19q13.2) and encodes for protein kinase B beta. The protein may play a role in post receptor insulin signaling, an important pathway in adipocyte formation and function.

*CAV1* gene encodes caveolin-1, a protein that serves in the formation of caveolae in plasma membranes. By binding fatty acids on the plasma membrane of the adipocyte and translocating them into lipid droplets, caveolin-1 appears to play a role in lipid droplet formation and adipocyte differentiation.

*PCYT1A* is involved in the phosphatidylcholine synthesis, a major component of cell membranes. Proteasome subunit beta type 8 (PSMB8) encodes a protein responsible for cell homeostasis and its deficiency leads to hypersecretion of interferons, cellular stress and inflammation. *WRN* encodes a helicase that plays an important role in repairing and maintaining the DNA structure. *POLD1* encodes the catalytic subunit of the DNA polymerase delta, an enzyme responsible for DNA repair and stability. *BLM* gene is responsible for the RecQ helicase production, a protein participating in the unwinding of the DNA helix. ADRA2A is the main presynaptic inhibitory feedback G protein-coupled receptor regulating norepinephrine release. Activation of *ADRA2A* inhibits cAMP production and reduces lipolysis in adipocytes.

The *CIDEC* gene is located on the short arm of chromosome 3 (3p25.3) and encodes for the CIDEC protein. CIDEC is expressed in the lipid droplets and plays a role in storage of fat within these structures. Mutation of the *CIDEC* gene may result in low levels of functional CIDEC protein, resulting in lack of ability of lipid droplets to store fat.

In acquired partial lipodystrophy (APL), or Barraquer-Simons syndrome, patients typically develop loss of subcutaneous fat during childhood or adolescence, though onset as late as the fourth or fifth decade of life has been reported. APL is characterized by a progressive loss of subcutaneous fat over months to years from the face, neck, arms, thorax, and upper abdomen. This fat loss typically progresses in a cephalocaudal fashion with sparing of the lower extremities, although the exact pattern of fat loss can vary. Some patients may have excess fat accumulation over the lower abdomen, gluteal region, and legs. Metabolic complications are less common with APL than with other lipodystrophy subtypes. The main cause of morbidity appears to be chronic renal disease (especially membranoproliferative glomerulonephritis). APL has also been associated with a number of autoimmune diseases, including dermatomyositis and systemic lupus erythematosus. Most patients with APL have low levels of serum complement 3 (C3) accompanied by detectable levels of a circulating autoantibody, C3 nephritic factor. APL is also more common in women than in men (estimated 4:1 ratio).

## Treatment and Administration

The present invention includes methods for treating partial lipodystrophy (e.g., FPLD) in a subject that has bene diagnosed as having or likely to have partial lipodystrophy by administering a therapeutically effective dose of LEPR agonist to the subject.

"Treat" or "treating" means to administer a LEPR agonist (e.g., mibavademab, H4H16650P2, H4H16679P2, H4H17319P2, H4H17321P2, H4H18417P2, H4H18438P2, H4H18445P2, H4H18446P2, H4H18449P2, H4H18482P2, H4H18487P2 and H4H18492P2) to a subject, having PLD, such that one or more signs and/or symptoms and/or clinical indicia of the PLD regresses or is eliminated and/or the progression thereof is inhibited (e.g., the disease in the subject is stabilized, reduced or eliminated). Thus, the present invention includes methods which include treating a subject having been determined to have or likely to have PLD with a LEPR agonist wherein one or more signs and/or symptoms and/or clinical indicia of PLD regresses, or is eliminated and/or the progression thereof is inhibited.

An effective or therapeutically effective dose of LEPR agonist for treating or preventing PLD refers to the amount of LEPR agonist sufficient to alleviate one or more signs and/or symptoms of PLD in the treated subject, whether by inducing the regression or elimination of such signs and/or symptoms or by inhibiting the progression of such signs and/or symptoms. In an embodiment of the invention, an effective or therapeutically effective dose of LEPR agonist is an initial intravenous loading dose of 5 mg/kg followed by weekly subcutaneous doses of 250-300 mg.

A subject or patient is a mammal such as a human (e.g., a male or female). In an embodiment of the invention, the subject is Caucasian, black, Mexican, Hispanic, Asian, South East Asian or Korean. In an embodiment of the invention, a subject has been diagnosed as having or as likely to have partial lipodystrophy (e.g., FPLD) by a method which is set forth herein, and/or possesses the diagnostic criteria indicative of having or likely having PLD (e.g., FPLD). For example, in an embodiment of the invention, the subject has (i) total body fat percentage greater than about 36%, and (ii) a trunk/leg fat percent ratio greater than about 1.35; or total body fat percentage less than or equal to about 36%; and (trunk/leg fat percent ratio)-(0.0311 X total body fat percentage) $\geq$ about 0.232; or (i) total body fat percentage greater than about 25.7%, and (ii) trunk/leg fat percent ratio greater than about 1.5; or (i) total body fat percentage less than or equal to about 25.7%, and (ii) (trunk/leg fat percent ratio)-(0.0429 X total body fat percentage) > about 0.4; or (i) total body fat percentage greater than about 27.5%, and (ii) trunk/leg fat percent ratio greater than about 1.5; or (i) total body fat percentage less than or equal to about 27.5%, and (ii) (trunk/leg fat percent ratio)-(0.0429 X total body fat percentage) > about 0.321; or trunk/leg fat percent ratio greater than 1.54 (in a Korean or Asian female) or 1.90 (in a Korean or Asian male). In an embodiment of the invention, the subject has fat percentages indicative of having PLD or as likely having PLD according to the flowchart in Figure 6.

In an embodiment of the invention, the subject also suffers from and/or is diagnosed as having a disease or condition associated with or caused by leptin deficiency or leptin resistance other than partial lipodystrophy, such as obesity, metabolic syndrome, diet-induced food craving, functional hypothalamic amenorrhea, type 1 diabetes, type 2 diabetes, insulin resistance, severe insulin resistance due to mutation in insulin receptor, Alzheimer's disease, leptin deficiency, leptin resistance, Leprechaunism/Donohue syndrome, and Rabson-Mendenhall syndrome.

## Pharmaceutical Compositions and Combinations

The present invention provides methods for using compositions that include LEPR agonists (e.g., anti-LEPR agonist antibodies and antigen-binding fragments thereof) (e.g., mibavademab, H4H16650P2, H4H16679P2, H4H17319P2, H4H17321P2, H4H18417P2, H4H18438P2, H4H18445P2, H4H18446P2, H4H18449P2, H4H18482P2, H4H18487P2 and H4H18492P2) and one or more ingredients.

To prepare pharmaceutical compositions of the LEPR agonists (e.g., mibavademab, H4H16650P2, H4H16679P2, H4H17319P2, H4H17321P2, H4H18417P2, H4H18438P2, H4H18445P2, H4H18446P2, H4H18449P2, H4H18482P2, H4H18487P2 and H4H18492P2), the LEPR agonist is admixed with a pharmaceutically acceptable carrier or excipient. See, e.g., Remington's Pharmaceutical Sciences and U.S. Pharmacopeia: National Formulary, Mack Publishing Company, Easton, Pa. (1984); Hardman, et al. (2001) Goodman and Gilman's The Pharmacological Basis of Therapeutics, McGraw-Hill, New York, N.Y.; Gennaro (2000) Remington: The Science and Practice of Pharmacy, Lippincott, Williams, and Wilkins, New York, N.Y.; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, N.Y. In an embodiment of the invention, the pharmaceutical composition is sterile. The use of such compositions, as discussed herein, are part of the present invention.

Pharmaceutical compositions of the present invention include pharmaceutically acceptable carriers, diluents, excipients and/or stabilizers, such as, for example, water, buffering agents, stabilizing agents, preservatives, isotonifiers, non-ionic detergents, antioxidants and/or other miscellaneous additives.

Disclosed are methods of using desiccated, e.g., freeze-dried, compositions comprising a LEPR agonist (e.g., mibavademab, H4H16650P2, H4H16679P2, H4H17319P2, H4H17321P2, H4H18417P2, H4H18438P2, H4H18445P2, H4H18446P2, H4H18449P2, H4H18482P2, H4H18487P2 or H4H18492P2), or a pharmaceutical composition thereof that includes a pharmaceutically acceptable carrier but substantially lacks water. Such methods may include the step of reconstituting the desiccated composition with an aqueous composition before administering to a subject.

In a further embodiment of the invention a LEPR agonist is administered, in a method of the present invention, in association with a further therapeutic agent. In an embodiment of the invention, the further therapeutic agent is recombinant human leptin; metreleptin; a PCSK9 inhibitor (*e.g.*, an anti-PCSK9 antibody, alirocumab, evolocumab, bococizumab, lodelcizumab, ralpancizumab); an HMG CoA reductase inhibitor (*e.g.*, atorvastatin, rosuvastatin, cerivastatin, pitavastatin, fluvastatin, simvastatin, lovastatin, pravastatin); ezetimibe; insulin; an insulin variant; an insulin secretagogue; metformin; a sulfonylurea; a sodium glucose cotransporter 2 (SGLT2) inhibitor (*e.g.*, dapaglifozin, canaglifozin, empagliflozin); a GLP-1 agonist or analogue (*e.g.*, extendin-4, exenatide, liraglutide, lixisenatide, albiglutide, dulaglutide); a glucagon (GCG) inhibitor (*e.g.*, an anti-GCG antibody); a glucagon receptor (GCGR) inhibitor (*e.g.*, an anti-GCGR antibody, a small molecule GCGR antagonist, a GCGR-specific antisense oligonucleotide; an anti-GCGR aptamer (*e.g.*, a Spiegelmer)); an angiopoietin-like protein (ANGPTL) inhibitor (*e.g.,* an anti-ANGPTL3 antibody, an anti-ANGPTL4 antibody, an anti-ANGPTL8 antibody); phentermine; orlistat; topiramate; bupropion; topiramate & phentermine; bupropion & naltrexone; bupropion & zonisamide; pramlintide & metreleptin; lorcaserin; cetilistat; tesofensine; velneperit; fish oil; pioglitazone; setmelanotide; a fibrate (*e.g.*, fenofibrate); prednisone; niacin; anticonvulsants; digoxin; coumadin; vitamin D; thyroxine; a thyroid supplement; a vitamin supplement; a calcium supplement; carnitine; coenzyme Q10; an anti-constipation medication; an anti-allergic medication; gabapentin; a narcotic; ketamine; lidocaine; and/or venlafaxine hydrochloride. In an embodiment of the invention, the anti-LEPR antibodies of the present invention are not formulated or administered with an anorectic agent.

The mode of administration of a LEPR agonist can vary. Routes of administration include oral, rectal, transmucosal, intestinal, parenteral; intramuscular, subcutaneous, intradermal, intramedullary, intrathecal, direct intraventricular, intravenous, intraperitoneal, intranasal, intraocular, inhalation, insufflation, topical, cutaneous, transdermal or intraarterial.

The term "in association with" indicates that components, a LEPR agonist along with another agent such as metreleptin, can be formulated into a single composition, *e.g.*, for simultaneous delivery, or formulated separately into two or more compositions (*e.g.,* a kit including each component). Each component can be administered to a subject at a different time than when the other component is administered; for example, each administration may be given non-simultaneously (*e.g.*, separately or sequentially) at intervals over a given period of time. Moreover, the separate components may be administered to a subject by the same or by a different route.

**EXAMPLES**

These examples exemplify the present invention are not a limitation thereof. Methods set forth in the Examples form part of the present invention.

**Example 1: Algorithm to Diagnose Partial Lipodystrophy using DXA Scans.**

In this example, development is described of an algorithm using trunk-leg fat ratio that is corrected for total adiposity which has superior test characteristics to using the trunk-leg ratio alone. The algorithm makes use of the insight that, in controls, the trunk-leg ratio is a function of total adiposity: the higher the adiposity, the higher the trunk-leg ratio. This is due to the greater expandability of trunk adiposity compared to leg adipose tissue. The approach was then generalized to applications when very little FPLD patient level data were available as long as there was a large data set of control subjects in the demographic of interest. This algorithm can be used to diagnose FPLD with a positive predictive value of greater than 50% when the prevalence is greater than 0.7% or a ~40-fold enrichment from the general population (See discussion *supra).*

Attempts to use DXA criteria to diagnose FPLD have used static measures such as "Trunk/Leg Fat Percent" greater than a constant (Ajluni N, Meral R, Neidert AH, Brady GF, Buras E, McKenna B, DiPaola F, Chenevert TL, Horowitz JF, Buggs-Saxton C, Rupani AR, Thomas PE, Tayeh MK, Innis JW, Omary MB, Conjeevaram H, Oral EA. Spectrum of disease associated with partial lipodystrophy: lessons from a trial cohort. Clin Endocrinol (Oxf) 2017; 86:698-707) and other attempts have used less static measures (Vasandani C, Li X, Sekizkardes H, Adams-Huet B, Brown RJ, Garg A. Diagnostic Value of Anthropometric Measurements for Familial Partial Lipodystrophy, Dunnigan Variety. J Clin Endocrinol Metab 2020; 105). Others have demonstrated diagnosing PLD based on the examination of images visualizing adipose tissue that are generated by DXA (Meral R, Ryan BJ, Malandrino N, Jalal A, Neidert AH, Muniyappa R, Akinci B, Horowitz JF, Brown RJ, Oral EA. "Fat Shadows" From DXA for the Qualitative Assessment of Lipodystrophy: When a Picture Is Worth a Thousand Numbers. Diabetes Care 2018; 41:2255-2258).

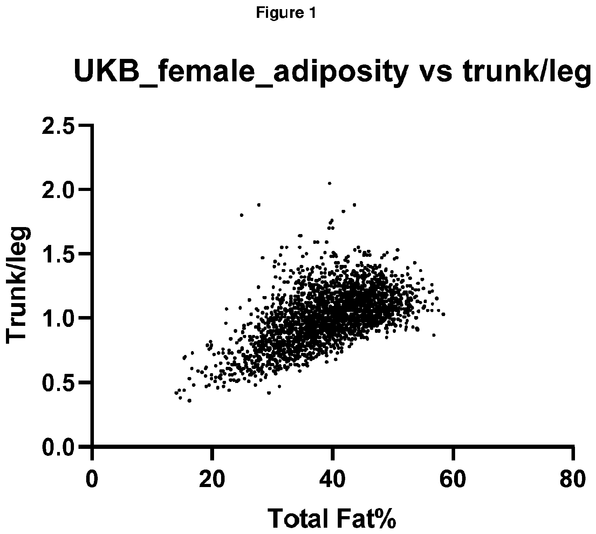

It was realized that the trunk/leg fat percent ratio should not be approached as a static measure, but rather as a function of adiposity. This was evident from plotting the relationship between adiposity and trunk/leg fat percent ratio for a large data set such as UK Biobank. The method was first used for females. It is clear in Figure 1 that, as women's adiposity increases, the trunk/leg fat percent ratio increases. As adiposity in women increased (cross-sectionally across the population) they preferentially add trunk fat as opposed to leg fat. The trunk fat is easier to gain or lose than leg fat. Refer to the adipose expandability hypothesis (Ghaben AL, Scherer PE. Adipogenesis and metabolic health. Nat Rev Mol Cell Biol 2019; 20:242-258). While control females are able to expand trunk fat more readily than FPLD females, FPLD females have much difficulty expanding leg adiposity.

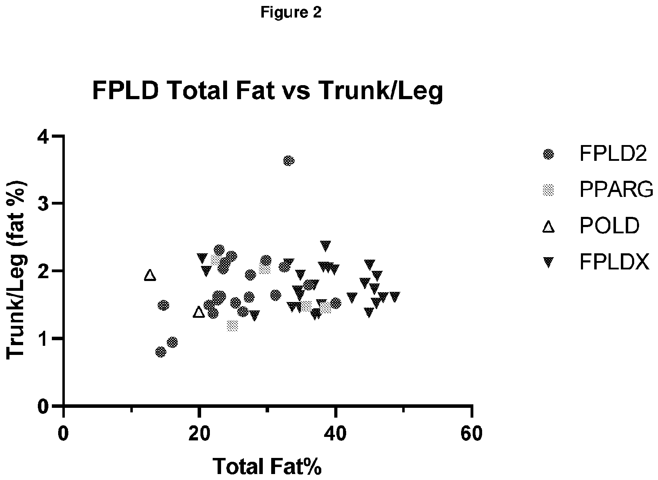

To create a diagnostic algorithm for FPLD, the following steps were taken: A recent paper showing DXA from individuals with FPLD (Meral (2018) (cited herein)) was examined. Individual DXA data from patients with FPLD were copied from Supplementary Materials Table 2 pdf into Excel (Microsoft Corp; Redmond, WA). The FPLD data points were plotted in GraphPad Prism (GraphPad Software; San Diego, CA) (Figure 2).

The data from several cohorts in the Fat Shadows study are set forth in Table 1-1. The Michigan cohort included 48 patients with lipodystrophy (41 female and 7 male, ages 13-65 years), 43 of whom had FPLD, and 70 control subjects (42 female and 28 male, ages 19-65 years). The NIH cohort included 13 patients with GL (generalized lipodystrophy) (9 female, 4 male, ages 14-40 years), 14 patients with FPLD (12 female, 2 male, ages 18-65 years) and 56 control subjects (48 female, 8 male, ages 18-65 years).

**Table 1-1. All subtypes, demographic characteristics, and average body composition characteristics of Michigan and NIH cohorts.**

| p. | Row | Col | Group | Genetic comments | NM | RB | RM | EO | BA | Sex | Race | Hispanic or Latino | Age | BMI | FFMI | Arms %fat | Legs %fat | Trunk %fat | Total %fat | Site | Table | ID |
|---|---|---|---|---|---|---|---|---|---|---|---|---|---|---|---|---|---|---|---|---|---|---|
| 5 | A | 1 | Control | | n | n | n | n | n | F | White | | 58 | 19.5 | 13.8 | 33.6 | 38.6 | 23.1 | 30.7 | NIH | DXA | KFQ39 |
| 5 | A | 2 | Control | | n | n | n | n | n | F | White | | 63 | 19.5 | 15.6 | 21.8 | 26.9 | 16.1 | 21.2 | NIH | DXA | QUN74 |
| 5 | A | 3 | Control | | | | n | n | | F | White | | 44 | 20.2 | 16.5 | 21.5 | 26.2 | 13.2 | 19.7 | NIH | DXA | WQW90 |
| 5 | A | 4 | Control | | n | n | n | n | n | F | White | | 61 | 20.2 | 15.1 | 24.6 | 34.6 | 29.5 | 28.8 | NIH | DXA | XDE96 |
| 5 | A | 5 | Control | | n | n | n | n | n | F | White | | 61 | 20.3 | 15.0 | 30.1 | 33.1 | 24.3 | 27.9 | NIH | DXA | JHD98 |
| 5 | A | 6 | Control | | n | n | n | n | n | F | White | | 40 | 20.5 | 13.4 | 36.2 | 40.4 | 34.4 | 36.2 | NIH | DXA | VZP99 |
| 5 | B | 1 | Control | | n | n | n | n | n | F | White | | 30 | 20.6 | 14.8 | 27.5 | 33.8 | 28.0 | 29.6 | NIH | DXA | OPG61 |
| 5 | B | 2 | Control | | n | n | | | n | F | White | | 21 | 21.3 | 13.7 | 36.5 | 39.4 | 34.3 | 35.6 | UM | Prodigy | IDL57 |
| 5 | B | 3 | Control | | | | n | n | | F | White | | 30 | 21.8 | 17.1 | 19.7 | 25.2 | 21.1 | 22.5 | NIH | DXA | UNQ13 |
| 5 | B | 4 | Control | | n | n | n | n | n | F | White | | 27 | 22.2 | 15.7 | 32.5 | 34.3 | 26.5 | 30.1 | NIH | DXA | YAG44 |
| 5 | B | 5 | Control | | n | n | n | n | n | F | White | | 28 | 22.3 | 15.3 | 28.6 | 32.0 | 25.9 | 28.3 | NIH | DXA | ZDO61 |
| 5 | B | 6 | Control | | n | n | n | n | n | F | White | | 54 | 22.4 | 15.3 | 29.4 | 30.0 | 28.6 | 28.8 | NIH | DXA | BFE14 |
| 5 | C | 1 | Control | | n | n | n | n | n | F | White | | 29 | 22.4 | 15.4 | 31.5 | 31.3 | 35.2 | 32.4 | NIH | DXA | PXF64 |
| 5 | C | 2 | Control | | n | n | n | n | n | F | White | | 26 | 22.4 | 16.0 | 29.6 | 35.6 | 26.2 | 29.6 | NIH | DXA | UYQ24 |
| 5 | C | 3 | Control | | n | n | n | n | n | F | White | | 59 | 22.5 | 17.6 | 28.2 | 33.4 | 13.7 | 23.2 | NIH | DXA | ANF11 |
| 5 | C | 4 | Control | | n | n | n | n | n | F | White | | 23 | 22.8 | 17.1 | 29.1 | 31.1 | 22.6 | 26.9 | NIH | DXA | MII85 |
| 5 | C | 5 | Control | | n | n | n | n | n | F | | Yes | 18 | 22.9 | 13.4 | 45.1 | 48.3 | 41.4 | 42.9 | NIH | DXA | JGD88 |
| 5 | C | 6 | Control | | n | n | n | n | n | F | White | | 19 | 23.0 | 15.9 | 33.6 | 36.8 | 30.2 | 32.6 | NIH | DXA | PVA92 |
| 6 | A | 1 | Control | | n | n | n | n | n | F | White | | 25 | 23.2 | 16.5 | 31.1 | 34.2 | 28.1 | 30.2 | NIH | DXA | ALS73 |
| 6 | A | 2 | Control | | n | n | n | n | n | F | White | | 23 | 23.3 | 17.6 | 28.2 | 31.0 | 22.4 | 26.0 | NIH | DXA | CHN79 |
| 6 | A | 3 | Control | | n | n | n | n | n | F | White | | 28 | 23.4 | 15.0 | 34.5 | 41.6 | 35.3 | 37.1 | NIH | DXA | KIT97 |
| 6 | A | 4 | Control | | | | n | n | | F | White | | 65 | 23.5 | 15.2 | 37.8 | 39.4 | 36.9 | 37.1 | NIH | DXA | LUR41 |
| 6 | A | 5 | Control | | n | n | | | n | F | White | | 19 | 23.6 | 16.5 | 28.3 | 30.6 | 30.7 | 29.9 | UM | Prodigy | LEK63 |
| 6 | A | 6 | Control | | | | n | n | | F | White | | 54 | 24.1 | 17.0 | 24.8 | 36.6 | 27.1 | 30.5 | NIH | DXA | ONQ49 |
| 6 | B | 1 | Control | | n | n | | | n | F | White | | 44 | 24.2 | 15.9 | 38.5 | 33.2 | 35.2 | 34.1 | UM | Prodigy | FJS39 |
| 6 | B | 2 | Control | | n | n | n | n | n | F | White | | 26 | 24.5 | 16.1 | 37.1 | 39.7 | 33.7 | 35.6 | NIH | DXA | IYY32 |
| 6 | B | 3 | Control | | n | n | | | n | F | White | | 35 | 24.6 | 14.6 | 39.6 | 38.9 | 41.8 | 39.5 | UM | Prodigy | NNK49 |
| 6 | B | 4 | Control | | n | n | n | n | n | F | Other | | 28 | 24.9 | 18.5 | 35.8 | 36.6 | 34.2 | 34.4 | NIH | DXA | ZQL51 |
| 6 | B | 5 | Control | | n | n | n | n | n | F | White | | 44 | 25.0 | 15.2 | 40.9 | 40.3 | 41.5 | 40.1 | NIH | DXA | AZA20 |
| 6 | B | 6 | Control | | n | n | n | n | n | F | | Yes | 19 | 27.1 | 15.3 | 45.0 | 47.2 | 44.3 | 44.1 | NIH | DXA | HNJ96 |
| 6 | C | 1 | Control | | n | n | | | n | F | White | No | 63 | 27.5 | 15.4 | 42.4 | 39.5 | 51.7 | 46.1 | UM | Prodigy | AEX51 |
| 6 | C | 2 | Control | | | | | | | F | | | 50 | 29.1 | 16.7 | 37.8 | 32.8 | 52.3 | 44.6 | UM | Prodigy | MXN85 |
| 6 | C | 3 | Control | | n | n | | | n | F | | | 53 | 30.1 | 17.3 | 39.1 | 38.8 | 47.0 | 42.5 | UM | Prodigy | FYG82 |
| 6 | C | 4 | Control | | n | n | | | n | F | White | | 26 | 30.2 | 17.8 | 35.3 | 41.0 | 43.6 | 40.8 | UM | Prodigy | UKQ25 |
| 6 | C | 5 | Control | | | | | | | F | White | | 29 | 30.6 | 16.5 | 41.0 | 48.4 | 47.1 | 45.9 | UM | Prodigy | LMO86 |
| 6 | C | 6 | Control | | n | n | | | y | F | White | No | 68 | 31.2 | 18.3 | 42.5 | 36.9 | 58.7 | 42.7 | UM | Prodigy | IYZ81 |
| 7 | A | 1 | Control | | n | n | | | n | F | White | No | 52 | 31.4 | 18.7 | 41.9 | 39.7 | 44.4 | 41.6 | UM | Prodigy | LJT53 |
| 7 | A | 2 | Control | | n | n | | | n | F | Black | No | 66 | 32.0 | 17.8 | 39.5 | 41.3 | 52.3 | 46.4 | UM | Prodigy | OPI79 |
| 7 | A | 3 | Control | | n | n | | | n | F | White | No | 59 | 32.1 | 19.9 | 37.2 | 35.1 | 44.7 | 40.0 | UM | Prodigy | OOH37 |
| 7 | A | 4 | Control | | n | n | | | n | F | Black | No | 58 | 32.1 | 18.8 | 39.2 | 39.5 | 47.2 | 42.7 | UM | Prodigy | OCF67 |
| 7 | A | 5 | Control | | n | n | n | n | n | F | White | | 46 | 32.2 | 16.2 | 48.3 | 49.6 | 55.5 | 51.1 | NIH | DXA | PXU82 |
| 7 | A | 6 | Control | | n | n | | | n | F | White | | 40 | 32.3 | 18.7 | 39.7 | 37.5 | 47.5 | 42.0 | UM | Prodigy | ANV47 |
| 7 | B | 1 | Control | | n | n | | | n | F | White | | 40 | 32.3 | 18.2 | 38.2 | 38.8 | 48.6 | 43.6 | UM | Prodigy | XNK63 |

# Table 1-1. All subtypes, demographic characteristics, and average body composition characteristics of Michigan and NIH cohorts (Continued).

| p. | Row | Col | Group | Genetic comments | NM | PB | RM | EC | BA | Sex | Race | Hispanic or Latino | Age | BMI | FFMI | Arms %fat | Legs %fat | Trunk %fat | Total %fat | Site | Table | ID |
|---|---|---|---|---|---|---|---|---|---|---|---|---|---|---|---|---|---|---|---|---|---|---|
| 7 | B | 2 | Control | | n | n | | | n | F | White | | 44 | 32.5 | 17.2 | 45.8 | 44.5 | 51.1 | 47.1 | UM | Prodigy | RQK74 |
| 7 | B | 3 | Control | | n | n | | | n | F | White | No | 64 | 32.7 | 18.2 | 42.8 | 44.8 | 48.6 | 45.8 | UM | Prodigy | XFY88 |
| 7 | B | 4 | Control | | n | n | | | n | F | White | No | 61 | 32.8 | 18.7 | 43.8 | 46.8 | 50.5 | 48.0 | UM | Prodigy | STH18 |
| 7 | B | 5 | Control | | n | n | | | n | F | White | No | 63 | 33.6 | 18.7 | 39.6 | 41.1 | 51.4 | 45.9 | UM | Prodigy | SRE71 |
| 7 | B | 6 | Control | | n | n | | | n | F | Black | | 27 | 33.9 | 17.6 | 42.5 | 51.3 | 48.5 | 47.9 | UM | Prodigy | BLR23 |
| 7 | C | 1 | Control | | | | n | n | | F | White | | 55 | 34.0 | 17.6 | 45.4 | 46.6 | 54.9 | 48.5 | NIH | DXA | YHA27 |
| 7 | C | 2 | Control | | n | n | | | n | F | White | | 38 | 34.1 | 17.9 | 45.3 | 51.8 | 46.5 | 47.3 | UM | Prodigy | SIC75 |
| 7 | C | 3 | Control | | n | n | | | n | F | White | | 30 | 34.3 | 17.1 | 41.7 | 53.2 | 51.6 | 50.1 | UM | Prodigy | IVL38 |
| 7 | C | 4 | Control | | n | n | | | n | F | White | | 30 | 34.7 | 19.0 | 46.8 | 45.8 | 48.3 | 45.2 | UM | Prodigy | PAU13 |
| 7 | C | 5 | Control | | n | n | n | n | n | F | White | | 60 | 34.8 | 18.8 | 47.8 | 53.1 | 46.9 | 48.3 | NIH | DXA | VMX81 |
| 7 | C | 6 | Control | | n | n | n | n | n | F | White | | 56 | 35.9 | 16.6 | 52.4 | 58.2 | 57.6 | 54.9 | NIH | DXA | KZI46 |
| 8 | A | 1 | Control | | n | n | | | n | F | White | No | 65 | 36.1 | 19.1 | 41.6 | 44.3 | 53.6 | 48.7 | UM | Prodigy | HUO71 |
| 8 | A | 2 | Control | | n | n | | | n | F | White | No | 59 | 36.2 | 18.4 | 45.4 | 51.8 | 52.3 | 50.4 | UM | Prodigy | GZ388 |
| 8 | A | 3 | Control | | n | n | | | n | F | White | | 39 | 36.2 | 21.8 | 32.8 | 39.6 | 43.2 | 39.8 | UM | Prodigy | WGY35 |
| 8 | A | 4 | Control | | n | n | | | n | F | White | | 24 | 36.3 | 20.0 | 44.1 | 47.0 | 45.2 | 44.9 | UM | Prodigy | LHJ12 |
| 8 | A | 5 | Control | | n | n | | | n | F | White | No | 49 | 36.4 | 21.8 | 44.2 | 40.6 | 46.5 | 43.5 | UM | Prodigy | FCG85 |
| 8 | A | 6 | Control | | n | n | | | n | F | White | | 37 | 36.4 | 19.0 | 43.7 | 40.5 | 54.8 | 47.8 | UM | Prodigy | SAE10 |
| 8 | B | 1 | Control | | n | n | n | n | n | F | White | | 48 | 36.8 | 17.0 | 53.9 | 47.3 | 54.6 | 50.6 | NIH | DXA | KGU10 |
| 8 | B | 2 | Control | | n | n | n | n | n | F | White | | 55 | 37.0 | 17.0 | 58.7 | 54.3 | 58.2 | 55.3 | NIH | DXA | OET15 |
| 8 | B | 3 | Control | | n | n | | | n | F | Black | | 24 | 37.2 | 18.3 | 43.1 | 48.3 | 50.2 | 48.0 | UM | Prodigy | WTY40 |
| 8 | B | 4 | Control | | n | n | n | n | n | F | White | | 35 | 38.1 | 20.1 | 48.9 | 48.1 | 51.2 | 48.6 | NIH | DXA | UOX79 |
| 8 | B | 5 | Control | | n | n | n | n | n | F | | Yes | 20 | 38.3 | 19.3 | 52.9 | 48.8 | 52.2 | 49.8 | NIH | DXA | GZC39 |
| 8 | B | 6 | Control | | n | n | n | n | n | F | White | | 48 | 38.4 | 20.2 | 51.2 | 47.5 | 52.1 | 49.2 | NIH | DXA | NAC28 |
| 8 | C | 1 | Control | | y | y | | | n | F | White | No | 67 | 38.7 | 21.1 | 29.5 | 35.7 | 53.3 | 45.8 | UM | Prodigy | JOL74 |
| 8 | C | 2 | Control | | n | n | | | n | F | White | No | 61 | 38.8 | 19.6 | 51.2 | 44.8 | 56.5 | 52.2 | UM | Prodigy | SDW74 |
| 8 | C | 3 | Control | | n | n | n | n | n | F | White | | 36 | 39.1 | 18.5 | 52.8 | 44.5 | 60.2 | 53.0 | NIH | DXA | WBU60 |
| 8 | C | 4 | Control | | n | n | | | n | F | White | | 25 | 39.4 | 22.8 | 37.3 | 37.4 | 47.7 | 42.0 | UM | Prodigy | IRZ78 |
| 8 | C | 5 | Control | | n | n | | | n | F | Asian | | 29 | 39.5 | 20.8 | 45.0 | 45.5 | 50.9 | 47.3 | UM | Prodigy | PPF17 |
| 8 | C | 6 | Control | | n | n | n | n | n | F | White | | 27 | 39.5 | 18.4 | 48.0 | 55.9 | 56.1 | 54.1 | NIH | DXA | YQT56 |
| 9 | A | 1 | Control | | n | n | | | n | F | Black | | 26 | 39.8 | 21.1 | 47.4 | 48.5 | 47.6 | 46.7 | UM | Prodigy | BMK37 |
| 9 | A | 2 | Control | | n | n | n | n | n | F | White | | 42 | 39.5 | 18.4 | 55.6 | 53.6 | 56.7 | 54.3 | NIH | DXA | CYX68 |
| 9 | A | 3 | Control | | n | n | | | n | F | White | No | 51 | 39.6 | 19.6 | 50.6 | 50.8 | 53.7 | 51.2 | UM | Prodigy | PTP54 |
| 9 | A | 4 | Control | | n | n | | | n | F | White | No | 52 | 40.0 | 20.4 | 43.9 | 52.1 | 52.3 | 50.3 | UM | Prodigy | FNB62 |
| 9 | A | 5 | Control | | n | n | | | n | F | White | No | 64 | 41.2 | 22.1 | 48.6 | 51.4 | 43.5 | 48.3 | UM | Prodigy | VHH82 |
| 9 | A | 6 | Control | | n | n | n | n | n | F | White | | 51 | 41.3 | 20.9 | 52.9 | 46.4 | 53.8 | 50.3 | NIH | DXA | RBU80 |
| 9 | B | 1 | Control | | n | n | | | n | F | Black | No | 61 | 41.3 | 21.9 | 41.7 | 52.3 | 50.4 | 49.1 | UM | Prodigy | KBU80 |
| 9 | B | 2 | Control | | n | n | n | n | n | F | White | | 39 | 42.2 | 19.9 | 49.4 | 50.1 | 58.6 | 53.5 | NIH | DXA | BR238 |
| 9 | B | 3 | Control | | n | n | n | n | n | F | White | | 52 | 42.4 | 21.0 | 51.8 | 53.6 | 52.4 | 51.7 | NIH | DXA | GXH69 |
| 9 | B | 4 | Control | | n | n | n | n | n | F | White | | 38 | 42.4 | 19.8 | 49.1 | 56.3 | 55.4 | 54.0 | NIH | DXA | PTE72 |
| 9 | B | 5 | Control | | n | n | n | n | n | F | White | | 50 | 42.5 | 21.4 | 46.1 | 45.2 | 57.0 | 51.0 | NIH | DXA | TLI73 |
| 9 | B | 6 | Control | | n | n | | | n | F | White | No | 54 | 42.9 | 21.1 | 55.5 | 54.1 | 53.7 | 53.1 | UM | Prodigy | ATB52 |
| 9 | C | 1 | Control | | n | n | n | n | n | F | White | | 41 | 43.0 | 19.5 | 53.2 | 52.0 | 60.8 | 55.5 | NIH | DXA | PMK61 |
| 9 | C | 2 | Control | | n | n | n | n | n | F | White | | 53 | 44.3 | 21.0 | 48.4 | 48.3 | 58.6 | 53.4 | NIH | DXA | UUY97 |

EP 4 294 257 B1

# Table 1-1. All subtypes, demographic characteristics, and average body composition characteristics of Michigan and NIH cohorts (Continued).

| p. | Row | Col | Group | Genetic comments | NM | RB | RW | EC | BA | Sex | Race | Hispanic or Latino | Age | BMI | FFMI | Arms %fat | Legs %fat | Trunk %fat | Total %fat | Site | Table | ID |
|----|-----|-----|-------|------------------|----|----|----|----|----|-----|------|--------------------|-----|-----|------|-----------|-----------|-----------|-----------|------|-------|-----|
| 9 | C | 3 | Control | | n | n | n | n | n | F | | Yes | 19 | 44.3 | 20.9 | 54.1 | 54.3 | 56.9 | 54.3 | NIH | DXA | ZRO74 |
| 9 | C | 4 | Control | | n | y | | | n | F | White | No | 57 | 44.7 | 24.0 | 44.6 | 38.4 | 53.0 | 47.6 | UM | Prodigy | MTZ18 |
| 9 | C | 5 | Control | | n | n | n | n | n | F | White | | 50 | 45.5 | 22.2 | 52.8 | 58.7 | 55.1 | 52.1 | NIH | DXA | YQJ68 |
| 9 | C | 6 | Control | | n | n | n | n | n | F | White | | 32 | 46.1 | 22.6 | 51.7 | 47.8 | 58.7 | 52.7 | NIH | DXA | VIJN71 |
| 10 | A | 1 | Control | | n | n | | | n | M | White | | 30 | 22.2 | 18.2 | 16.0 | 17.2 | 20.8 | 18.5 | UM | Prodigy | EJO49 |
| 10 | A | 2 | Control | | n | n | | | n | M | White | | 20 | 22.8 | 18.6 | 14.5 | 19.1 | 19.4 | 18.6 | UM | Prodigy | EWG59 |
| 10 | A | 3 | Control | | n | n | n | n | n | M | White | | 62 | 23.5 | 19.4 | 19.5 | 21.0 | 17.4 | 19.1 | NIH | DXA | WIU51 |
| 10 | A | 4 | Control | | n | n | ? | n | n | M | White | | 64 | 24.4 | 19.1 | 17.9 | 21.7 | 26.5 | 23.4 | NIH | DXA | LGD67 |
| 10 | A | 5 | Control | | n | n | n | n | n | M | White | | 54 | 24.5 | 18.3 | 20.8 | 22.1 | 31.5 | 26.8 | NIH | DXA | WVT82 |
| 10 | A | 6 | Control | | n | n | n | n | n | M | White | | 48 | 24.8 | 18.6 | 20.7 | 27.6 | 27.4 | 26.4 | NIH | DXA | ISLF38 |
| 10 | B | 1 | Control | | n | n | n | n | n | M | White | | 49 | 25.3 | 19.1 | 21.0 | 21.8 | 31.1 | 26.0 | NIH | DXA | ISLF38 |
| 10 | B | 2 | Control | | n | n | | | n | M | White | No | 65 | 27.1 | 19.3 | 22.8 | 25.3 | 36.4 | 30.7 | UM | Prodigy | BLC25 |
| 10 | B | 3 | Control | | y | y | | | n | M | | No | 58 | 27.6 | 20.6 | 25.6 | 18.0 | 38.2 | 28.6 | UM | Prodigy | VGB36 |
| 10 | B | 4 | Control | | n | n | | | n | M | White | No | 65 | 28.5 | 18.1 | 25.5 | 25.9 | 39.1 | 32.6 | UM | Prodigy | AMK51 |
| 10 | B | 5 | Control | | n | y | | | n | M | White | No | 62 | 29.3 | 20.6 | 22.9 | 20.3 | 41.4 | 32.1 | UM | Prodigy | EVP48 |
| 10 | B | 6 | Control | | n | y | | | n | M | White | No | 67 | 29.3 | 19.7 | 30.7 | 26.1 | 42.3 | 35.4 | UM | Prodigy | YBG67 |
| 10 | C | 1 | Control | | n | n | n | n | n | M | White | | 63 | 30.0 | 23.0 | 19.5 | 18.5 | 29.4 | 24.5 | NIH | DXA | MEB58 |
| 10 | C | 2 | Control | | n | n | | | n | M | White | No | 60 | 30.1 | 20.2 | 24.3 | 27.7 | 40.4 | 33.4 | UM | Prodigy | TLP75 |
| 10 | C | 3 | Control | | n | y | | | n | M | White | No | 24 | 31.0 | 23.6 | 18.4 | 17.0 | 33.5 | 25.8 | UM | Prodigy | GNA53 |
| 10 | C | 4 | Control | | n | n | | | n | M | White | | 58 | 31.1 | 18.5 | 29.7 | 28.4 | 47.6 | 38.4 | UM | Prodigy | BZY56 |
| 10 | C | 5 | Control | | n | y | | | n | M | Asian | No | 44 | 31.5 | 22.3 | 25.3 | 22.4 | 36.8 | 30.4 | UM | Prodigy | YBV41 |
| 10 | C | 6 | Control | | n | y | | | n | M | Black | No | 58 | 31.8 | 20.5 | 29.8 | 31.2 | 43.7 | 37.2 | UM | Prodigy | WJY11 |
| 11 | A | 1 | Control | | y | y | | | y | M | White | No | 55 | 32.5 | 22.3 | 24.3 | 24.7 | 38.7 | 32.6 | UM | Prodigy | MRP51 |
| 11 | A | 2 | Control | | n | n | | | n | M | | | 60 | 32.6 | 20.3 | 31.4 | 33.7 | 44.3 | 38.4 | UM | Prodigy | YBS75 |
| 11 | A | 3 | Control | | n | n | | | n | M | White | No | 62 | 33.0 | 21.1 | 33.8 | 28.3 | 44.0 | 37.2 | UM | Prodigy | OOG38 |
| 11 | A | 4 | Control | | y | y | | | n | M | White | No | 52 | 33.1 | 23.6 | 25.7 | 28.4 | 37.8 | 30.3 | UM | Prodigy | JGP42 |
| 11 | A | 5 | Control | | n | n | | | n | M | Black | | 32 | 34.2 | 22.3 | 27.6 | 28.4 | 41.8 | 34.6 | UM | Prodigy | PZR33 |
| 11 | A | 6 | Control | | n | n | | | n | M | White | No | 64 | 35.5 | 20.3 | 32.4 | 33.6 | 50.0 | 43.4 | UM | Prodigy | LCL49 |
| 11 | B | 1 | Control | | n | n | | | n | M | White | | 24 | 35.6 | 22.3 | 31.7 | 29.3 | 44.2 | 37.1 | UM | Prodigy | NEX71 |
| 11 | B | 2 | Control | | n | n | | | n | M | Black | | 22 | 36.0 | 22.0 | 32.9 | 39.9 | 41.0 | 38.6 | UM | Prodigy | QVG22 |
| 11 | B | 3 | Control | | n | n | | | n | M | White | No | 65 | 36.1 | 20.1 | 35.4 | 42.0 | 50.5 | 45.2 | UM | Prodigy | MUW85 |
| 11 | B | 4 | Control | | n | n | | | n | M | White | | 35 | 36.4 | 21.0 | 36.1 | 34.7 | 48.1 | 42.3 | UM | Prodigy | AUL87 |
| 11 | B | 5 | Control | | n | n | | | n | M | White | No | 57 | 36.8 | 23.3 | 31.8 | 30.5 | 45.3 | 38.0 | UM | Prodigy | KQP47 |
| 11 | B | 6 | Control | | n | y | | | n | M | White | No | 44 | 37.6 | 22.6 | 32.4 | 32.9 | 46.8 | 40.0 | UM | Prodigy | TRV14 |
| 11 | C | 1 | Control | | n | n | | | n | M | White | No | 57 | 37.7 | 24.2 | 26.7 | 33.1 | 42.8 | 37.4 | UM | Prodigy | XUT81 |
| 11 | C | 2 | Control | | n | n | | | n | M | White | No | 62 | 40.4 | 23.9 | 31.8 | 37.6 | 45.1 | 41.0 | UM | Prodigy | HVL82 |
| 11 | C | 3 | Control | | n | n | n | n | n | M | White | | 47 | 40.9 | 22.9 | 34.4 | 37.3 | 52.6 | 45.6 | NIH | DXA | BKD94 |
| 11 | C | 4 | Control | | n | n | | | n | M | White | | 64 | 41.0 | 27.1 | 30.5 | 28.6 | 42.8 | 35.1 | UM | Prodigy | KQO60 |
| 11 | C | 5 | Control | | n | n | n | n | n | M | White | | 44 | 41.7 | 24.1 | 39.2 | 32.1 | 52.7 | 44.1 | NIH | DXA | IBA47 |
| 11 | C | 6 | Control | | n | n | | | n | M | White | No | 60 | 43.1 | 23.6 | 40.8 | 45.3 | 56.2 | 48.5 | UM | Prodigy | LAJ54 |
| 12 | A | 1 | CGL1 | AGPAT2 | | | | | | F | White | | 33 | 16.8 | 15.7 | 9.4 | 7.2 | 4.2 | 6.9 | NIH | DXA | VDF29 |
| 12 | A | 2 | CGL1 | AGPAT2 | | | | | | F | | Yes | 14 | 18.6 | 16.3 | 18.1 | 16.4 | 9.6 | 13.6 | NIH | DXA | MCN25 |
| 12 | A | 3 | CGL1 | AGPAT2 | | | | | | F | | Yes | 33 | 18.7 | 17.2 | 13.4 | 8.9 | 6.7 | 9.3 | NIH | DXA | IFF99 |

EP 4 294 257 B1

# Table 1-1. All subtypes, demographic characteristics, and average body composition characteristics of Michigan and NIH cohorts (Continued).

| p. | Row | Col | Group | Genetic comments | NM | RB | RM | EC | BA | Sex | Race | Hispanic or Latino | Age | BMI | FFMI | Arms %fat | Legs %fat | Trunk %fat | Total %fat | Site | Table | ID |
|---|---|---|---|---|---|---|---|---|---|---|---|---|---|---|---|---|---|---|---|---|---|---|
| 12 | A | 4 | CGL1 | AGPAT2 | | | | | | F | White | | 32 | 19.9 | 19.2 | 4.2 | 4.6 | 4.8 | 5.2 | NIH | DXA | HSE26 |
| 12 | A | 5 | CGL1 | AGPAT2 | | | | | | F | | Yes | 20 | 26.9 | 19.0 | 11.5 | 10.5 | 10.0 | 11.2 | NIH | DXA | JRB21 |
| 12 | A | 6 | CGL1 | AGPAT2 | | | | | | F | Black | | 20 | 25.4 | 23.7 | 7.0 | 7.8 | 6.8 | 7.5 | NIH | DXA | YSH16 |
| 12 | B | 1 | CGL1 | AGPAT2 | | | | | | M | Black | | 25 | 24.9 | 23.3 | 6.4 | 7.6 | 5.3 | 7.3 | NIH | DXA | XHD64 |
| 12 | B | 2 | CGL2 | Presumed BSCL2. Gene sequence information not available | | | | | | F | White | | 14 | 18.2 | 17.1 | 10.8 | 8.0 | 8.3 | 8.4 | NIH | DXA | VX168 |
| 12 | B | 3 | CGL2 | BSCL2 | | | | | | M | Asian | | 16 | 16.6 | 15.4 | 11.1 | 10.0 | 6.0 | 9.0 | NIH | DXA | UIW42 |
| 12 | B | 4 | CGL2 | BSCL2 | | | | | | M | White | | 18 | 24.2 | 22.9 | 8.4 | 8.1 | 5.3 | 7.2 | NIH | DXA | GEW39 |
| 12 | B | 5 | GL LMNA p.T10-linked | LMNA p.T10? Sekinoz et al (19), Hussain et al (20) | | | | | | F | Black | No | 13 | 14.7 | 13.5 | 10.2 | 11.0 | 5.4 | 8.8 | UM | Prodigy | CXI19 |
| 12 | B | 6 | CGL PCYT1A-linked | PCYT1A Payne et al (21) | | | | | | F | White | No | 27 | 14.4 | 13.4 | 8.9 | 7.5 | 7.4 | 8.9 | NIH | DXA | HKV51 |
| 12 | C | 1 | AGL | | | | | | | F | White | | 40 | 17.0 | 16.0 | 12.1 | 7.3 | 4.5 | 7.3 | NIH | DXA | PBN25 |
| 12 | C | 2 | AGL | | | | | | | F | White | No | 19 | 21.3 | 20.0 | 5.7 | 6.6 | 5.1 | 6.7 | UM | Prodigy | AFC45 |
| 12 | C | 3 | AGL | | | | | | | M | | Yes | 20 | 18.6 | 17.8 | 7.4 | 7.4 | 4.3 | 6.8 | NIH | DXA | FNN85 |
| 12 | C | 4 | AGL | | | | | | | M | White | No | 15 | 19.2 | 13.3 | 6.1 | 6.1 | 6.7 | 8.4 | UM | Prodigy | KAC12 |
| 13 | A | 1 | FPLD2 | LMNA, non-hotspot | y | y | | | y | F | | No | 33 | 21.5 | 18.5 | 14.3 | 15.5 | 12.5 | 14.3 | UM | Prodigy | ONR25 |
| 13 | A | 2 | FPLD2 | LMNA, non-hotspot | | | | | | F | | Yes | 15 | 21.5 | 18.4 | 17.1 | 15.9 | 15.0 | 16.0 | NIH | DXA | ZLX34 |
| 13 | A | 3 | FPLD2 | LMNA, non-hotspot Ajluni et al (8) | y | y | | | y | F | White | No | 37 | 27.2 | 18.6 | 37.4 | 11.2 | 40.7 | 33.1 | UM | Prodigy | DSC40 |
| 13 | A | 4 | FPLD2 | LMNA p.R482L Ajluni et al (22) | y | y | | | y | F | White | No | 59 | 20.5 | 15.6 | 16.8 | 14.4 | 30.6 | 23.8 | UM | Prodigy | BZA67 |
| 13 | A | 5 | FPLD2 | LMNA p.R482L Ajluni et al (22) | y | y | | | y | F | White | No | 30 | 31.6 | 25.1 | 21.6 | 16.2 | 26.4 | 22.7 | UM | Prodigy | ILG65 |
| 13 | A | 6 | FPLD2 | LMNA p.R482Q Ajluni et al (8) | y | y | | | y | F | | | 59 | 21.3 | 17.1 | 17.5 | 14.4 | 29.3 | 23.5 | UM | Prodigy | YFQ93 |
| 13 | B | 1 | FPLD2 | LMNA p.R482Q Ajluni et al (22) | y | y | | | y | F | White | | 55 | 22.7 | 17.1 | 20.9 | 20.1 | 32.4 | 27.3 | UM | Prodigy | WQF13 |
| 13 | B | 2 | FPLD2 | LMNA p.R482Q Ajluni et al (22) | y | y | | | y | F | White | No | 42 | 24.0 | 19.7 | 15.5 | 16.8 | 25.1 | 21.4 | UM | Prodigy | PLU27 |
| 13 | B | 3 | FPLD2 | LMNA p.R482Q Ajluni et al (22) | y | y | | | y | F | White | No | 49 | 24.5 | 17.6 | 24.7 | 17.8 | 38.4 | 29.8 | UM | Prodigy | FAW62 |
| 13 | B | 4 | FPLD2 | LMNA p.R482Q Ajluni et al (8) | y | y | | | y | F | White | No | 57 | 24.9 | 19.6 | 15.0 | 12.8 | 29.6 | 22.9 | UM | Prodigy | OZF80 |
| 13 | B | 5 | FPLD2 | LMNA p.R482Q Ajluni et al (8) | y | y | | | y | F | | | 48 | 25.3 | 18.7 | 22.6 | 17.6 | 34.2 | 27.5 | UM | Prodigy | BIA63 |
| 13 | B | 6 | FPLD2 | LMNA p.R482Q Ajluni et al (22) | y | y | | | y | F | White | | 48 | 25.9 | 20.8 | 24.7 | 22.8 | 37.4 | 31.2 | UM | Prodigy | VKF34 |
| 13 | C | 1 | FPLD2 | LMNA p.R482Q Ajluni et al (22) | y | y | | | y | F | White | | 48 | 32.9 | 21.6 | 28.5 | 24.4 | 43.7 | 36.0 | UM | Prodigy | ZYS55 |
| 13 | C | 2 | FPLD2 | LMNA p.R482Q | y | y | | | y | F | White | No | 38 | 33.6 | 24.6 | 22.4 | 21.3 | 29.8 | 26.4 | UM | Prodigy | TS887 |
| 13 | C | 3 | FPLD2 | LMNA p.R482Q Ajluni et al (22) | y | y | | | y | F | White | No | 62 | 38.1 | 23.1 | 31.9 | 30.5 | 46.5 | 40.0 | UM | Prodigy | SZK30 |
| 13 | C | 4 | FPLD2 | LMNA p.R482W | y | y | y | y | y | F | White | | 38 | 23.8 | 20.7 | 13.6 | 10.8 | 16.1 | 14.7 | NIH | DXA | VH121 |
| 13 | C | 5 | FPLD2 | LMNA p.R482W Ajluni et al (8) | | | | | | F | White | No | 41 | 26.1 | 19.9 | 22.7 | 18.7 | 28.6 | 25.3 | UM | Prodigy | BBB10 |
| 13 | C | 6 | FPLD2 | LMNA p.R482W | y | y | y | y | y | F | White | | 19 | 26.6 | 20.7 | 22.7 | 16.5 | 26.8 | 23.1 | NIH | DXA | NXH65 |
| 14 | A | 1 | FPLD2 | LMNA, non-hotspot | y | y | | | y | F | White | No | 58 | 23.2 | 18.3 | 14.6 | 18.4 | 25.3 | 22.0 | UM | Prodigy | OBM76 |
| 14 | A | 2 | FPLD2 | LMNA, non-hotspot Ajluni et al (22) | | | | | | F | White | No | 45 | 27.7 | 18.6 | 27.4 | 19.4 | 39.9 | 32.5 | UM | Prodigy | YZX31 |
| 14 | A | 3 | FPLD2 | LMNA, non-hotspot Ajluni et al (8) | y | y | | | y | F | White | No | 33 | 19.9 | 15.4 | 22.8 | 14.1 | 31.3 | 24.7 | UM | Prodigy | GUX67 |
| 14 | A | 4 | FPLD2 | LMNA, variant to be verified | y | y | y | y | y | F | White | | 18 | 26.5 | 20.7 | 19.8 | 17.0 | 28.7 | 22.7 | NIH | DXA | XBM73 |
| 14 | A | 5 | FPLD2 | LMNA p.R482Q | y | y | | | y | M | White | No | 48 | 37.8 | 27.8 | 25.7 | 18.3 | 32.9 | 27.3 | UM | Prodigy | ZRZ36 |
| 15 | A | 1 | FPLDX | PPARG p.A261V, FPLD3 by OMIM, MITER probability of pathogenicity=85.7%, Ajluni et al | n | n | | | n | F | White | | 64 | 31.7 | 19.9 | 33.5 | 30.6 | 44.6 | 38.5 | UM | Prodigy | GEJ25 |
| 15 | A | 2 | FPLDX | PPARG p.C161V, FPLD3 by OMIM, MITER probability of pathogenicity=86.7% | n | y | | | n | F | White | Yes | 34 | 25.9 | 20.0 | 24.7 | 22.5 | 26.7 | 24.9 | UM | Prodigy | CHD38 |
| 15 | A | 3 | FPLDX | PPARG, FPLD3 by OMIM | y | y | y | y | y | F | White | | 27 | 23.6 | 18.6 | 19.5 | 13.0 | 28.1 | 22.5 | NIH | DXA | QG350 |
| 15 | A | 4 | FPLDX | PPARG, FPLD3 by OMIM | | | y | y | | F | White | | 54 | 24.2 | 17.4 | 24.4 | 18.3 | 37.3 | 28.6 | NIH | DXA | LTP90 |

EP 4 294 257 B1

## Table 1-1. All subtypes, demographic characteristics, and average body composition characteristics of Michigan and NIH cohorts (Continued).

| p. | Row | Col | Group | Genetic comments | NM | RB | RM | EO | BA | Sex | Race | Hispanic or Latino | Age | BMI | FFMI | Arms %fat | Legs %fat | Trunk %fat | Total %fat | Site | Table | ID |
|---|---|---|---|---|---|---|---|---|---|---|---|---|---|---|---|---|---|---|---|---|---|---|
| 15 | A | 5 | FPLDX | PPARG, FPLD3 by OMIM | y | y | y | y | y | F | White | | 51 | 28.6 | 17.9 | 39.5 | 27.2 | 40.3 | 35.7 | NIH | DXA | XNN72 |
| 15 | A | 6 | FPLDX | PPARG, p.E352K, FPLD3 by OMIM, MITER probability of pathogenicity=98.2% | y | y | | | n | M | White | No | 52 | 28.4 | 22.9 | 15.5 | 18.8 | 23.1 | 20.9 | UM | Prodigy | WDA15 |
| 15 | B | 1 | FPLDX | POLD1 p.E1067K Aljuni et al (8) | n | n | | | y | F | White | Yes | 34 | 17.8 | 15.5 | 15.6 | 7.9 | 15.4 | 12.7 | UM | Prodigy | IGX98 |
| 15 | B | 2 | FPLDX | POLD1 p.E1067K Aljuni et al (8) | | | | | | F | White | Yes | 14 | 18.3 | 15.2 | 25.9 | 16.0 | 22.4 | 19.9 | UM | Prodigy | HDI83 |
| 15 | B | 3 | FPLDX | | | | | | | F | White | No | 12 | 17.7 | 14.2 | 18.1 | 13.0 | 25.9 | 21.0 | UM | Prodigy | IDY52 |
| 15 | B | 4 | FPLDX | | y | y | | | n | F | White | No | 54 | 24.0 | 15.5 | 36.9 | 30.6 | 41.4 | 36.9 | UM | Prodigy | CES33 |
| 15 | B | 5 | FPLDX | | y | y | y | y | n | F | White | | 45 | 26.0 | 18.9 | 30.0 | 23.0 | 30.6 | 28.1 | NIH | DXA | NLA27 |
| 15 | B | 6 | FPLDX | | y | y | y | y | y | F | White | | 47 | 26.7 | 18.2 | 28.6 | 18.7 | 41.4 | 33.1 | NIH | DXA | CFT80 |
| 15 | C | 1 | FPLDX | | | | | | | F | White | | 18 | 26.8 | 17.8 | 26.0 | 22.9 | 44.4 | 34.8 | NIH | DXA | PWR43 |
| 15 | C | 2 | FPLDX | | n | y | | | n | F | White | No | 45 | 27.0 | 18.1 | 29.8 | 34.4 | 41.5 | 34.4 | UM | Prodigy | PKT82 |
| 15 | C | 3 | FPLDX | | y | y | | | y | F | White | No | 33 | 27.8 | 17.1 | 35.8 | 23.8 | 46.0 | 39.8 | UM | Prodigy | MTJ87 |
| 15 | C | 4 | FPLDX | | y | y | | | y | F | White | No | 63 | 28.0 | 15.8 | 42.7 | 31.4 | 54.4 | 45.7 | UM | Prodigy | ECD84 |
| 15 | C | 5 | FPLDX | | y | y | | | y | F | Black | | 23 | 30.2 | 24.5 | 17.7 | 11.6 | 25.3 | 20.4 | UM | Prodigy | ZQB78 |
| 15 | C | 6 | FPLDX | | y | y | y | y | y | F | White | | 41 | 30.3 | 18.9 | 38.5 | 19.8 | 46.8 | 38.5 | NIH | DXA | YSD77 |
| 16 | A | 1 | FPLDX | | | | | | | F | White | No | 13 | 30.5 | 19.7 | 37.9 | 31.0 | 42.4 | 37.5 | UM | Prodigy | GPV67 |
| 16 | A | 2 | FPLDX | | n | n | | | n | F | White | No | 63 | 30.7 | 18.3 | 41.3 | 30.6 | 48.9 | 42.4 | UM | Prodigy | NEG22 |
| 16 | A | 3 | FPLDX | | y | y | | | y | F | White | No | 58 | 31.0 | 17.9 | 37.1 | 28.4 | 54.6 | 46.1 | UM | Prodigy | EQN50 |
| 16 | A | 4 | FPLDX | | n | n | n | n | n | F | White | | 54 | 31.0 | 17.2 | 43.8 | 37.1 | 51.3 | 44.9 | NIH | DXA | UTU82 |
| 16 | A | 5 | FPLDX | | y | y | | | y | F | White | No | 58 | 31.0 | 20.6 | 32.7 | 24.9 | 40.6 | 34.7 | UM | Prodigy | XQQ50 |
| 16 | A | 6 | FPLDX | | y | y | | | y | F | White | No | 39 | 31.1 | 21.2 | 36.9 | 28.2 | 38.4 | 33.6 | UM | Prodigy | TSZ36 |
| 16 | B | 1 | FPLDX | | y | y | y | y | y | F | White | | 29 | 31.6 | 19.8 | 36.9 | 23.4 | 47.9 | 38.9 | NIH | DXA | YGE23 |
| 16 | B | 2 | FPLDX | | | | | | | F | White | No | 57 | 32.1 | 17.9 | 45.5 | 35.1 | 56.5 | 48.7 | UM | Prodigy | AFP45 |
| 16 | B | 3 | FPLDX | | y | y | | | y | F | | No | 51 | 32.7 | 18.9 | 29.1 | 28.9 | 52.5 | 44.3 | UM | Prodigy | BPZ32 |
| 16 | B | 4 | FPLDX | | y | y | | | y | F | White | No | 53 | 35.8 | 22.5 | 36.6 | 28.9 | 43.4 | 37.5 | UM | Prodigy | MDW89 |
| 16 | B | 5 | FPLDX | | y | y | | | y | F | Other | Yes | 65 | 37.0 | 24.1 | 33.8 | 27.2 | 39.7 | 34.7 | UM | Prodigy | RNQ70 |
| 16 | B | 6 | FPLDX | | y | y | y | y | y | F | White | | 39 | 37.8 | 21.1 | 37.9 | 26.3 | 54.8 | 45.0 | NIH | DXA | CLR70 |
| 16 | C | 1 | FPLDX | | | | | | | F | White | No | 45 | 38.2 | 24.3 | 39.5 | 22.2 | 45.7 | 38.2 | UM | Prodigy | QKC43 |
| 16 | C | 2 | FPLDX | | y | y | | | y | F | White | No | 55 | 38.7 | 20.7 | 45.1 | 34.3 | 55.0 | 47.0 | UM | Prodigy | LAM66 |
| 16 | C | 3 | FPLDX | | y | y | | | n | F | White | No | 34 | 38.8 | 25.2 | 28.6 | 24.6 | 44.1 | 36.8 | UM | Prodigy | EYO66 |
| 16 | C | 4 | FPLDX | | n | n | | | n | F | White | No | 32 | 40.9 | 22.2 | 41.8 | 34.0 | 51.6 | 46.0 | UM | Prodigy | VKN31 |
| 16 | C | 5 | FPLDX | | n | y | ? | ? | n | M | White | | 65 | 28.0 | 20.0 | 20.5 | 19.2 | 28.3 | 24.1 | NIH | DXA | TGQ88 |
| 16 | C | 6 | FPLDX | | y | y | y | y | n | M | White | | 46 | 32.1 | 25.3 | 13.5 | 13.4 | 30.2 | 22.7 | NIH | DXA | LQP70 |
| 17 | A | 1 | FPLDX | | n | y | | | y | M | White | No | 62 | 32.9 | 20.5 | 29.1 | 24.1 | 50.4 | 39.5 | UM | Prodigy | QNT68 |
| 17 | A | 2 | FPLDX | | y | y | | | y | M | White | No | 38 | 33.1 | 22.7 | 29.3 | 21.6 | 39.5 | 33.0 | UM | Prodigy | EYC46 |
| 17 | A | 3 | FPLDX | | n | n | | | n | M | White | No | 34 | 34.0 | 26.5 | 33.4 | 29.0 | 47.0 | 40.7 | UM | Prodigy | NSF35 |
| 18 | A | 1 | APL | | | | | | | F | White | No | 40 | 29.5 | 20.8 | 14.5 | 25.3 | 31.5 | 31.0 | UM | Prodigy | FTR25 |
| 18 | A | 2 | APL | | | | | | | M | White | No | 24 | 24.0 | 20.7 | 5.1 | 17.9 | 14.8 | 14.5 | UM | Prodigy | WNI47 |

P, row and col=page, row and column of Meral *et al.* displaying fat shadow image of subject. Group= experimental or control group under which subject is classified; AGL=Acquired Generalized Lipodystrophy; APL=Acquired Partial Lipodystrophy; CGL=Congenital Generalized Lipodystrophy; CGL1=Congenital Generalized Lipodystrophy type 1 (AGPAT2); CGL2=Congenital Generalized Lipodystrophy type 2 (BSCL2); FPLD=Familial Partial Lipodystrophy syndrome; FPLD1=Familial Partial Lipodystrophy type 1, Köbberling variety; FPLD2=Familial Partial Lipodystrophy type 2, Dunnigan variety (LMNA); FPLD3=Familial Partial Lipodystrophy type 3 (PPARG); and FPLDX=All FPLD other than FPLD2.

NM=Noemi Malandrino's FPLD predictions (NIH); RB=Rebecca J. Brown's FPLD predictions (NIH); RM=Rasimcan Meral's FPLD predictions (UM); EO=Elif A. Oral's FPLD predictions (UM); and BA=Barış Akıncı's FPLD predictions (independent, Dokuz Eylul University)
BM=body mass index
FFMI=Fat Free Mass Index (Kg/m$^2$)

There was a total of 55 female FPLD patients with DXA data presented in Meral (2018). The FPLD diagnoses of the patients in Meral (2018) was based on the opinion of a clinical expert in FPLD based on physical examination, medical history and laboratory values. Different colors were given to the different subtypes of FPLD since they had different genetic etiologies and differed somewhat from each other clinically. FPLDX (n=33) was designated by the authors as patients that did not have FPLD2 (n=22). FPLDX can be sub-divided into FPLDX with known genetic etiology (n=7) and FPLDX with unknown genetic etiology (n=26). The FPLDX with known genetic etiology consisted of 5 female patients with *PPARG* variants (FPLD3) and 2 patients with *POLO* variants. *POLD* variants have been associated with FPLD, but do not have an OMIM FPLD designation. The remaining FPLDX patients could have had either FPLD1 (Kobberlings) or another form of FPLD, the genetic basis for which is currently unknown, or possibly even atypical APLD (acquired partial lipodystrophy).

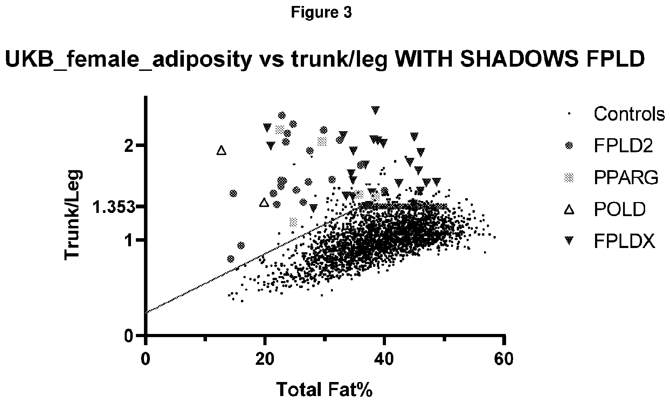

The FPLD datapoints were plotted alongside the UKB dataset (controls). See Figure 3. From this, it was determined that trunk-leg ratio was positively correlated with total body fat (TF) percentage such that higher trunk-leg fat ratios (TLR) were seen in those with higher body fat percentages. However, the relationship changes with the body fat percentage. Specifically, the change in TLR per change in TF% decreases as TF% increases. The FPLD subjects could be separated from the control subjects with a curvilinear function, but to simplify this, two lines were drawn. A line (line 1) was created as a best-fit by eye in Prism for individuals with higher adiposity to distinguish controls from FPLD subjects at a trunk/leg value of 1.353 and adiposity of >36%. A second line (line 2) was drawn in Prism to separate FPLD and control data points in those patients with total body fat percentage ≤36%.

The Prism graph was pasted into webplot digitizer (apps.automeris.io/wpd/) and the values for the slope of line 2 and the y-intercept from line 2 were determined. FPLD was taken as the area above these 2 lines. A datapoint was considered to be FPLD if it was above either line. The 2 lines intersect at an adiposity of 36%.

The line is defined (y=mx+b, where m is the slope and b is the y-intercept) as

$$\text{trunk/leg fat percent ratio}=(.0311 \times \text{Total body fat\%})+0.232$$

which was rearranged to give:

$$\text{trunk/leg fat percent ratio} -(.0311 \times \text{Total body fat\%}) = 0.232$$

So, the area above this line represented those with FPLD and was given as

$$(\text{trunk/leg fat percent ratio})-(.0311 \times \text{Total body fat\%}) \geq 0.232$$

This yielded diagnostic criteria of FPLD as follows:

Is adiposity (total body fat percentage) >36%?
If YES, is trunk/leg fat percent ratio >1.353?

  IF YES→7FPLD
  IF NO→not-FPLD

IF NO
IS (trunk/leg fat percent ratio)-(.0311 x Total body fat%) ≥ 0.232

  IF YES→FPLD
  IF NO→not FPLD

Since the algorithm was trained on the UKB set, it was then tested on the NHANES (National Health and Nutrition Examination Survey) (www.cdc.gov/nchs/nhanes/index.htm) data set of 2347 females. There were 15 false positives in NHANES giving a specificity of 99.3%. This false positive rate is an upper limit and made the assumption that no female NHANES subjects had FPLD. This is a reasonable assumption given that the NHANES females DXA dataset corresponded to 2347 females and the high-estimate for prevalence of FPLD was 1:6,000. Of note, of these 15 false positives, 4 came from the branch of subjects with total body fat of >36% (n=1249) and trunk/leg fat percent ratio of >1.353 and 11 false positives came from the branch of subjects with total body fat 20-36% (n=1095) and a value of (trunk/leg-0.0311 x total percent fat) >0.232. This represented a specificity of 99.7% for subjects with total fat >36% and a specificity of 99.0% for subjects with total fat ≤36%.

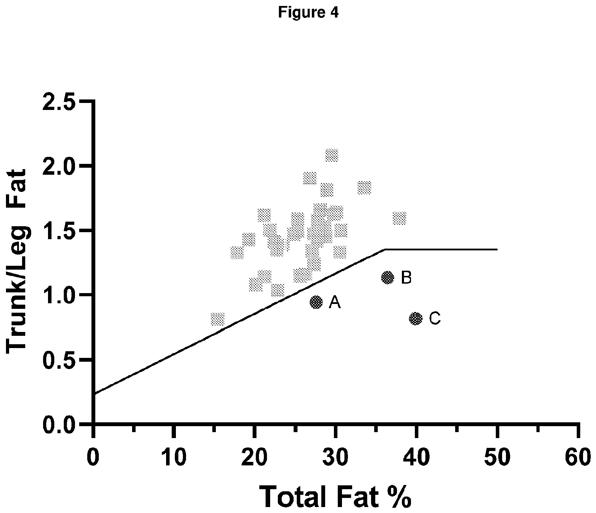

With a specificity of 99.3%, the sensitivity of the algorithm was examined. Since the algorithm was trained on the data set contained in Meral (2018), a second data set to test the algorithm was sought. Blinded, de-identified DXA results from patients in a lipodystrophy clinic were examined. Data on 37 PLD subjects were analyzed. The diagnosis of FPLD in these subjects was based on the clinical opinion of a lipodystrophy expert who considered the patient's medical history, physical examination, and laboratory values. Thirty-four were correctly identified with the algorithm as shown in Figure 4.

The 3 patients (squares A, B and C) that were not detected by the algorithm (FALSE NEGATIVES) were described as follows:

(A) Her history describes loss of fat with likely autoimmunity starting at age 9, but also a positive family history of similar fat distribution. Physical exam stated "lack of subcutaneous fat on extremities and abdomen, and increases subcutaneous fat in chin and neck. BMI only 21.5 with severe insulin resistance. Did not resemble Kobberling's. She was negative for mutations in *LMNA.* HbA1c 10.6, TG 253.

(B) Physical exam did not describe patterns of adipose loss and simply noted that she had markedly muscular calves. She was negative for mutations in *LMNA*. May not really have PL. HbA1c 7.1, TG 216.

(C) Patient is noted to have diagnoses of both Dunnigan's and Emery-Dreifuss muscular dystrophy, so presumably has a *LMNA* mutation. HbA1c 5.9, TG 165. Patient possibly has had replacement of muscle by fat in the limbs or the diagnosis is incorrect.

*HbA1 c is hemoglobin A1c
*TG is triglyceride level (mg/dl)

The presence of autoimmunity in Patient (A) raised the possibility of an APLD diagnosis; there was not certainty regarding the diagnosis of FPLD in Patient (B); and Patient (C) had clinical features of FPLD2, but did not have marked metabolic abnormalities. Given this information, and lacking a definitive "gold standard", the range for sensitivity of the algorithm is 92-100%, depending on whether 0-3 of patients (A), (B), (C) are eliminated from the analysis. The true sensitivity is most likely 95-97% with exclusion of patient (A) and or (B). With a specificity of 99.3% and a sensitivity of 95-97%, this approach had a positive likelihood ratio of ~136 and a negative likelihood ratio of 0.03-0.05.

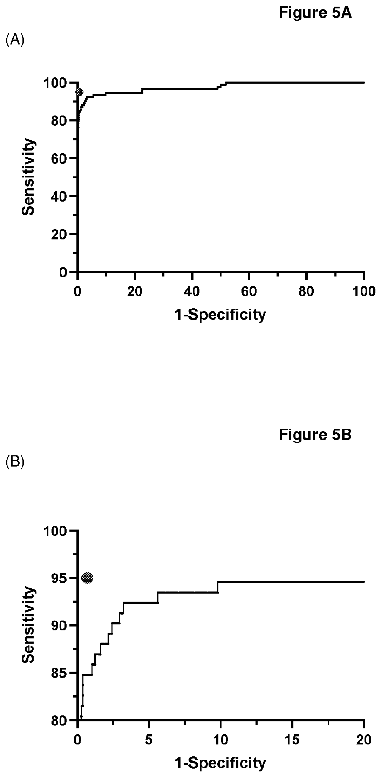

Next, the algorithm to the ROC (Receiver Operating Characteristic) curve using trunk/leg fat percent ratio as a single term discriminator between FPLD females (92: 55 from the published paper and the 37 cases from the NIH) and female controls (NHANES) were compared. In the following graphs, the dot indicates the present algorithm. The first graph (Figure 5 (A)) shows the entire range of specificity and sensitivity while the second graph (Figure 5 (B)) is a blow-up in the region of interest to show more detail. These data demonstrated that our algorithm was superior in both sensitivity and specificity to the use of the trunk/leg ratio alone. Specifically, some have endorsed a trunk/leg cut-off of 1.5 for the diagnosis of PLD (Ajluni et al., Spectrum of disease associated with partial lipodystrophy: lessons from a trial cohort. Clin Endocrinol (Oxf). 2017 May;86(5):698-707). When this cut-off is applied to the 92 FPLD subjects we analyzed, it resulted in a specificity of 99.9% but a sensitivity of only 57.6%. This is because there were many PLD patients with trunk/leg ratio of <1.5, especially at lower total fat percentages and that fact is only taken into account with our algorithm. In order to achieve a specificity of 99.3% (at a trunk/leg ratio of 1.3), the trunk/leg ratio would achieve a sensitivity of only 84.8%. Conversely, in order to achieve a sensitivity of 95% (at a trunk/leg ratio of 0.943), using the trunk/leg ratio alone would only achieve a specificity of 77%.

A modification of the algorithm is to put an upper bound on permissible total fat percent. This is reasonable since a patient with very high body fat is likely to have metabolic disease that is more related to excess fat in certain regions than metabolic disease due to lipoatrophy. In the data sets above, the highest total fat percent in a FPLD patient was 48.7%, so we may use an upper limit of total fat percent of 50%. This modification does not alter the sensitivity or specificity of our algorithm. However, it should be noted that the specificity of this algorithm was tested in the NHANES database. The use of the 50% upper limit can be useful in other populations with a higher rate of obesity.

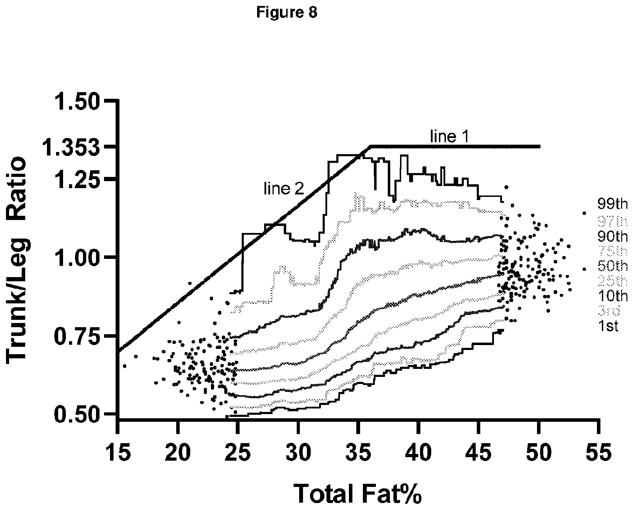

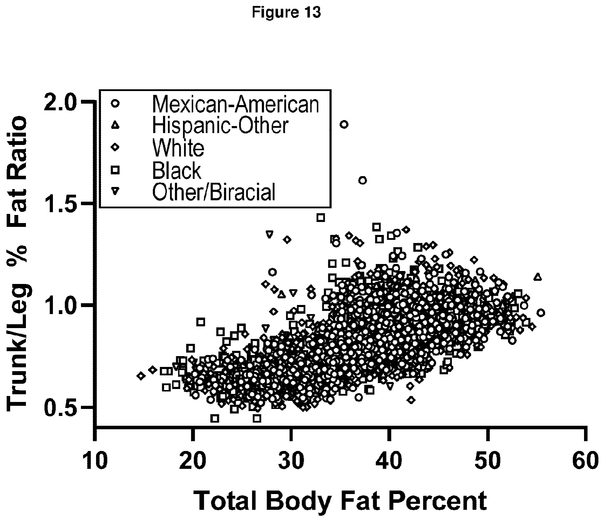

The relationship between total adiposity and trunk-leg fat ratios may differ among different ethnic groups. It may therefore be inappropriate to use the FPLD diagnostic DXA algorithm derived from UKB and NHANES, which is predominantly made up of Caucasians of European ancestry in other countries with a different ethnic composition. We have validated this algorithm for all 5 ethnicities defined in NHANES by evaluating them separately. The relationship between trunk/leg fat percent ratio and total body fat percent is similar among the five different ethnicities in NHANES (Figure 13). However, analysis of data from Korea indicate the NHANES-based algorithm does not apply to this population. Therefore, the current invention embodies using the 99th percentile for trunk-leg ratio normalized to total body fat to determine the diagnostic criteria for any population in which there are sufficient control data to establish a 99th percentile with confidence. People of South East Asian ethnicity suffer from greater metabolic abnormalities at a given body mass index compared to people of European ancestry. As mentioned above, it would be desirable to derive different DXA criteria for different ethnicities using appropriate large control data sets. As opposed to going through the above process *de novo,* an alternative approach using percentile curves could be more adaptable. We examined the distribution of trunk-leg ratios among females in NHANES and plotted the values for the 1st, 3rd, 10th, 25th, 50th, 75th, 90th, 97th and 99th percentile alongside the discriminator lines separating FPLD from controls according to the algorithm. The percentile curves were generated by sorting the subjects by total adiposity from least to greatest. We first looked at the first 300 subjects (subject 1-subject 300) and calculated the mean total adiposity (for the x-value) and then determined the corresponding percentiles for subject 1-subject 300 corresponding to that x-value. The values for the 1st percentile of the 300 patients is the value corresponding to the third lowest trunk-leg ratio among those 300 subjects. The process was repeated moving to the next set of 300 patients (subject 2- subject 301) until the last 300 subjects (with the highest trunk-leg ratio) were reached, thus generating the percentile curves (Figure 8). The more extreme percentiles (i.e., 1st and 99th) are not as smooth as the more central percentiles (i.e., 50th). The NHANES percentile better exemplified the positive relationship that was seen in the scatter plot of the individual subject values for total adiposity and trunk-leg ratios. Because the window of 300 subjects looked at 150 to the left and 150 to the right, it was not possible to calculate the percentile curves for the first or last 150 subjects. For this reason, those values are shown individually as points. The window of 300 was chosen arbitrarily to maximize the smoothness of percentile curves with getting as much coverage as possible for the curves across the entire range of total adiposity. While the slopes of the different percentile curves are different from each other, they closely approximate the slope of the discriminator function. In addition, the discriminator function lies near the curve for the 99th percentile of trunk-leg ratio for total percent fat.

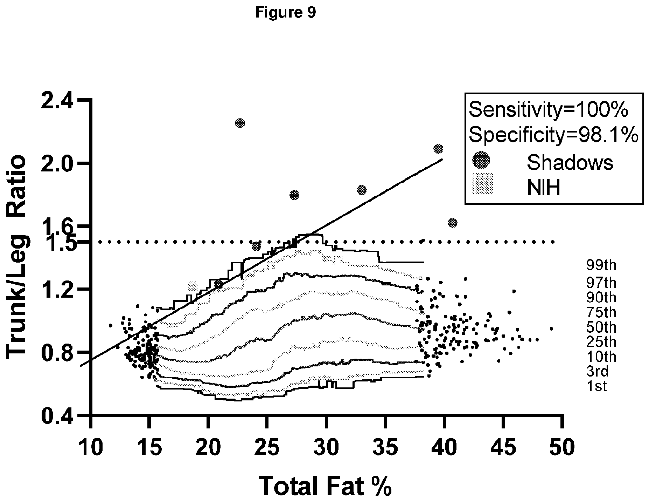

The percentile approach can be used to create diagnostic algorithms for demographic groups in which there are few or no FPLD subjects as long as there is a large data set of controls from subjects in that demographic group. As an example, consider males which make up only ~10% of FPLD subjects and for which there is a scarcity of DXA data available for FPLD patients, but large data sets of control subjects (also NHANES). We can create a discriminator function for males using percentiles as shown in Figure 9. With only 7 FPLD male DXA data points, the "quanta" interval for sensitivity is 14%. An algorithm could correctly predict 7/7 patients or 6/7 patients and so on, but this minimal difference of 1/7 is equivalent to a change in sensitivity of 14.3%. For this reason, we draw 2 discriminator functions: 1 with 100% sensitivity (7/7) and 1 with 86% sensitivity (6/7). For 100% sensitivity the discriminator function is

FOR total body fat percentage >27.5%
IF trunk/leg fat percent ratio >1.5 THEN patient has FPLD
FOR total body fat percentage ≤27.5%
IF trunk/leg fat percent ratio > 0.0429* Total body fat%+ 0.321 THEN patient has FPLD which is rearranged to:
IF trunk/leg fat percent ratio -(0.0429*Total body fat%) >0.321 THEN patient has FPLD

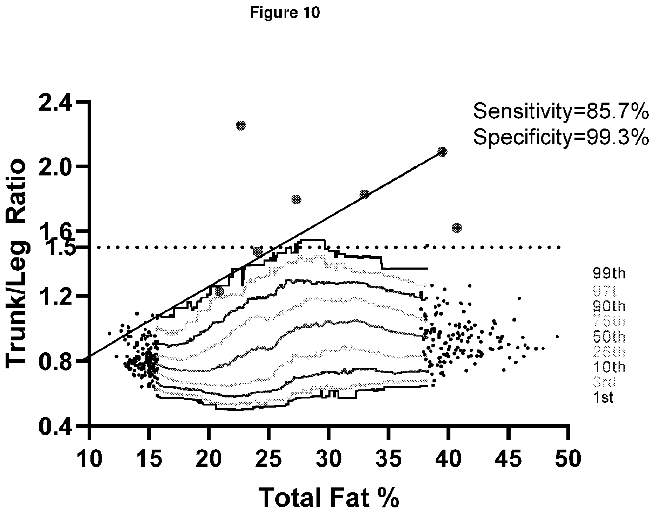

With 100% sensitivity, there are 49 false positives corresponding to a specificity of 98.1%. If we accept a lower sensitivity of 86% (6/7), the number of false positives is reduced three-fold to 15 corresponding to an increase in specificity to 99.3% The discriminator function, when sensitivity is set at 86%, has the same slope as the discriminator function with the sensitivity set at 100% with a different y-intercept. See Figure 10.

The discriminator function with a sensitivity of 86% is:

FOR Total body fat percentage >25.7%
IF trunk/leg fat percent ratio is >1.5 THEN patient has FPLD
FOR Total body fat%≤25.7%
IF trunk/leg fat percent ratio > .0429*Total body fat%+ 0.4 THEN patient has FPLD which is rearranged to:
IF trunk/leg fat percent ratio -.0429*Total body fat% >0.4 THEN patient has FPLD

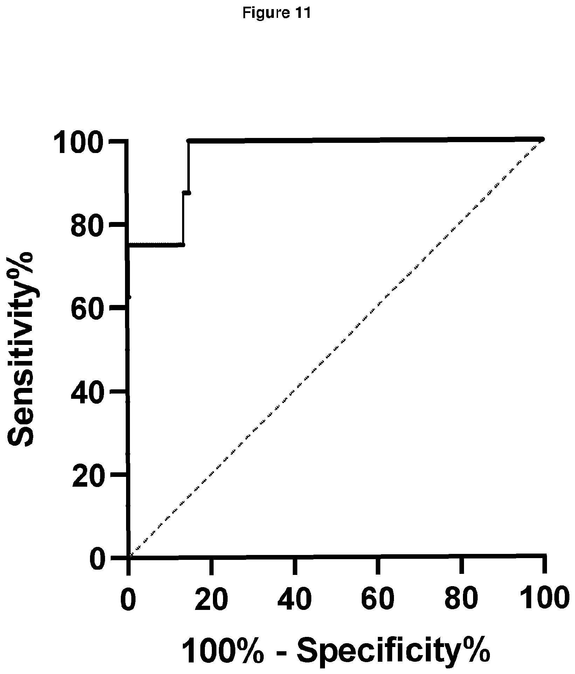

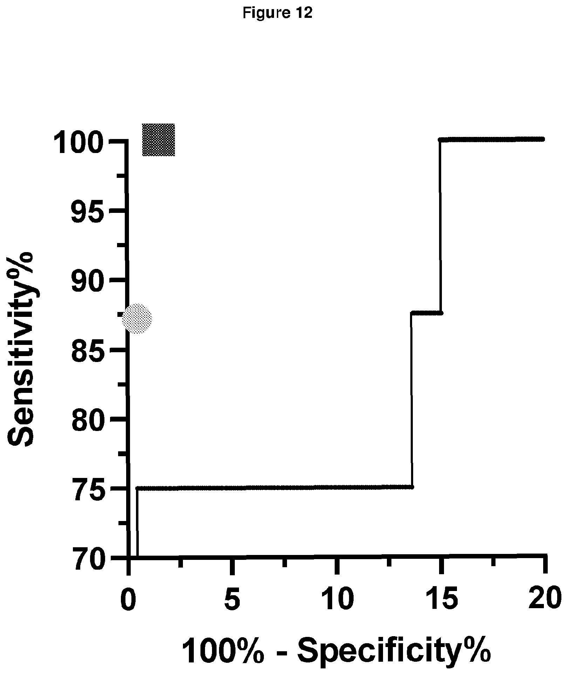

Next, we examined the performance of these two discriminators compared to using the trunk-leg ratio alone in a receiver operator curve. See Figure 11. The 2 discriminator functions can best be seen when the ROC curve is blown up. See Figure 12.

We next made use of the fact that NHANES has ethnicity data. Specifically, the following ethnicity categories are used: White, Black, Mexican-American, Hispanic-other (not Mexican), and Other/Biracial. For the female data set, we can see that the 5 different groups have a similar relationship between trunk-leg ratio and total body fat percent. (see Figure 13). Of the 2347 NHANES females, 659 (28%) were Mexican-American, 81 (4%) were described as Hispanic-other, 871 (37%) were white, 612 (26%) were black and 124 (5%) were described as other-biracial. Of the 55 FPLD females, 48 (87%) were white, 1 (2%) was black, 1 (2%) described as other, and 5 (9%) in which no racial information was provided. While the racial composition of the FPLD and NHANES data sets are different, the fact that there were not significant differences between the races in NHANES suggests no errors will be introduced by comparing these 2 groups of subjects.

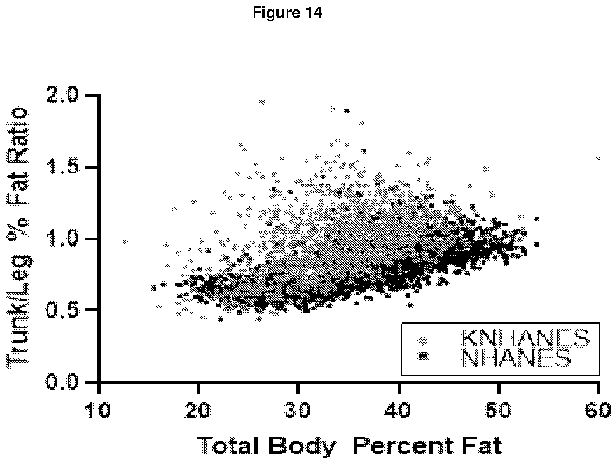

First, for women, the relationship between trunk-leg ratio is very similar among these different groups. However, Asians are not represented in NHANES. To determine if Asians have a similar relationship between trunk-leg ratio and adiposity as observed in the other ethnic groups, we examined data from the 2011 sample representing 1524 women in the Korean NHANES (KNHANES). Women in NHANES had slightly higher total fat percent compared to women in KNHANES (median [IQR] were 36.6 [30.8, 41.8] and 34.3 [30.1, 38.5], respectively. Given that trunk-leg fat ratio is increased with increased total fat percent, one may expect NHANES to have higher trunk-leg fat ratio than KNHANES, but the opposite is true. Women in KNHANES had a significantly higher trunk-leg fat ratio compared to women in NHANES (0.97±0.22 vs 0.82±0.16, p<0.001). This indicated that Korean women had a higher trunk-leg ratio than American or British women (where Asians were a small percentage of the population). If the algorithm used to discriminate between FPLD and non-FPLD in the American and UK population is applied to the Korean population, the specificity is much lower. Specifically, of the 1524 Korean women, 588 had a total percent fat ≥36% and among them there were 28 who would have falsely been identified as having FPLD (specificity = 95.2%). Of the 1524 Korean women, 936 had a total body fat percent ≤36 and 127 of them would have been falsely identified as having FPLD (specificity=86.4%). The combined specificity of the total sample of 1524 women in KNHANES would be 89.8%. Despite the absence of published reports of Korean women with FPLD, we can determine criteria to discriminate between controls and FPLD subjects by using the 99th percentile of trunk-leg ratio normalized for total body fat percent. While there is a positive relationship between total percent body fat and trunk-leg ratio in Korean women, the slope of the line decreases at higher percentiles, such that a single trunk-leg ratio of 1.54 can be used in this population. The increased trunk-leg fat ratio in Korean women was largely due to a decrease in leg fat as opposed to an increase in trunk fat. See Figure 14.

We examined the percentile curves of trunk-leg fat ratio for total body fat in Korean women and observed that the positive relationship between these variables was blunted, particularly when looking at the upper percentile curves. Therefore, for determining criteria to discriminate between FPLD and controls in Korean women, a single term criteria of trunk-leg fat percent ratio equivalent to the 99th percentile for the entire population (>1.54) would mostly likely be appropriate. FPLD is likely in a Korean women with TLR>1.54. The same principle holds for Korean men such that FPLD is likely in a Korean man with TLR>1.90.

## Example 2: Decision-tree Algorithm for Diagnosing PLD.

As discussed in Example 1, attempts to use DXA criteria to diagnose FPLD have used static measures such as "Trunk/Leg Fat Percent" greater than a constant. Other attempts have used less static measures such as a leg fat % <1%ile for age in comparison to large datasets of control populations. However, this approach is only valid for one type of FPLD, FPLD2.

The methods described herein use much more of the information from the fat percentage in the trunk and limbs. A core question in classifying FLPD depends on the decision variable. Here the decision variable is Categorical, FPLD, or non-FPLD. A classification decision tree is a good fit to answer such a question. Decision trees are built using a heuristic called recursive partitioning (Breiman et al., Classification and Regression Trees. CRC Press; 1984.). This approach is also commonly known as divide-and-conquer because it splits the data into subsets which are then split repeatedly into even smaller subsets, and so on and so forth until the process stops when the algorithm determines that the data within the subsets are sufficiently homogenous, or another stopping criterion has been met.

## The Training Dataset for The Decision Tree Model

To put these observations in practice to create a diagnostic algorithm for FPLD, the following steps were taken. We examined a recent paper showing DXA from individuals with FPLD (Meral et al., "Fat Shadows" From DXA for the Qualitative Assessment of Lipodystrophy: When a Picture Is Worth a Thousand Numbers. Diabetes Care 2018; 41:2255-2258). Individual DXA data from patients with FPLD was copied from Supplementary Materials Table 2 pdf into a spreadsheet (MS Excel). Software R 4.0.2 was used for all modeling and realizations.

The following calculations were performed:

Let $\chi$: $x_1, x_2, ..., x_i$ as the input vector; the decision tree classifiers are constructed by repeated splits of the subsets of $\chi$ into C classes. For split node A, we defined an impurity function $I(A)$ to represent the uncertainty of splitting in such node.

$$I(A) = \sum_{i=1}^{C} f(p_{iA})$$

Where i indicates the class i; C is the number of classes; and p is the probability of node A being in class i.

There are two common candidates for function $f$; the information index $f(p) = -p\log(p)$ and the Gini index $f(p) = p(1 - p)$. Given the selcted $f(p)$, the split in node A is determined by maximizing the impurity reduction $\Delta I$.

$$\Delta I = p(A)I(A) - p(A_L)I(A_L) - p(A_R)I(A_R)$$

The subnotation of *L* denotes the node being split into left and *vice versa* for subnotation R. The impurity function is repeatedly calculated to determine the splits. The modeling for the decision tree classifier was implemented using the *RPART* package in R 4.0.2 (Therneau & Atkinson (2019). rpart: Recursive Partitioning and Regression Trees. R package version 4.1-15. https://CRAN.R-project.org/package=rpart).

The decision tree model was created using 55 FPLD patients and 90 control patients. Using the DXA fat tissue percentage in leg, trunk, and arm, the decision tree classifies the patient as PLD and non-PLD. In this method, the training set was broken down into smaller and smaller subsets while at the same time an associated decision tree gets incrementally developed. The cross-validation method was used to choose the best depth of the tree. The following example code was implemented to fit a decision tree classifier.

```
fit <- rpart(groupPLD ~ Arm + Leg + Trunk, data = PLD.dataset, method = 'class',
        control = rpart.control(xval = 10, minbucket = 2, cp = 0.01))
```

**Table 2-1, Cross-validation Results**

| CP | nsplit | rel error | xerror | xstd |
|---|---|---|---|---|
| 1 0.781818 | 0 | 1.000000 | 1.00000 | 0.106232 |
| 2 0.054545 | 1 | 0.218182 | 0.27273 | 0.066676 |
| 3 0.036364 | 3 | 0.109091 | 0.23636 | 0.062548 |
| 4 0.018182 | 5 | 0.036364 | 0.23636 | 0.062548 |
| 5 0.010000 | 6 | 0.018182 | 0.23636 | 0.062548 |

From Table 2-1; the number of terminal nodes (*nsplit*) with 5 and 6 have the least cross-validation error (*xerror*). The simpler tree with the number of nodes equal to 5 was chosen as the final tree (Figure 6).

The final tree could be verbally interpreted in the following algorithm.

1. Is the fat fraction in leg is greater than or equal to 30.85?

    1.1. If yes, then is the fat fraction in leg is greater or equal to 37.25?

        1.1.1. If yes, then the subject is classified as

        1.1.2. If no, then is the fat fraction in arm less than 37.85?

            1.1.2.1. If yes, then the subject is classified as not-PLD.

            1.1.2.2. If no, then is the fat fraction in trunk less than 51?

                1.1.2.2.1. If yes, then the subject is classified as not-PLD.

                1.1.2.2.2. If no, then the subject is classified as PLD.

    1.2. If no, then is the fat fraction in leg greater than or equal to 24.5?

        1.2.1. If yes, then is the fat fraction in trunk less than 34.55?

            1.2.1.1. If yes, then the subject is classified as not-PLD.

            1.2.1.2. If no, then the subject is classified as PLD.

        1.2.2. If no, then the subject is classified as PLD.

A combined dataset from NIH and NHANES were used to test the utility of the fitted tree model. In the NHANES dataset, there were 2347 females. It was assumed that all females in the NHANES dataset were non-FPLD subjects. In the NIH dataset, there were 38 PLD patients and 19 non-PLD patients. Below is the confusion matrix for the combined dataset using the fitted model.

**Table 2-2. Confusion Matrix**

|  |  | Predicted from Tree classifier | |
| --- | --- | --- | --- |
|  |  | FPLD | Non-FPLD |
| Actual source | FPLD | 32 | 6 |
|  | Non-FPLD | 53 | 2313 |
| Sensitivity : 0.84<br>Specificity : 0.98 | | | |

In the combined dataset, 53 out of 2347 non-PLD subjects were mis-classified as FPLD; 6 out of 38 PLD subjects were mis-classified as not-PLD. The proposed decision tree classifier had great power in detecting the FPLD patients while keeping the false negative rate in a low level. The sensitivity and the specificity from the decision tree classifier were 0.84 and 0.98, respectively.

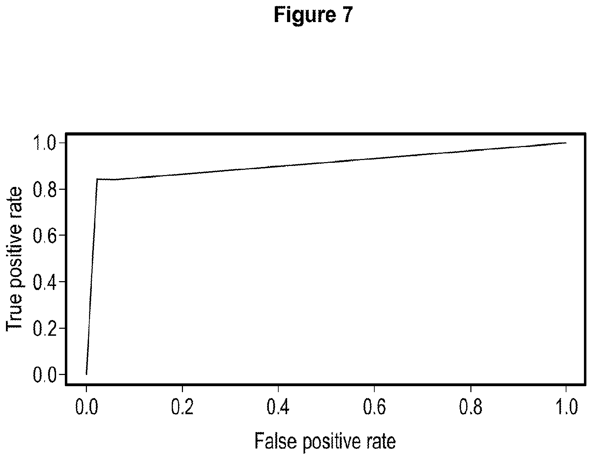

The ROC curve for the combined dataset of NIH and NHANES showed that the performance of the final tree classifier performed well. The ROC curve was very close to the (0, 1) point. The AUC for the ROC curve was 0.94. See Figure 7

An example of how the decision tree classifier classified subjects into different categories is listed below.

*e.g.,* 1: A female subject with leg fat percentage of 34%, arm fat percentage of 34%, and trunk fat percentage of 38.6% will be classified as **not**-PLD. The decision process is as followed:

1. Is the fat fraction in leg is greater than or equal to 30.85?
1.1. If yes, then is the fat fraction in leg is greater or equal to 37.25?
1.1.1. If no, then is the fat fraction in arm less than 37.85?
1.1.1.1. If no, then is the fat fraction in trunk less than 51?
1.1.1.1.1. If yes, then the subject is classified as

## Claims

1. A LEPR agonist for use in a method for treating partial lipodystrophy, in a subject, comprising determining whether the subject has or is likely to have partial lipodystrophy by a method comprising determining

   (A)

   (1) if

   (i) total body fat percentage is greater than 36%;
   and
   (ii) trunk/leg fat percent ratio is greater than 1.35;

   or
   (2) if

   (i) total body fat percentage is less than or equal to 36%;
   and
   (ii) (trunk/leg fat percent ratio)-(0.0311 X total body fat percentage) $\geq$ 0.232

   wherein, if the criteria are met, the subject has or is likely to have partial lipodystrophy and, optionally, if the criteria are not met, the patient does not have or is not likely to have partial lipodystrophy, further optionally wherein the subject is a female; or

   (B)

   (1) if

   (i) total body fat percentage is greater than 25.7%;

and

(ii) trunk/leg fat percent ratio is greater than 1.5;

or
(2) if

(i) total body fat percentage is less than or equal to 25.7%;
and
(ii) (trunk/leg fat percent ratio)-(0.0429 X total body fat percentage) > 0.4

wherein, if the criteria are met, the subject has or is likely to have partial lipodystrophy and, optionally, if the criteria are not met, the patient does not have or is not likely to have partial lipodystrophy, further optionally wherein the subject is a male; or

(C)

(1) if

(i) total body fat percentage is greater than 27.5%;
and
(ii) trunk/leg fat percent ratio is greater than 1.5;

or
(2) if

(i) total body fat percentage is less than or equal to 27.5%;
and
(ii) (trunk/leg fat percent ratio)-(0.0429 X total body fat percentage) > 0.321,
wherein, if the criteria are met, the subject has or is likely to have partial lipodystrophy and, optionally, if the criteria are not met, the patient does not have or is not likely to have partial lipodystrophy, further optionally wherein the subject is a male;
and if the subject has partial lipodystrophy or is likely to have lipodystrophy, then administering, to the subject, an effective amount of the LEPR agonist.

2. The LEPR agonist for use according to claim 1 wherein the subject is in a population

• that includes a greater number of subjects with PLD than that of the general population;
• having a pre-test probability of greater than or equal to about 0.7% or greater than or equal to about 1 in 142;
• of first- and second-degree relatives of subjects with PLD;
• of non-obese diabetics;
• of individuals with triglycerides >about 400 mg/dL; and/or
• of individuals in which the clinician notices an altered fat distribution.

3. The LEPR agonist for use according to any one of claims 1-2 wherein:

(a) the partial lipodystrophy is familial partial lipodystrophy (FPLD); and/or
(b) the partial lipodystrophy is familial partial lipodystrophy (FPLD) which is FPLD1, FPLD2, FPLD3, FPLD4, FPLD5, FPLD6 or FPLD7; and/or
(c) said fat percentages are determined by Dual-energy X-ray Absorptiometry using a 3-compartment model.

4. The LEPR agonist for use according to any one of claims 1-3 wherein the LEPR agonist is an isolated agonist antibody or antigen-binding fragment that binds specifically to LEPR.

5. The LEPR agonist for use according to any one of claims 1-4 wherein:

(a) the subject suffers from one or more selected from the group consisting of:

• acanthosis nigricans;

- atherosclerosis;
- atherosclerotic coronary heart disease;
- cardiac arrhythmia;
- cardiomyopathy;
- congestive heart failure;
- coronary artery disease;
- diabetes;
- dyslipidemia;
- eruptive xanthoma;
- glucose intolerance;
- hepatic steatosis;
- hepatomegaly;
- hirsutism;
- hyperandrogenemia;
- hyperglycemia;
- hypertension;
- hypertriglyceridemia;
- insulin resistance;
- liver cirrhosis;
- muscular dystrophy;
- myopathy;
- non-alcoholic steatohepatitis;
- oligomenorrhoea;
- pancreatitis;
- polycystic ovary syndrome;
- proteinuric renal disease;
- severe insulin resistance; and
- subfertility; and/or

(b) the subject has a homozygous or heterozygous mutation in the *LMNA, PPARG, PLIN1, AKT2, LIPE, CIDEC, ZMPSTE24, PIK3R1, ANDRA2A, CAV1, PCYT1A, PSMB8, WRN, POLD1,* and/or the *BLM* gene.

6. The LEPR agonist for use according to any one of claims 1-5 wherein:

(a) the LEPR agonist is an isolated agonist antibody or antigen-binding fragment that binds specifically to LEPR comprising:

(i) a light chain variable region that comprises the LCDR1, LCDR2 and LCDR3 of a light chain variable region that comprises the amino acid sequence set forth in SEQ ID NO: 10; and
a heavy chain variable region that comprises the HCDR1, HCDR2 and HCDR3 of a heavy chain variable region that comprises the amino acid sequence set forth in SEQ ID NO: 2;
(ii) a light chain variable region that comprises the LCDR1, LCDR2 and LCDR3 of a light chain variable region that comprises the amino acid sequence set forth in SEQ ID NO: 10; and
a heavy chain variable region that comprises the HCDR1, HCDR2 and HCDR3 of a heavy chain variable region that comprises the amino acid sequence set forth in SEQ ID NO: 18;
(iii) a light chain variable region that comprises the LCDR1, LCDR2 and LCDR3 of a light chain variable region that comprises the amino acid sequence set forth in SEQ ID NO: 10; and
a heavy chain variable region that comprises the HCDR1, HCDR2 and HCDR3 of a heavy chain variable region that comprises the amino acid sequence set forth in SEQ ID NO: 26;
(iv) a light chain variable region that comprises the LCDR1, LCDR2 and LCDR3 of a light chain variable region that comprises the amino acid sequence set forth in SEQ ID NO: 10; and
a heavy chain variable region that comprises the HCDR1, HCDR2 and HCDR3 of a heavy chain variable region that comprises the amino acid sequence set forth in SEQ ID NO: 34;
(v) a light chain variable region that comprises the LCDR1, LCDR2 and LCDR3 of a light chain variable region that comprises the amino acid sequence set forth in SEQ ID NO: 10; and
a heavy chain variable region that comprises the HCDR1, HCDR2 and HCDR3 of a heavy chain variable region that comprises the amino acid sequence set forth in SEQ ID NO: 42;
(vi) a light chain variable region that comprises the LCDR1, LCDR2 and LCDR3 of a light chain variable region that comprises the amino acid sequence set forth in SEQ ID NO: 10; and

a heavy chain variable region that comprises the HCDR1, HCDR2 and HCDR3 of a heavy chain variable region that comprises the amino acid sequence set forth in SEQ ID NO: 50;

(vii) a light chain variable region that comprises the LCDR1, LCDR2 and LCDR3 of a light chain variable region that comprises the amino acid sequence set forth in SEQ ID NO: 10; and

a heavy chain variable region that comprises the HCDR1, HCDR2 and HCDR3 of a heavy chain variable region that comprises the amino acid sequence set forth in SEQ ID NO: 58;

(viii) a light chain variable region that comprises the LCDR1, LCDR2 and LCDR3 of a light chain variable region that comprises the amino acid sequence set forth in SEQ ID NO: 10; and

a heavy chain variable region that comprises the HCDR1, HCDR2 and HCDR3 of a heavy chain variable region that comprises the amino acid sequence set forth in SEQ ID NO: 66;

(ix) a light chain variable region that comprises the LCDR1, LCDR2 and LCDR3 of a light chain variable region that comprises the amino acid sequence set forth in SEQ ID NO: 10; and

a heavy chain variable region that comprises the HCDR1, HCDR2 and HCDR3 of a heavy chain variable region that comprises the amino acid sequence set forth in SEQ ID NO: 74;

(x) a light chain variable region that comprises the LCDR1, LCDR2 and LCDR3 of a light chain variable region that comprises the amino acid sequence set forth in SEQ ID NO: 90; and

a heavy chain variable region that comprises the HCDR1, HCDR2 and HCDR3 of a heavy chain variable region that comprises the amino acid sequence set forth in SEQ ID NO: 82;

(xi) a light chain variable region that comprises the LCDR1, LCDR2 and LCDR3 of a light chain variable region that comprises the amino acid sequence set forth in SEQ ID NO: 90; and

a heavy chain variable region that comprises the HCDR1, HCDR2 and HCDR3 of a heavy chain variable region that comprises the amino acid sequence set forth in SEQ ID NO: 98; or

(xii) a light chain variable region that comprises the LCDR1, LCDR2 and LCDR3 of a light chain variable region that comprises the amino acid sequence set forth in SEQ ID NO: 90; and

a heavy chain variable region that comprises the HCDR1, HCDR2 and HCDR3 of a heavy chain variable region that comprises the amino acid sequence set forth in SEQ ID NO: 106; or

an antibody or antigen-binding fragment that binds to the same epitope as any one or more of (i)-(xii) and/or competes for binding to LEPR with any one or more of (i)-(xii); or

(b) the LEPR agonist is an isolated agonist antibody or antigen-binding fragment that binds specifically to LEPR comprising:

(i) a light chain variable region that comprises the amino acid sequence set forth in SEQ ID NO: 10; and a heavy chain variable region that comprises the amino acid sequence set forth in SEQ ID NO: 2;

(ii) a light chain variable region that comprises the amino acid sequence set forth in SEQ ID NO: 10; and a heavy chain variable region that comprises the amino acid sequence set forth in SEQ ID NO: 18;

(iii) a light chain variable region that comprises the amino acid sequence set forth in SEQ ID NO: 10; and a heavy chain variable region that comprises the amino acid sequence set forth in SEQ ID NO: 26;

(iv) a light chain variable region that comprises the amino acid sequence set forth in SEQ ID NO: 10; and a heavy chain variable region that comprises the amino acid sequence set forth in SEQ ID NO: 34;

(v) a light chain variable region that comprises the amino acid sequence set forth in SEQ ID NO: 10; and a heavy chain variable region that comprises the amino acid sequence set forth in SEQ ID NO: 42;

(vi) a light chain variable region that comprises the amino acid sequence set forth in SEQ ID NO: 10; and a heavy chain variable region that comprises the amino acid sequence set forth in SEQ ID NO: 50;

(vii) a light chain variable region that comprises the amino acid sequence set forth in SEQ ID NO: 10; and a heavy chain variable region that comprises the amino acid sequence set forth in SEQ ID NO: 58;

(viii) a light chain variable region that comprises the amino acid sequence set forth in SEQ ID NO: 10; and a heavy chain variable region that comprises the amino acid sequence set forth in SEQ ID NO: 66;

(ix) a light chain variable region that comprises the amino acid sequence set forth in SEQ ID NO: 10; and a heavy chain variable region that comprises the amino acid sequence set forth in SEQ ID NO: 74;

(x) a light chain variable region that comprises the amino acid sequence set forth in SEQ ID NO: 90; and a heavy chain variable region that comprises the amino acid sequence set forth in SEQ ID NO: 82;

(xi) a light chain variable region that comprises the amino acid sequence set forth in SEQ ID NO: 90; and a heavy chain variable region that comprises the amino acid sequence set forth in SEQ ID NO: 98;

or

(xii) a light chain variable region that comprises the amino acid sequence set forth in SEQ ID NO: 90; and

a heavy chain variable region that comprises the amino acid sequence set forth in SEQ ID NO: 106; or

is an antibody or antigen-binding fragment that binds to the same epitope as any one or more of (i)-(xii) and/or competes for binding to LEPR with any one or more of (i)-(xii)

or

is mibavademab.

7. The LEPR agonist for use according to any one of claims 1-6, wherein the method further comprises administering, to the subject, a further therapeutic agent in association with the LEPR agonist, optionally wherein the further therapeutic agent is recombinant human leptin; metreleptin; a PCSK9 inhibitor; an anti-PCSK9 antibody or antigen-binding fragment thereof; alirocumab; evolocumab; bococizumab; lodelcizumab; ralpancizumab; an HMG CoA reductase inhibitor; atorvastatin; rosuvastatin; cerivastatin; pitavastatin; fluvastatin; simvastatin; lovastatin; pravastatin; ezetimibe; insulin; an insulin variant; an insulin secretagogue; metformin; a sulfonylurea; a sodium glucose cotransporter 2 (SGLT2) inhibitor; dapaglifozin; canaglifozin; empagliflozin; a GLP-1 agonist or analogue; exenatide; liraglutide; lixisenatide; albiglutide; dulaglutide; a glucagon (GCG) inhibitor; an anti-GCG antibody; a glucagon receptor (GCGR) inhibitor; an anti-GCGR antibody; a small molecule GCGR antagonist; a GCGR-specific antisense oligonucleotide; an anti-GCGR aptamer; a GCGR spiegelmer; an angiopoietin-like protein (ANGPTL) inhibitor; an anti-ANGPTL3 antibody; an anti-ANGPTL4 antibody; an anti-ANGPTL8 antibody; phentermine; orlistat; topiramate; bupropion; topiramate & phentermine; bupropion & naltrexone; bupropion & zonisamide; pramlintide & metreleptin; lorcaserin; cetilistat; tesofensine; velneperit; fish oil; pioglitazone; setmelanotide; a fibrate; fenofibrate; prednisone; niacin; anticonvulsants; digoxin; coumadin; vitamin D; thyroxine; a thyroid supplement; a vitamin supplement; a calcium supplement; carnitine; coenzyme Q10; an anti-constipation medication; an anti-allergic medication; gabapentin; a narcotic; ketamine; lidocaine; and/or venlafaxine hydrochloride.

## Patentansprüche

1. LEPR-Agonist zur Verwendung in einem Verfahren zur Behandlung einer partiellen Lipodystrophie bei einem Patienten, das die Bestimmung umfasst, ob der Patient eine partielle Lipodystrophie hat oder wahrscheinlich hat, durch ein Verfahren, das folgende Bestimmung umfasst:

(A)

(1) wenn

(i) der Gesamtkörperfettanteil größer als 36% ist; und
(ii) das prozentuale Verhältnis von Rumpf- zu Beinfett größer als 1,35 ist; oder

(2) wenn

(i) der Gesamtkörperfettanteil kleiner als oder gleich 36 % ist und
(ii) (prozentuales Verhältnis Rumpf/Beinfett)-(0,0311 X Gesamtkörperfettanteil) ≥ 0,232,

wobei, wenn die Kriterien erfüllt sind, der Patient eine partielle Lipodystrophie hat oder wahrscheinlich hat, und optional, wenn die Kriterien nicht erfüllt sind, der Patient keine partielle Lipodystrophie hat oder wahrscheinlich nicht hat, wobei ferner optional der Patient eine Frau ist; oder

(B)

(1) wenn

(i) der Gesamtkörperfettanteil größer als 25,7 % ist; und
(ii) das prozentuale Verhältnis von Rumpf- zu Beinfett größer als 1,5 ist; oder

(2) wenn

(i) der Gesamtkörperfettanteil kleiner als oder gleich 25,7 % ist und

(ii) (prozentuales Verhältnis Rumpf/Beinfett)-(0,0429 X Gesamtkörperfettanteil) > 0,4,

wobei, wenn die Kriterien erfüllt sind, der Patient eine partielle Lipodystrophie hat oder wahrscheinlich hat, und optional, wenn die Kriterien nicht erfüllt sind, der Patient keine partielle Lipodystrophie hat oder wahrscheinlich nicht hat, wobei ferner optional der Patient ein Mann ist; oder

(C)

(1) wenn

(i) der Gesamtkörperfettanteil größer als 27,5% ist; und

(ii) das prozentuale Verhältnis von Rumpf- zu Beinfett größer als 1,5 ist;

oder

(2) wenn

(i) der Gesamtkörperfettanteil kleiner als oder gleich 27,5 % ist; und

(ii) (prozentuales Verhältnis Rumpf/Beinfett)-(0,0429 X Gesamtkörperfettanteil) > 0,321,

wobei, wenn die Kriterien erfüllt sind, der Patient eine partielle Lipodystrophie hat oder wahrscheinlich hat, und optional, wenn die Kriterien nicht erfüllt sind, der Patient keine partielle Lipodystrophie hat oder wahrscheinlich nicht hat, wobei ferner optional der Patient ein Mann ist;

und wenn die Person eine partielle Lipodystrophie hat oder wahrscheinlich eine Lipodystrophie hat, dann Verabreichung einer wirksamen Menge des LEPR-Agonisten an die Person.

2. LEPR-Agonist zur Verwendung nach Anspruch 1, wobei das Subjekt zu einer Population gehört

• die eine größere Anzahl von Probanden mit PLD umfasst als die der allgemeinen Bevölkerung;
• mit einer Vortest-Wahrscheinlichkeit von mehr als oder gleich etwa 0,7% oder mehr als oder gleich etwa 1 in 142;
• von Verwandten ersten und zweiten Grades von Personen mit PLD;
• von nicht fettleibigen Diabetikern;
• von Personen mit Triglyceriden > ca. 400 mg/dl; und/oder
• von Personen, bei denen der Kliniker eine veränderte Fettverteilung feststellt.

3. LEPR-Agonist zur Verwendung nach einem der Ansprüche 1 bis 2, wobei:

(a) die partielle Lipodystrophie eine familiäre partielle Lipodystrophie (FPLD) ist; und/oder
(b) die partielle Lipodystrophie eine familiäre partielle Lipodystrophie (FPLD) ist, die FPLD1, FPLD2, FPLD3, FPLD4, FPLD5, FPLD6 oder FPLD7 ist; und/oder
(c) die Fettanteile mittels Dual-Energy-Röntgenabsorptiometrie unter Verwendung eines 3-Kompartiment-Modells bestimmt werden.

4. LEPR-Agonist zur Verwendung nach einem der Ansprüche 1 bis 3, wobei der LEPR-Agonist ein isolierter Agonisten-Antikörper oder ein Antigen-bindendes Fragment ist, das spezifisch an LEPR bindet.

5. LEPR-Agonist zur Verwendung nach einem der Ansprüche 1-4, wobei:

(a) das Subjekt an einer oder mehreren Erkrankungen leidet, die aus der Gruppe ausgewählt sind, bestehend aus:

• Acanthosis nigricans;
• Atherosklerose;
• atherosklerotische koronare Herzkrankheit;
• Herzrhythmusstörungen;
• Kardiomyopathie;

- kongestive Herzinsuffizienz;
- koronare Herzkrankheit;
- Diabetes;
- Dyslipidämie;
- eruptives Xanthom;
- Glukoseintoleranz;
- hepatische Steatose;
- Hepatomegalie;
- Hirsutismus;
- Hyperandrogenämie;
- Hyperglykämie;
- Bluthochdruck;
- Hypertriglyceridämie;
- Insulinresistenz;
- Leberzirrhose;
- Muskeldystrophie;
- Myopathie;
- nicht-alkoholische Steatohepatitis;
- Oligomenorrhöe;
- Bauchspeicheldrüsenentzündung;
- Syndrom der polyzystischen Ovarien;
- proteinurische Nierenerkrankung;
- schwere Insulinresistenz; und
- Subfertilität; und/oder

(b) der Patient eine homozygote oder heterozygote Mutation im *LMNA-, PPARG-, PLIN1-, AKT2-, LIPE-, CIDEC-, ZMPSTE24-, PIK3R1-, ANDRA2A-, CAV1-, PCYT1A-, PSMBB-, WRN-, POLD1-* und/oder *BLM-Gen* aufweist.

6. LEPR-Agonist zur Verwendung nach einem der Ansprüche 1-5, wobei:

(a) der LEPR-Agonist ein isolierter Agonisten-Antikörper oder ein Antigen-bindendes Fragment ist, der/das spezifisch an LEPR bindet, umfassend:

(i) eine variable Region der leichten Kette, die LCDR1, LCDR2 und LCDR3 einer variablen Region der leichten Kette umfasst, die die in SEQ ID NO: 10 dargestellte Aminosäuresequenz umfasst; und eine variable Region der schweren Kette, die HCDR1, HCDR2 und HCDR3 einer variablen Region der schweren Kette umfasst, die die in SEQ ID NO: 2 dargestellte Aminosäuresequenz umfasst;
(ii) eine variable Region der leichten Kette, die LCDR1, LCDR2 und LCDR3 einer variablen Region der leichten Kette umfasst, die die in SEQ ID NO: 10 dargestellte Aminosäuresequenz umfasst; und eine variable Region der schweren Kette, die HCDR1, HCDR2 und HCDR3 einer variablen Region der schweren Kette umfasst, die die in SEQ ID NO: 18 dargestellte Aminosäuresequenz umfasst;
(iii) eine variable Region der leichten Kette, die LCDR1, LCDR2 und LCDR3 einer variablen Region der leichten Kette umfasst, die die in SEQ ID NO: 10 dargestellte Aminosäuresequenz umfasst; und eine variable Region der schweren Kette, die HCDR1, HCDR2 und HCDR3 einer variablen Region der schweren Kette umfasst, die die in SEQ ID NO: 26 dargestellte Aminosäuresequenz umfasst;
(iv) eine variable Region der leichten Kette, die LCDR1, LCDR2 und LCDR3 einer variablen Region der leichten Kette umfasst, die die in SEQ ID NO: 10 dargestellte Aminosäuresequenz umfasst; und eine variable Region der schweren Kette, die HCDR1, HCDR2 und HCDR3 einer variablen Region der schweren Kette umfasst, die die in SEQ ID NO: 34 dargestellte Aminosäuresequenz umfasst;
(v) eine variable Region der leichten Kette, die LCDR1, LCDR2 und LCDR3 einer variablen Region der leichten Kette umfasst, die die in SEQ ID NO: 10 dargestellte Aminosäuresequenz umfasst; und eine variable Region der schweren Kette, die HCDR1, HCDR2 und HCDR3 einer variablen Region der schweren Kette umfasst, die die in SEQ ID NO: 42 dargestellte Aminosäuresequenz umfasst;
(vi) eine variable Region der leichten Kette, die LCDR1, LCDR2 und LCDR3 einer variablen Region der leichten Kette umfasst, die die in SEQ ID NO: 10 dargestellte Aminosäuresequenz umfasst; und eine variable Region der schweren Kette, die HCDR1, HCDR2 und HCDR3 einer variablen Region der schweren Kette umfasst, die die in SEQ ID NO: 50 dargestellte Aminosäuresequenz umfasst;
(vii) eine variable Region der leichten Kette, die LCDR1, LCDR2 und LCDR3 einer variablen Region der leichten Kette umfasst, die die in SEQ ID NO: 10 dargestellte Aminosäuresequenz umfasst; und eine variable Region der schweren Kette, die HCDR1, HCDR2 und HCDR3 einer variablen Region der schweren Kette umfasst, die die in SEQ ID NO: 58 dargestellte Aminosäuresequenz umfasst;

(viii) eine variable Region der leichten Kette, die LCDR1, LCDR2 und LCDR3 einer variablen Region der leichten Kette umfasst, die die in SEQ ID NO: 10 dargestellte Aminosäuresequenz umfasst; und eine variable Region der schweren Kette, die HCDR1, HCDR2 und HCDR3 einer variablen Region der schweren Kette umfasst, die die in SEQ ID NO: 66 dargestellte Aminosäuresequenz umfasst;

(ix) eine variable Region der leichten Kette, die LCDR1, LCDR2 und LCDR3 einer variablen Region der leichten Kette umfasst, die die in SEQ ID NO: 10 dargestellte Aminosäuresequenz umfasst; und eine variable Region der schweren Kette, die HCDR1, HCDR2 und HCDR3 einer variablen Region der schweren Kette umfasst, die die in SEQ ID NO: 74 dargestellte Aminosäuresequenz umfasst;

(x) eine variable Region der leichten Kette, die LCDR1, LCDR2 und LCDR3 einer variablen Region der leichten Kette umfasst, die die in SEQ ID NO: 90 dargestellte Aminosäuresequenz umfasst; und eine variable Region der schweren Kette, die HCDR1, HCDR2 und HCDR3 einer variablen Region der schweren Kette umfasst, die die in SEQ ID NO: 82 dargestellte Aminosäuresequenz umfasst;

(xi) eine variable Region der leichten Kette, die LCDR1, LCDR2 und LCDR3 einer variablen Region der leichten Kette umfasst, die die in SEQ ID NO: 90 dargestellte Aminosäuresequenz umfasst; und eine variable Region der schweren Kette, die HCDR1, HCDR2 und HCDR3 einer variablen Region der schweren Kette umfasst, die die in SEQ ID NO: 98 dargestellte Aminosäuresequenz umfasst; oder

(xii) eine variable Region der leichten Kette, die LCDR1, LCDR2 und LCDR3 einer variablen Region der leichten Kette umfasst, die die in SEQ ID NO: 90 dargestellte Aminosäuresequenz umfasst; und

eine variable Region der schweren Kette, die HCDR1, HCDR2 und HCDR3 einer variablen Region der schweren Kette umfasst, die die in SEQ ID NO: 106 dargestellte Aminosäuresequenz umfasst; oder

ein Antikörper oder ein Antigen-bindendes Fragment, der/das an dasselbe Epitop wie einer oder mehrere der Punkte (i)-(xii) bindet und/oder mit einem oder mehreren der Punkte (i)-(xii) um die Bindung an LEPR konkurriert; oder

(b) der LEPR-Agonist ein isolierter Agonisten-Antikörper oder ein Antigen-bindendes Fragment ist, der/das spezifisch an LEPR bindet, umfassend:

(i) eine variable Region der leichten Kette, die die in SEQ ID NO: 10 dargestellte Aminosäuresequenz umfasst; und eine variable Region der schweren Kette, die die in SEQ ID NO: 2 dargestellte Aminosäuresequenz umfasst;

(ii) eine variable Region der leichten Kette, die die in SEQ ID NO: 10 dargestellte Aminosäuresequenz umfasst; und eine variable Region der schweren Kette, die die in SEQ ID NO: 18 dargestellte Aminosäuresequenz umfasst;

(iii) eine variable Region der leichten Kette, die die in SEQ ID NO: 10 dargestellte Aminosäuresequenz umfasst; und eine variable Region der schweren Kette, die die in SEQ ID NO: 26 dargestellte Aminosäuresequenz umfasst;

(iv) eine variable Region der leichten Kette, die die in SEQ ID NO: 10 dargestellte Aminosäuresequenz umfasst; und eine variable Region der schweren Kette, die die in SEQ ID NO: 34 dargestellte Aminosäuresequenz umfasst;

(v) eine variable Region der leichten Kette, die die in SEQ ID NO: 10 dargestellte Aminosäuresequenz umfasst; und eine variable Region der schweren Kette, die die in SEQ ID NO: 42 dargestellte Aminosäuresequenz umfasst;

(vi) eine variable Region der leichten Kette, die die in SEQ ID NO: 10 dargestellte Aminosäuresequenz umfasst; und eine variable Region der schweren Kette, die die in SEQ ID NO: 50 dargestellte Aminosäuresequenz umfasst;

(vii) eine variable Region der leichten Kette, die die in SEQ ID NO: 10 dargestellte Aminosäuresequenz umfasst; und

eine variable Region der schweren Kette, die die in SEQ ID NO: 58 dargestellte Aminosäuresequenz umfasst;

(viii) eine variable Region der leichten Kette, die die in SEQ ID NO: 10 dargestellte Aminosäuresequenz umfasst; und

eine variable Region der schweren Kette, die die in SEQ ID NO: 66 dargestellte Aminosäuresequenz umfasst;

(ix) eine variable Region der leichten Kette, die die in SEQ ID NO: 10 dargestellte Aminosäuresequenz umfasst; und

eine variable Region der schweren Kette, die die in SEQ ID NO: 74 dargestellte Aminosäuresequenz umfasst;

(x) eine variable Region der leichten Kette, die die in SEQ ID NO: 90 dargestellte Aminosäuresequenz umfasst; und

eine variable Region der schweren Kette, die die in SEQ ID NO: 82 dargestellte Aminosäuresequenz umfasst;

(xi) eine variable Region der leichten Kette, die die in SEQ ID NO: 90 dargestellte Aminosäuresequenz umfasst; und

eine variable Region der schweren Kette, die die in SEQ ID NO: 98 dargestellte Aminosäuresequenz umfasst;

oder

(xii) eine variable Region der leichten Kette, die die in SEQ ID NO: 90 dargestellte Aminosäuresequenz umfasst; und

eine variable Region der schweren Kette, die die in SEQ ID NO: 106 dargestellte Aminosäuresequenz umfasst;

oder

der ein Antikörper oder ein Antigen-bindendes Fragment ist, der/das an dasselbe Epitop wie einer oder mehrere der Punkte (i)-(xii) bindet und/oder mit einem oder mehreren der Punkte (i)-(xii) um die Bindung an LEPR konkurriert; oder

oder

Mibavademab ist.

**7.** LEPR-Agonist zur Verwendung nach einem der Ansprüche 1 bis 6, wobei das Verfahren ferner die Verabreichung eines weiteren therapeutischen Mittels an das Subjekt in Verbindung mit dem LEPR-Agonisten umfasst, optional wobei das weitere therapeutische Mittel Folgendes ist: rekombinantes menschliches Leptin; Metreleptin; ein PCSK9-Inhibitor; ein Anti-PCSK9-Antikörper oder ein Antigen-bindendes Fragment davon; Alirocumab; Evolocumab; Bococizumab; Lodelcizumab; Ralpancizumab; ein HMG-CoA-Reduktase-Hemmer; Atorvastatin; Rosuvastatin; Cerivastatin; Pitavastatin; Fluvastatin; Simvastatin; Lovastatin; Pravastatin; Ezetimib; Insulin; eine Insulinvariante; ein Insulinsekretagogum; Metformin; ein Sulfonylharnstoff; ein Natrium-Glukose-Cotransporter 2- (SGLT2) Hemmer; Dapaglifozin; Canaglifozin; Empagliflozin; ein GLP-1-Agonist oder Analogon; Exenatid; Liraglutid; Lixisenatid; Albiglutid; Dulaglutid; ein Glucagon- (GCG) Inhibitor; ein Anti-GCG-Antikörper; ein Glucagon-Rezeptor-(GCGR) Inhibitor; ein Anti-GCGR-Antikörper; ein niedermolekularer GCGR-Antagonist; ein GCGR-spezifisches Antisense-Oligonukleotid; ein Anti-GCGR-Aptamer; ein GCGR-Spiegelmer; ein Angiopoietin-ähnlicher Protein- (ANGPTL) Inhibitor; ein Anti-ANGPTL3-Antikörper; ein Anti-ANGPTL4-Antikörper; ein Anti-ANGPTL8-Antikörper; Phentermin; Orlistat; Topiramat; Bupropion; Topiramat und Phentermin; Bupropion und Naltrexon; Bupropion und Zonisamid; Pramlintid und Metreleptin; Lorcaserin; Cetilistat; Tesofensin; Velneperit; Fischöl; Pioglitazon; Setmelanotid; ein Fibrat; Fenofibrat; Prednison; Niacin; Antikonvulsiva; Digoxin; Coumadin; Vitamin D; Thyroxin; ein Schilddrüsenpräparat; ein Vitaminpräparat; ein Kalziumpräparat; Carnitin; Coenzym Q10; ein Medikament gegen Verstopfung; ein antiallergisches Medikament; Gabapentin; ein Narkotikum; Ketamin; Lidocain; und/oder Venlafaxinhydrochlorid.

## Revendications

**1.** Agoniste de LEPR pour utilisation dans une méthode pour le traitement de lipodystrophie partielle, chez un sujet, comprenant le fait de déterminer la possibilité que le sujet est atteint ou est susceptible d'être atteint de lipodystrophie partielle par le biais d'une méthode comprenant le fait de déterminer

(A)

(1) si

(i) un pourcentage de masse graisseuse corporelle total est supérieur à 36 % ; et
(ii) rapport en pour cent de masse graisseuse tronc/jambe est supérieur à 1,35 ; ou

(2) si

(i) un pourcentage de masse graisseuse corporelle total est inférieur ou égal à 36 % ; et
(ii) (rapport en pour cent de masse graisseuse tronc/jambe)-(0,0311 X pourcentage de masse graisseuse corporelle total) $\geq$ 0,232,

dans lequel, si les critères sont respectés, le sujet est atteint ou est susceptible d'être atteint de lipodystrophie partielle et, facultativement, si les critères ne sont pas respectés, le patient n'est pas atteint ou n'est pas susceptible d'être atteint de lipodystrophie partielle, en outre facultativement dans lequel le sujet est une femme ; ou

(B)

(1) si

(i) un pourcentage de masse graisseuse corporelle total est supérieur à 25,7 % ; et
(ii) rapport en pour cent de masse graisseuse tronc/jambe est supérieur à 1,5 ; ou

(2) si

(i) un pourcentage de masse graisseuse corporelle total est inférieur ou égal à 25,7 % ; et
(ii) (rapport en pour cent de masse graisseuse tronc/jambe)-(0,0429 X pourcentage de masse graisseuse corporelle total) > 0,4

dans lequel, si les critères sont respectés, le sujet est atteint ou est susceptible d'être atteint de lipodystrophie partielle et, facultativement, si les critères ne sont pas respectés, le patient n'est pas atteint ou n'est pas susceptible d'être atteint de lipodystrophie partielle, en outre facultativement dans lequel le sujet est un homme ; ou

(C)

(1) si

(i) un pourcentage de masse graisseuse corporelle total est supérieur à 27,5 % ;
et
(ii) rapport en pour cent de masse graisseuse tronc/jambe est supérieur à 1,5 ; ou

(2) si

(i) un pourcentage de masse graisseuse corporelle total est inférieur ou égal à 27,5 % ; et
(ii) (rapport en pour cent de masse graisseuse tronc/jambe)-(0,0429 X pourcentage de masse graisseuse corporelle total) > 0,321,

dans lequel, si les critères sont respectés, le sujet est atteint ou est susceptible d'être atteint de lipodystrophie partielle et, facultativement, si les critères ne sont pas respectés, le patient n'est pas atteint ou n'est pas susceptible d'être atteint de lipodystrophie partielle, en outre facultativement dans lequel le sujet est un homme ; et, si le sujet est atteint de lipodystrophie partielle ou est susceptible d'être atteint de lipodystrophie, alors le fait d'administrer, au sujet, une quantité efficace de l'agoniste de LEPR.

2. Agoniste de LEPR pour utilisation selon la revendication 1 dans lequel le sujet est dans une population

• qui inclut un nombre plus grand de sujets atteint de PLD que celui de la population générale ;
• ayant une probabilité pré-test supérieure ou égale à environ 0,7 % ou supérieure ou égale à environ 1 sur 142 ;

EP 4 294 257 B1

• de membres de famille au premier et deuxième degré de sujets atteints de PLD ;
• de diabétiques non obèses ;
• d'individus avec des triglycérides > environ 400 mg/dL ; et/ou
• d'individus chez qui le médecin remarque une distribution de masse graisseuse altérée.

3. Agoniste de LEPR pour utilisation selon l'une quelconque des revendications 1 et 2, dans lequel :

(a) la lipodystrophie partielle est une lipodystrophie partielle familiale (FPLD) ; et/ou
(b) la lipodystrophie partielle est une lipodystrophie partielle familiale (FPLD) qui est FPLD1, FPLD2, FPLD3, FPLD4, FPLD5, FPLD6, ou FPLD7 ; and/ou
(c) lesdits pourcentages de masse graisseuse sont déterminés par absorptiométrie à rayons X à double énergie en utilisant un modèle à 3 compartiments.

4. Agoniste de LEPR pour utilisation selon l'une quelconque des revendications 1 à 3, dans lequel l'agoniste de LEPR est un anticorps agoniste isolé ou fragment de liaison à l'antigène qui se lie spécifiquement à LEPR.

5. Agoniste de LEPR pour utilisation selon l'une quelconque des revendications 1 à 4 dans lequel :

(a) le sujet souffre d'un ou de plusieurs troubles sélectionnés parmi le groupe constitué de :

• acanthosis nigricans ;
• athérosclérose ;
• maladie cardiaque coronaire athérosclérotique ;
• arythmie cardiaque ;
• cardiomyopathie ;
• insuffisance cardiaque congestive ;
• maladie artérielle coronaire ;
• diabète ;
• dyslipidémie ;
• xanthome éruptif ;
• intolérance au glucose ;
• stéatose hépatique ;
• hépatomégalie ;
• hirsutisme ;
• hyperandrogénémie ;
• hyperglycémie ;
• hypertension ;
• hypertriglyceridémie ;
• résistance à l'insuline ;
• cirrhose du foie ;
• dystrophie musculaire ;
• myopathie ;
• stéatohépatite non alcoolique ;
• oligoménorrhée ;
• pancréatite ;
• syndrome des ovaires polykystiques ;
• maladie rénale protéinurique ;
• sévère résistance à l'insuline ; et
• sous-fertilité ; et/ou

(b) le sujet présente une mutation homozygote ou hétérozygote dans le gène *LMNA, PPARG, PLIN1, AKT2, LIPE, CIDEC, ZMPSTE24, PIK3R1, ANDRA2A, CAV1, PCYTIA, PSMB8, WRN, POLD1,* et/ou *BLM.*

6. Agoniste de LEPR pour utilisation selon l'une quelconque des revendications 1 à 5 dans lequel :

(a) l'agoniste de LEPR est un anticorps agoniste isolé ou fragment de liaison à l'antigène qui se lie spécifiquement à LEPR comprenant :

45

(i) une région variable de chaîne légère qui comprend la LCDR1, la LCDR2, et la LCDR3 d'une région variable de chaîne légère qui comprend la séquence d'acides aminés présentée dans SEQ ID n° : 10 ; et une région variable de chaîne lourde qui comprend la HCDR1, la HCDR2, et la HCDR3 d'une région variable de chaîne lourde qui comprend la séquence d'acides aminés présentée dans SEQ ID n° : 2 ;

(ii) une région variable de chaîne légère qui comprend la LCDR1, la LCDR2, et la LCDR3 d'une région variable de chaîne légère qui comprend la séquence d'acides aminés présentée dans SEQ ID n° : 10 ; et une région variable de chaîne lourde qui comprend la HCDR1, la HCDR2, et la HCDR3 d'une région variable de chaîne lourde qui comprend la séquence d'acides aminés présentée dans SEQ ID n° : 18 ;

(iii) une région variable de chaîne légère qui comprend la LCDR1, la LCDR2, et la LCDR3 d'une région variable de chaîne légère qui comprend la séquence d'acides aminés présentée dans SEQ ID n° : 10 ; et une région variable de chaîne lourde qui comprend la HCDR1, la HCDR2, et la HCDR3 d'une région variable de chaîne lourde qui comprend la séquence d'acides aminés présentée dans SEQ ID n° : 26 ;

(iv) une région variable de chaîne légère qui comprend la LCDR1, la LCDR2, et la LCDR3 d'une région variable de chaîne légère qui comprend la séquence d'acides aminés présentée dans SEQ ID n° : 10 ; et une région variable de chaîne lourde qui comprend la HCDR1, la HCDR2, et la HCDR3 d'une région variable de chaîne lourde qui comprend la séquence d'acides aminés présentée dans SEQ ID n° : 34 ;

(v) une région variable de chaîne légère qui comprend la LCDR1, la LCDR2, et la LCDR3 d'une région variable de chaîne légère qui comprend la séquence d'acides aminés présentée dans SEQ ID n° : 10 ; et une région variable de chaîne lourde qui comprend la HCDR1, la HCDR2, et la HCDR3 d'une région variable de chaîne lourde qui comprend la séquence d'acides aminés présentée dans SEQ ID n° : 42 ;

(vi) une région variable de chaîne légère qui comprend la LCDR1, la LCDR2, et la LCDR3 d'une région variable de chaîne légère qui comprend la séquence d'acides aminés présentée dans SEQ ID n° : 10 ; et une région variable de chaîne lourde qui comprend la HCDR1, la HCDR2, et la HCDR3 d'une région variable de chaîne lourde qui comprend la séquence d'acides aminés présentée dans SEQ ID n° : 50 ;

(vii) une région variable de chaîne légère qui comprend la LCDR1, la LCDR2, et la LCDR3 d'une région variable de chaîne légère qui comprend la séquence d'acides aminés présentée dans SEQ ID n° : 10 ; et une région variable de chaîne lourde qui comprend la HCDR1, la HCDR2, et la HCDR3 d'une région variable de chaîne lourde qui comprend la séquence d'acides aminés présentée dans SEQ ID n° : 58 ;

(viii) une région variable de chaîne légère qui comprend la LCDR1, la LCDR2, et la LCDR3 d'une région variable de chaîne légère qui comprend la séquence d'acides aminés présentée dans SEQ ID n° : 10 ; et une région variable de chaîne lourde qui comprend la HCDR1, la HCDR2, et la HCDR3 d'une région variable de chaîne lourde qui comprend la séquence d'acides aminés présentée dans SEQ ID n° : 66 ;

(ix) une région variable de chaîne légère qui comprend la LCDR1, la LCDR2, et la LCDR3 d'une région variable de chaîne légère qui comprend la séquence d'acides aminés présentée dans SEQ ID n° : 10 ; et une région variable de chaîne lourde qui comprend la HCDR1, la HCDR2, et la HCDR3 d'une région variable de chaîne lourde qui comprend la séquence d'acides aminés présentée dans SEQ ID n° : 74 ;

(x) une région variable de chaîne légère qui comprend la LCDR1, la LCDR2, et la LCDR3 d'une région variable de chaîne légère qui comprend la séquence d'acides aminés présentée dans SEQ ID n° : 90 ; et une région variable de chaîne lourde qui comprend la HCDR1, la HCDR2, et la HCDR3 d'une région variable de chaîne lourde qui comprend la séquence d'acides aminés présentée dans SEQ ID n° : 82 ;

(xi) une région variable de chaîne légère qui comprend la LCDR1, la LCDR2, et la LCDR3 d'une région variable de chaîne légère qui comprend la séquence d'acides aminés présentée dans SEQ ID n° : 90 ; et une région variable de chaîne lourde qui comprend la HCDR1, la HCDR2, et la HCDR3 d'une région variable de chaîne lourde qui comprend la séquence d'acides aminés présentée dans SEQ ID n° : 98 ;

ou

(xii) une région variable de chaîne légère qui comprend la LCDR1, la LCDR2, et la LCDR3 d'une région variable de chaîne légère qui comprend la séquence d'acides aminés présentée dans SEQ ID n° : 90 ; et

une région variable de chaîne lourde qui comprend la HCDR1, la HCDR2, et la HCDR3 d'une région variable de chaîne lourde qui comprend la séquence d'acides aminés présentée dans SEQ ID n° : 106 ;
ou
un anticorps ou fragment de liaison à l'antigène qui se lie au même épitope qu'un ou plusieurs quelconques de (i)-(xii) et/ou rivalise pour se lier à LEPR avec un ou plusieurs quelconques de (i)-(xii) ;
ou

(b) l'agoniste de LEPR est un anticorps agoniste isolé ou fragment de liaison à l'antigène qui se lie spécifiquement à LEPR comprenant :

(i) une région variable de chaîne légère qui comprend la séquence d'acides aminés présentée dans SEQ ID n° : 10 ; et

une région variable de chaîne lourde qui comprend la séquence d'acides aminés présentée dans SEQ ID n° : 2 ;

(ii) une région variable de chaîne légère qui comprend la séquence d'acides aminés présentée dans SEQ ID n° : 10 ; et

une région variable de chaîne lourde qui comprend la séquence d'acides aminés présentée dans SEQ ID n° : 18 ;

(iii) une région variable de chaîne légère qui comprend la séquence d'acides aminés présentée dans SEQ ID n° : 10 ; et

une région variable de chaîne lourde qui comprend la séquence d'acides aminés présentée dans SEQ ID n° : 26 ;

(iv) une région variable de chaîne légère qui comprend la séquence d'acides aminés présentée dans SEQ ID n° : 10 ; et

une région variable de chaîne lourde qui comprend la séquence d'acides aminés présentée dans SEQ ID n° : 34 ;

(v) une région variable de chaîne légère qui comprend la séquence d'acides aminés présentée dans SEQ ID n° : 10 ; et

une région variable de chaîne lourde qui comprend la séquence d'acides aminés présentée dans SEQ ID n° : 42 ;

(vi) une région variable de chaîne légère qui comprend la séquence d'acides aminés présentée dans SEQ ID n° : 10 ; et

une région variable de chaîne lourde qui comprend la séquence d'acides aminés présentée dans SEQ ID n° : 50 ;

(vii) une région variable de chaîne légère qui comprend la séquence d'acides aminés présentée dans SEQ ID n° : 10 ; et

une région variable de chaîne lourde qui comprend la séquence d'acides aminés présentée dans SEQ ID n° : 58 ;

(viii) une région variable de chaîne légère qui comprend la séquence d'acides aminés présentée dans SEQ ID n° : 10 ; et

une région variable de chaîne lourde qui comprend la séquence d'acides aminés présentée dans SEQ ID n° : 66 ;

(ix) une région variable de chaîne légère qui comprend la séquence d'acides aminés présentée dans SEQ ID n° : 10 ; et

une région variable de chaîne lourde qui comprend la séquence d'acides aminés présentée dans SEQ ID n° : 74 ;

(x) une région variable de chaîne légère qui comprend la séquence d'acides aminés présentée dans SEQ ID n° : 90 ; et

une région variable de chaîne lourde qui comprend la séquence d'acides aminés présentée dans SEQ ID n° : 82 ;

(xi) une région variable de chaîne légère qui comprend la séquence d'acides aminés présentée dans SEQ ID n° : 90 ; et

une région variable de chaîne lourde qui comprend la séquence d'acides aminés présentée dans SEQ ID n° : 98 ;

ou

(xii) une région variable de chaîne légère qui comprend la séquence d'acides aminés présentée dans SEQ ID n° : 90 ; et

une région variable de chaîne lourde qui comprend la séquence d'acides aminés présentée dans SEQ ID n° : 106 ;

ou

est un anticorps ou fragment de liaison à l'antigène qui se lie au même épitope qu'un ou plusieurs quelconques de (i)-(xii) et/ou rivalise pour se lier à LEPR avec un ou plusieurs quelconques de (i)-(xii)

ou

est mibavadémab.

7. Agoniste de LEPR pour utilisation selon l'une quelconque des revendications 1 à 6, dans lequel la méthode comprend en outre le fait d'administrer, au sujet, un agent thérapeutique supplémentaire en association avec l'agoniste de

**EP 4 294 257 B1**

LEPR, facultativement dans lequel l'agent thérapeutique supplémentaire est : leptine humaine recombinante ; métréleptine ; un inhibiteur de PCSK9 ; un anticorps anti-PCSK9 ou fragment de celui-ci de liaison à l'antigène ; alirocumab ; évolocumab ; bococizumab ; lodelcizumab ; ralpancizumab ; un inhibiteur de HMG-CoA réductase ; atorvastatine ; rosuvastatine ; cérivastatine ; pitavastatine ; fluvastatine ; simvastatine ; lovastatine ; pravastatine ; ezétimibe ; insuline ; un variant d'insuline ; un sécrétagogue d'insuline ; metformine ; une sulfonylurée ; un inhibiteur de cotransporteur de glucose sodique 2 (SGLT2) ; dapaglifozine ; canaglifozine ; empagliflozine ; un agoniste de GLP-1 ou analogue ; exénatide ; liraglutide ; lixisénatide ; albiglutide ; dulaglutide ; un inhibiteur de glucagon (GCG) ; un anticorps anti-GCG ; un inhibiteur de récepteur de glucagon (GCGR) ; un anticorps anti-GCGR ; une petite molécule antagoniste de GCGR ; un oligonucléotide antisens spécifique de GCGR ; un aptamère anti-GCGR ; un Spiegelmer GCGR ; un inhibiteur de protéine de type angiopoïétine (ANGPTL) ; un anticorps anti-ANGPTL3 ; un anticorps anti-ANGPTL4 ; un anticorps anti-ANGPTL8 ; phentermine ; orlistat ; topiramate ; bupropion ; topiramate & phentermine ; bupropion & naltrexone ; bupropion & zonisamide ; pramlintide & métréleptine ; lorcasérine ; cétilistat ; tésofensine ; velneperit ; huile de poisson ; pioglitazone ; setmélanotide ; un fibrate ; fénofibrate ; prédnisone ; niacine ; anticonvulsants ; digoxine ; coumadine ; vitamine D ; thyroxine ; un complément pour thyroïde ; un complément de vitamine ; un complément de calcium ; carnitine; coenzyme Q10 ; un médicament anti-constipation ; un médicament anti-allergique ; gabapentine ; un narcotique ; kétamine ; lidocaïne ; et/ou chlorhydrate de venlafaxine.

Figure 1

# UKB_female_adiposity vs trunk/leg

Figure 2

# FPLD Total Fat vs Trunk/Leg

**Figure 3**

# UKB_female_adiposity vs trunk/leg WITH SHADOWS FPLD

Figure 4

**Figure 5A**

(A)

**Figure 5B**

(B)

## Figure 6

Figure 7

Figure 8

Figure 9

Figure 10

Figure 11

Figure 12

Figure 13

Figure 14

Figure 15

Figure 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63152100
- US 2016056465 W
- WO 2017066204 A
- WO 201766204 A

**Non-patent literature cited in the description**

- **AKINCI et al.** *Lipodystrophy Syndromes: Presentation and Treatment*, 24 April 2018
- Endotext [Internet. South Dartmouth (MA): MDText.com, Inc. 2000
- **BROWN et al.** , The Diagnosis and Management of Lipodystrophy Syndromes: A Multi-Society Practice Guideline. *The Journal of clinical endocrinology and metabolism*, 2016, vol. 101, 4500-4511
- **HANDELSMAN et al.** , The clinical approach to the detection of lipodystrophy - an AACE consensus statement.. *Endocr Pract*, 2013, vol. 19, 107-116
- **ARAÚJO-VILAR** ; **SANTINI**. Diagnosis and treatment of lipodystrophy: a step-by-step approach. *Journal of Endocrinological Investigation*, 2019, vol. 42, 61-73
- **AJLUNI et al.** , Spectrum of disease associated with partial lipodystrophy: lessons from a trial cohort.. *Clin Endocrinol (Oxf)*, 2017, vol. 86, 698-707
- **VASANDANI et al.** , Diagnostic Value of Anthropometric Measurements for Familial Partial Lipodystrophy, Dunnigan Variety. *J Clin Endocrinol Metab*, 2020, vol. 105 (7), 2132-2141
- **KIM et al.** The Origin and Composition of Korean Ethnicity Analyzed by Ancient and Present-Day Genome Sequences. *Genome Biol. Evol.*, 2020, vol. 12 (5), 553-565
- A global reference for human genetic variation. *Nature*, 2015, vol. 526, 68
- **JEON et al.** Korean Genome Project: 1094 Korean personal genomes with clinical information. *Science Advances*, 2020, vol. 6 (22)
- **KWON** ; **FARRELL**. The Magnitude and Challenge of False-Positive Newborn Screening Test Results. *Arch Pediatr Adolesc Med.*, 2000, vol. 154 (7), 714-718
- Tools for measuring bone in children and adolescents. **WARD et al.** Bone densitometry in growing patients -Guidelines for clinical practice. Humana Press, 2007, 15-40
- **MERAL et al.** Fat Shadows' from DXA for the Qualitative Assessment of Lipodystrophy: When a Picture Is Worth a Thousand Numbers. *Diabetes Care*, 2018, vol. 41, 2255-2258
- **HIND et al.** , In vivo precision of the GE Lunar iDXA densitometer for the measurement of total body composition and fat distribution in adults.. *Eur J Clin Nutr*, 2011, vol. 65, 140-142
- **CHEN et al.** , Precision of total body BMD and body composition using the GE Lunar Prodigy densitometer [Abstract. *The American Society for Bone and Mineral Research (ASBMR)*, 2005
- **KIEBZAK et al.** Measurement precision of body composition variables using the lunar DPX-L densitometer. *J Clin Densitom*, 2000, vol. 3, 35-41
- **CORDERO-MACLNTYRE et al.** , Reproducibility of DXA in obese women.. *J Clin Densitom.*, 2002, vol. 5, 35-44
- **GENTON et al.** , Comparison of body weight and composition measured by two different dual energy X-ray absorptiometry devices and three acquisition modes in obese women.. *Clin Nutr*, 2006, vol. 25, 428-437
- **JOHNSON et al.** , Precision and stability of dual-energy X-ray absorptiometry measurements.. *Calcif Tissue Int*, 1991, vol. 49, 174-178
- **REZZI et al.** , Body composition precision with the Lunar iDXA [abstract].. *J Clin Densitom*, 2009, vol. 12 (402), 176
- **TOTHILL et al.** , Comparisons between Hologic, Lunar and Norland dual-energy X-ray absorptiometers and other techniques used for whole-body soft tissue measurements.. *Eur J Clin Nutr*, 1994, vol. 48, 781-794
- **TOOMBS et al.** The Impact of Recent Technological Advances on the Trueness and Precision of DXA to Assess Body Composition. *Obesity*, 2012, vol. 20, 30-39
- **KABAT et al.** Sequences of Proteins of Immunological Interest. National Institutes of Health, 1991
- **KABAT**. *Adv. Prot. Chem.*, 1978, vol. 32, 1-75

- **KABAT et al.** *J. Biol. Chem.*, 1977, vol. 252, 6609-6616
- **CHOTHIA et al.** *J Mol. Biol.*, 1987, vol. 196, 901-917
- **CHOTHIA et al.** *Nature*, 1989, vol. 342, 878-883
- *WHO Drug Information*, 2020, vol. 34 (4)
- **ALTSCHUL et al.** *FEBS J*, 2005, vol. 272 (20), 5101-5109
- **ALTSCHUL, S. F. et al.** *J. Mol. Biol.*, 1990, vol. 215, 403-410
- **GISH, W. et al.** *Nature Genet.*, 1993, vol. 3, 266-272
- **MADDEN, T. L. et al.** *Meth. Enzymol.*, 1996, vol. 266, 131-141
- **ALTSCHUL, S. F. et al.** *Nucleic Acids Res.*, 1997, vol. 25, 3389-3402
- **ZHANG, J. et al.** *Genome Res.*, 1997, vol. 7, 649-656
- **WOOTTON, J. C. et al.** *Comput. Chem.*, 1993, vol. 17, 149-163
- **HANCOCK, J. M. et al.** *Comput. Appl. Biosci.*, 1994, vol. 10, 67-70
- A model of evolutionary change in proteins. **DAYHOFF, M. O. et al.** Atlas of Protein Sequence and Structure. Natl. Biomed. Res. Found, 1978, vol. 5, 345-352
- Matrices for detecting distant relationships.. **SCHWARTZ, R. M. et al.** Atlas of Protein Sequence and Structure. Natl. Biomed. Res. Found., 1978, vol. 5, 353-358
- **ALTSCHUL, S. F**. *J. Mol. Biol.*, 1991, vol. 219, 555-565
- **STATES, D. J et al.** *Methods*, 1991, vol. 3, 66-70
- **HENIKOFF, S. et al.** *Proc. Natl. Acad. Sci. USA*, 1992, vol. 89, 10915-10919
- **ALTSCHUL, S.F. et al.** *J. Mol. Evol.*, 1993, vol. 36, 290-300
- **KARLIN, S. et al.** *Proc. Natl. Acad. Sci. USA*, 1990, vol. 87, 2264-2268
- **KARLIN, S. et al.** *Proc. Natl. Acad. Sci. USA*, 1993, vol. 90, 5873-5877
- **DEMBO, A. et al.** *Ann. Prob.*, 1994, vol. 22, 2022-2039
- Evaluating the statistical significance of multiple distinct local alignments.. **ALTSCHUL, S. F**. Theoretical and Computational Methods in Genome Research, 1-14
- **HANDELSMAN et al.** The Clinical Approach to the Detection of Lipodystrophy - An AACE Consensus Statement. *Endocr Pract.*, 2013, vol. 19 (1), 107-116
- **HUSSAIN** ; **GARG**. Lipodystrophy Syndromes. *Endocrinology and Metabolism Clinics of North America*, December 2016, vol. 45 (4), 783-797
- **JÉRU et al.** , Clinical Utility Gene Card for: Familial partial lipodystrophy. *European Journal of Human Genetics*, 2016, vol. 25, e1-e5
- **GEORGE et al.** *Science*, 2004, vol. 304 (5675), 1325-1328
- **ZOLOTOV et al.** A. Homozygous LIPE mutation in siblings with multiple symmetric lipomatosis, partial lipodystrophy, and myopathy. *Am. J. Med. Genet.*, 2017, vol. 173A, 190-194
- **BERGER et al.** , Familial lipodystrophy associated with neurodegeneration and congenital cataracts.. *Neurology*, 2002, vol. 58, 43-47
- **CAO et al.** , Heterozygous CAV1 frameshift mutations (MIM 601047) in patients with atypical partial lipodystrophy and hypertriglyceridemia. *Lipids Health Dis*, 2008, vol. 7, 3
- **GARG et al.** , Whole exome sequencing identifies de novo heterozygous CAV1 mutations associated with a novel neonatal onset lipodystrophy syndrome.. *Am. J. Med. Genet*, 2015, vol. 167A, 1796-1806
- **RAZANI et al.** , Caveolin-1-deficient mice are lean, resistant to diet-induced obesity, and show hypertriglyceridemia with adipocyte abnormalities.. *J. Biol. Chem.*, 2002, vol. 277, 8635-8647
- Remington's Pharmaceutical Sciences and U.S. Pharmacopeia: National Formulary. Mack Publishing Company, 1984
- **HARDMAN et al.** Goodman and Gilman's The Pharmacological Basis of Therapeutics. McGraw-Hill, 2001
- **GENNARO**. Remington: The Science and Practice of Pharmacy. Lippincott, Williams, and Wilkins, 2000
- Pharmaceutical Dosage Forms: Parenteral Medications. Marcel Dekker, 1993
- Pharmaceutical Dosage Forms: Tablets. Marcel Dekker, 1990
- Pharmaceutical Dosage Forms: Disperse Systems. Marcel Dekker, 1990
- **WEINER** ; **KOTKOSKIE**. Excipient Toxicity and Safety. Marcel Dekker, Inc., 2000
- **AJLUNI N** ; **MERAL R** ; **NEIDERT AH** ; **BRADY GF** ; **BURAS E** ; **MCKENNA B** ; **DIPAOLA F** ; **CHENEVERT TL** ; **HOROWITZ JF** ; **BUGGS-SAXTON C**. Spectrum of disease associated with partial lipodystrophy: lessons from a trial cohort. *Clin Endocrinol (Oxf)*, 2017, vol. 86, 698-707
- **VASANDANI C** ; **LI X** ; **SEKIZKARDES H** ; **ADAMS-HUET B** ; **BROWN RJ** ; **GARG A**. Diagnostic Value of Anthropometric Measurements for Familial Partial Lipodystrophy, Dunnigan Variety. *J Clin Endocrinol Metab*, 2020, 105

- **MERAL R** ; **RYAN BJ** ; **MALANDRINO N** ; **JALAL A** ; **NEIDERT AH** ; **MUNIYAPPA R** ; **AKINCI B** ; **HOROWITZ JF** ; **BROWN RJ** ; **ORAL EA**. Fat Shadows'' From DXA for the Qualitative Assessment of Lipodystrophy: When a Picture Is Worth a Thousand Numbers. *Diabetes Care*, 2018, vol. 41, 2255-2258
- **GHABEN AL** ; **SCHERER PE**. Adipogenesis and metabolic health. *Nat Rev Mol Cell Biol*, 2019, vol. 20, 242-258
- **AJLUNI et al.** Spectrum of disease associated with partial lipodystrophy: lessons from a trial cohort.. *Clin Endocrinol (Oxf).*, May 2017, vol. 86 (5), 698-707
- **BREIMAN et al.** Classification and Regression Trees. CRC Press, 1984
- **MERAL et al.** Fat Shadows'' From DXA for the Qualitative Assessment of Lipodystrophy: When a Picture Is Worth a Thousand Numbers. *Diabetes Care*, 2018, vol. 41, 2255-2258
- **THERNEAU** ; **ATKINSON**. rpart: Recursive Partitioning and Regression Trees. *R package version 4.1-15*, 2019, https://CRAN.R-project.org/package=rpart